# EUROPEAN PATENT APPLICATION

(11) **EP 1 264 843 A2**
(43) Date of publication of application: **11.12.2002**
(21) Application number: 02251812.0
(22) Date of filing: 12.03.2002
(51) Int. Cl.: C07K 14/47, C07K 16/18, C12N 5/16, C12N 15/12, C12N 15/62, C12N 15/63

(54) **38554, 57301 and 58324, Human organic ion transporters and uses therefor**

(30) Priority: 12.03.2001 US 275172 P
(71) Applicant: MILLENIUM PHARMACEUTICALS, INC., Cambridge, MA 02139 (US)
(72) Inventor: Silos-Santiago, Inmaculada, Del Mar, CA 92014 (US); Curtis, Rory A.J., Southborough MA 01772 (US)
(74) Representative: Lunt, Mark George Francis

(57) **Abstract**

The invention provides isolated nucleic acids molecules, designated 38554, 57301 or 58324 nucleic acid molecules, which encode novel SLC21 or SLC22 family members. The invention also provides antisense nucleic acid molecules, recombinant expression vectors containing 38554, 57301 or 58324 nucleic acid molecules, host cells into which the expression vectors have been introduced, and nonhuman transgenic animals in which a 38554, 57301 or 58324 gene has been introduced or disrupted. The invention still further provides isolated 38554, 57301 or 58324 proteins, fusion proteins, antigenic peptides and anti-38554, 57301 or 58324 antibodies. Diagnostic and therapeutic methods utilizing compositions of the invention are also provided.

## Description

Cellular membranes differentiate the contents of a cell from the surrounding environment. Membranes also serve as effective barriers against the unregulated influx of hazardous or unwanted compounds, and the unregulated efflux of desirable compounds. However, the cell does need a supply of desired compounds and removal of waste products. Transport proteins which are embedded (singly or in complexes) in the cellular membrane (reviewed by Oh and Amidon (1999) in *Membrane Transporters as Drug Targets,* ed. Amidon and Sadee, Kluwer Academic/Plenum Publishers, New York, Chapter 1) are major providers of these functions. There are two general classes of membrane transport proteins: channels or pores, and transporters (also known as carriers or permeases). Channels and transporters differ in their translocation mechanisms. Channels are hydrophilic group-lined protein tunnels whose opening by a regulatory event allow free, rapid passage of their charge-, size-, and geometry-selected small ions down their concentration gradients. Transporters specifically and selectively bind the molecules they move, some with and some against their concentration gradients, across membranes. The binding mechanism causes the action of transporters to be slow and saturable.

Transport molecules are specific for a particular target solute or class of solutes, and are also present in one or more specific membranes. Transport molecules localized to the plasma membrane permit an exchange of solutes with the surrounding environment, while transport molecules localized to intracellular membranes *(e.g.,* membranes of the mitochondrion, peroxisome, lysosome, endoplasmic reticulum, nucleus, or vacuole) permit import and export of molecules from organelle to organelle or to the cytoplasm. For example, in the case of the mitochondrion, transporters in the inner and outer mitochondrial membranes permit the import of sugar molecules, calcium ions, and water (among other molecules) into the organelle and the export of newly synthesized ATP to the cytosol.

Transporters can move molecules by two types of processes. In one process, "facilitated diffusion," transporters move molecules with their concentration gradients. In the other process, "active transport," transporters move molecules against their concentration gradients. Active transport to move a molecule against its gradient requires energy. Primary active transporters, such as Na⁺/K⁺ ATPases or ABC transporters use energy from ATP hydrolysis or light, and establish ion gradients and membrane potential energy. Secondary active transporters, such as the H⁺/peptide transporter, use the pH or ion gradients established by primary active transporters to transport other molecules. In secondary active transport, the transporter uses two separate binding sites to move the primary ion down its concentration gradient to produce the energy to move the secondary solute against its gradient. The coupled solute either travels in the same direction as the primary solute (symport) or in the opposite direction (antiport). Transporters play important roles in the ability of the cell to regulate homeostasis, to grow and divide, and to communicate with other cells, *e.g.*, to transport signaling molecules, such as hormones, reactive oxygen species, ions, neurotransmitters or vitamins. A wide variety of human diseases and disorders are associated with defects in transporter or other membrane transport molecules, including certain types of liver disorders *(e.g.,* due to defects in transport of long-chain fatty acids (Al Odaib *et al.* (1998) *New Eng. J. Med.* 339:1752-1757), hyperlysinemia (mitochondrial lysine transport defect (Oyanagi *et al.* (1986) *Inherit. Metab. Dis.* 9:313-316), and cataract (Wintour (1997) *Clin. Exp. Pharmacol. Physiol.* 24(1):1-9).

There are over 30 families of secondary transporters, also known as solute carriers or SLC (reviewed by Berger, *et al.* (2000) in *The Kidney: Physiology and Pathophysiology*, eds. Seldin DW and Giebisch G., Lippincott, Williams & Wilkins, Philadelphia 1:107-138; also see www.gene.ucl.ac.uk/nomenclature for human proteins). The SLC families are classified according to the pair of molecules they move. The SLC21 and 22 families transport organic ions.

The present invention is based, in part, on the discovery of novel organic ion transporters, referred to herein as "38554," "57301" or "58324". The transporters of the invention are homologous to SLC21 family or SLC22 family members.

The nucleotide sequence of a cDNA encoding 38554 is shown in SEQ ID NO: 1 and SEQ ID NO:22, and the amino acid sequence of a 38554 polypeptide is shown in SEQ ID NO:2 and SEQ ID NO:23. In addition, the nucleotide sequence of the coding region is depicted in SEQ ID NO:3 and SEQ ID NO:24.

The nucleotide sequence of a cDNA encoding 57301 is shown in SEQ ID NO:4, and the amino acid sequence of a 57301 polypeptide is shown in SEQ ID NO:5. In addition, the nucleotide sequence of the coding region is depicted in SEQ ID NO:6.

The nucleotide sequence of a cDNA encoding 58324 is shown in SEQ ID NO:7, and the amino acid sequence of a 58324 polypeptide is shown in SEQ ID NO:8. In addition, the nucleotide sequence of the coding region is depicted in SEQ ID NO:9. Accordingly, in one aspect, the invention features a nucleic acid molecule which encodes a 38554, 57301 or 58324 protein or polypeptide, *e.g.*, a biologically active portion of a 38554, 57301 or 58324 protein. In a preferred embodiment, the isolated nucleic acid molecule encodes a polypeptide having the amino acid sequence of SEQ ID NO:2, 5, 8 or 23. In other embodiments, the invention provides isolated 38554 nucleic acid molecules having the nucleotide sequence shown in SEQ ID NO:1, SEQ ID NO:3, SEQ ID NO:22, SEQ ID NO:24; isolated 57301 nucleic acid molecules having the nucleotide sequence shown in SEQ ID NO:4 or SEQ ID NO:6; isolated 58324 nucleic acid molecules having the nucleotide sequence shown in SEQ ID NO:7 or SEQ ID NO:9. In other embodiments, the invention provides a nucleic acid molecule comprising a nucleotide sequence which is at least 86% identical to the nucleotide sequence of SEQ ID NO:7 or SEQ ID NO:9. In other embodiments, the invention provides a nucleic acid molecule comprising a fragment of at least 417 nucleotides of the nucleotide sequence of SEQ ID NO: 7 or SEQ ID NO:9. In other embodiments, the invention provides a nucleic acid molecule which encodes a fragment of a polypeptide comprising the amino acid sequence of SEQ ID NO:2, wherein the fragment comprises at least 15 contiguous amino acids of SEQ ID NO: 2; a nucleic acid molecule which encodes a fragment of a polypeptide comprising the amino acid sequence of SEQ ID NO:23, wherein the fragment comprises at least 15 contiguous amino acids of SEQ ID NO: 23; a nucleic acid molecule which encodes a fragment of a polypeptide comprising the amino acid sequence of SEQ ID NO:5, wherein the fragment comprises at least 15 contiguous amino acids of SEQ ID NO: 5; a nucleic acid molecule which encodes a fragment of a polypeptide comprising the amino acid sequence of SEQ ID NO:8, wherein the fragment comprises at least 15 contiguous amino acids of SEQ ID NO: 8.

In still other embodiments, the invention provides nucleic acid molecules that are sufficiently or substantially identical (*e.g.*, naturally occurring allelic variants) to the nucleotide sequence shown in SEQ ID NO:1, SEQ ID NO:3, SEQ ID NO:4, SEQ ID NO:6, SEQ ID NO:7, SEQ ID NO:9, SEQ ID NO:22, or SEQ ID NO:24. In other embodiments, the invention provides a nucleic acid molecule which hybridizes under stringent hybridization conditions to a nucleic acid molecule comprising the nucleotide sequence of SEQ ID NO:1, SEQ ID NO:3, SEQ ID NO:4, SEQ ID NO:6, SEQ ID NO:7, SEQ ID NO:9, SEQ ID NO:22, or SEQ ID NO:24, wherein the nucleic acid encodes a full length 38554, 57301 or 58324 protein or an active fragment thereof.

In a related aspect, the invention further provides nucleic acid constructs which include 38554, 57301 or 58324 nucleic acid molecules described herein. In certain embodiments, the nucleic acid molecules of the invention are operatively linked to native or heterologous regulatory sequences. Also included are vectors and host cells containing the 38554, 57301 or 58324 nucleic acid molecules of the invention *e.g.,* vectors and host cells suitable for producing polypeptides.

In another related aspect, the invention provides nucleic acid fragments suitable as primers or hybridization probes for the detection of 38554, 57301 or 58324-encoding nucleic acids.

In still another related aspect, isolated nucleic acid molecules that are antisense to a 38554, 57301 or 58324 encoding nucleic acid molecule are provided.

In another aspect, the invention features 38554, 57301 or 58324 polypeptides, and biologically active or antigenic fragments thereof that are useful, *e.g.*, as reagents or targets in assays applicable to treatment and diagnosis of transporter-associated or other 38554-, 57301- or 58324-associated disorders. In another embodiment, the invention provides 38554, 57301 or 58324 polypeptides having 38554, 57301 or 58324 activities. Preferred polypeptides are 38554, 57301 or 58324 proteins including at least one transporter domain, *e.g.*, an orgainc ion transporter domain, a transmembrane domain and, preferably, having 38554, 57301 or 58324 activity, *e.g.*, 38554, 57301 or 58324 activity as described herein.

In other embodiments, the invention provides 38554, 57301 or 58324 polypeptides, *e.g.,* a 38554, 57301 or 58324 polypeptide having the amino acid sequence shown in SEQ ID NO:2, SEQ ID NO:5, SEQ ID NO:8, SEQ ID NO:23; an amino acid sequence that is sufficiently or substantially identical to the amino acid sequence shown in SEQ ID NO:2, SEQ ID NO:5, SEQ ID NO:8, or SEQ ID NO:23; or an amino acid sequence encoded by a nucleic acid molecule having a nucleotide sequence which hybridizes under stringent hybridization conditions to a nucleic acid molecule comprising the nucleotide sequence of SEQ ID NO:1, SEQ ID NO:3, SEQ ID NO:22, or SEQ ID NO:24, wherein the nucleic acid encodes a full length 38554 protein or an active fragment thereof; to a nucleic acid molecule comprising the nucleotide sequence of SEQ ID NO:4 or SEQ ID NO:6, wherein the nucleic acid encodes a full length 57301 protein or an active fragment thereof; or to a nucleic acid molecule comprising the nucleotide sequence of SEQ ID NO:7 or SEQ ID NO:9, wherein the nucleic acid encodes a full length 58324 protein or an active fragment thereof. In other embodiments, the inventopn providesa polypeptide which is encoded by a nucleic acid molecule comprising a nucleotide sequence of SEQ ID NO: 1 or SEQ ID NO:3, or a complement thereof; a nucleotide sequence of SEQ ID NO: 22 or SEQ ID NO:24, or a complement thereof; a nucleotide sequence of SEQ ID NO: 4 or SEQ ID NO:6, or a complement thereof; or a nucleotide sequence which is at least 86% identical to a nucleic acid comprising the nucleotide sequence of SEQ ID NO: 7 or SEQ ID NO:9, or a complement thereof. In other embodiments, the invention provides a fragment of a polypeptide comprising the amino acid sequence of SEQ ID NO:2, SEQ ID NO:23, SEQ ID NO:5, or SEQ ID NO:8, wherein the fragment comprises at least 15 contiguous amino acids of SEQ ID NO:2, SEQ ID NO:23, SEQ ID NO:5, or SEQ ID NO:8.

In a related aspect, the invention further provides nucleic acid constructs which include a 38554, 57301 or 58324 nucleic acid molecule described herein.

In a related aspect, the invention provides 38554, 57301 or 58324 polypeptides or fragments operatively linked to non-38554, 57301 or 58324 polypeptides to form fusion proteins.

In another aspect, the invention features antibodies and antigen-binding fragments thereof, that react with, or more preferably specifically or selectively bind 38554, 57301 or 58324 polypeptides.

In another aspect, the invention provides methods of screening for compounds that modulate the expression or activity of the 38554, 57301 or 58324 polypeptides or nucleic acids.

In still another aspect, the invention provides a process for modulating 38554, 57301 or 58324 polypeptide or nucleic acid expression or activity, *e.g.*, using the compounds identified in the screens described herein. In certain embodiments, the methods involve treatment of conditions related to aberrant activity or expression of the 38554, 57301 or 58324 polypeptides or nucleic acids, such as conditions involving aberrant or deficient organic ion transport, organic ion absorption or excretion, inter- or intra-cellular signaling, hormonal responses, and cellular proliferation or differentiation. Examples of such disorders, *e.g.*, organic ion transporter or other 38554-, 57301-, or 58324-associated disorders, include but are not limited to, pain disorders, nervous system disorders, immune, *e.g.*, inflammatory disorders, testicular disorders, kidney disorders, or angiogenesis disorders.

The invention also provides assays for determining the activity of or the presence or absence of 38554, 57301 or 58324 polypeptides or nucleic acid molecules in a biological sample, including for disease diagnosis.

In a further aspect, the invention provides assays for determining the presence or absence of a genetic alteration in a 38554, 57301 or 58324 polypeptide or nucleic acid molecule, including for disease diagnosis.

In another aspect, the invention features a two dimensional array having a plurality of addresses, each address of the plurality being positionally distinguishable from each other address of the plurality, and each address of the plurality having a unique capture probe, *e.g.*, a nucleic acid or peptide sequence. At least one address of the plurality has a capture probe that recognizes a 38554, 57301 or 58324 molecule. In one embodiment, the capture probe is a nucleic acid, *e.g.*, a probe complementary to a 38554, 57301 or 58324 nucleic acid sequence. In another embodiment, the capture probe is a polypeptide, *e.g.*, an antibody specific for 38554, 57301 or 58324 polypeptides. Also featured is a method of analyzing a sample by contacting the sample to the aforementioned array and detecting binding of the sample to the array.

Other features and advantages of the invention will be apparent from the following detailed description, and from the claims.

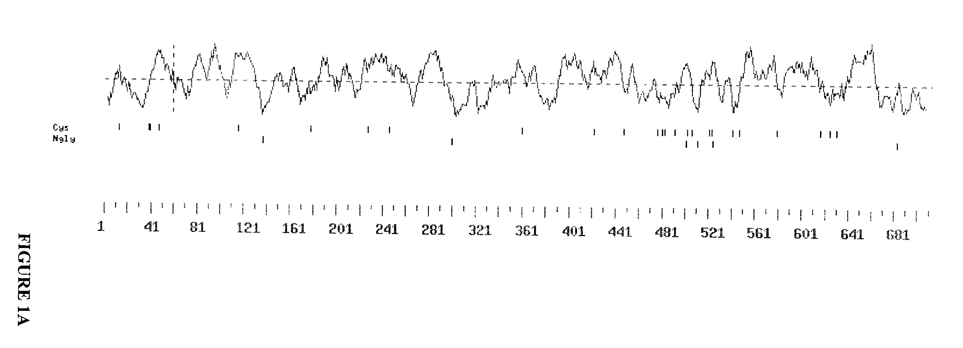

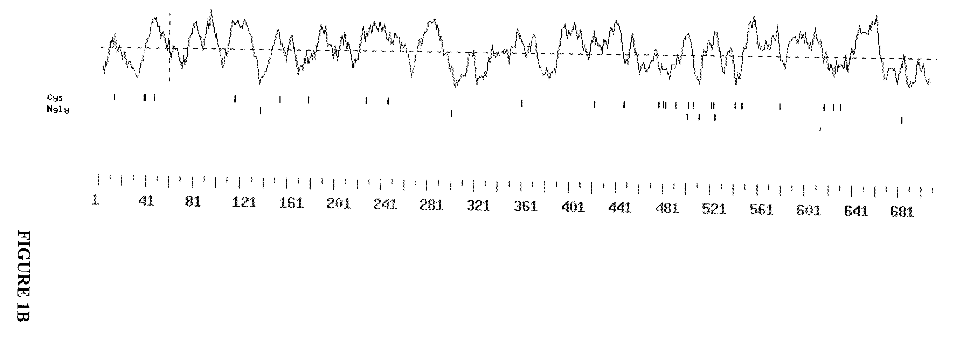

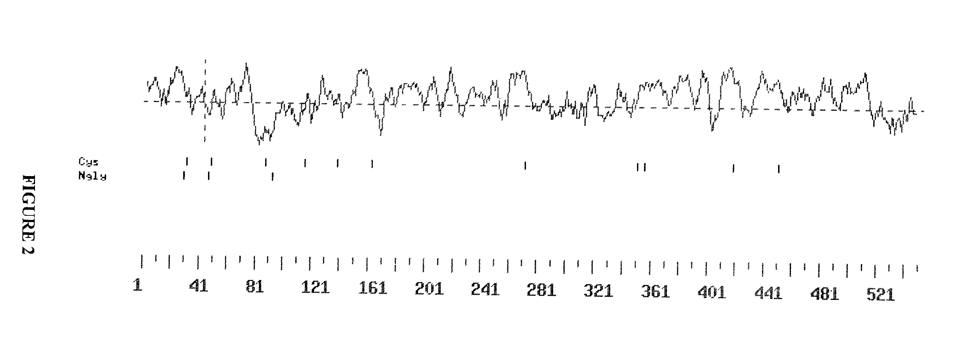

*Figures 1A-1B* depict a hydropathy plot of human 38554. Fbh38554FL is depicted in Figure 1A and Fbh 38554Fla is depicted in Figure 1B. Relatively hydrophobic residues are shown above the dashed horizontal line, and relatively hydrophilic residues are below the dashed horizontal line. The cysteine residues (cys) are indicated by short vertical lines just below the hydropathy trace. The numbers corresponding to the amino acid sequence of human 38554 are indicated. Polypeptides of the invention include fragments which include: all or part of a hydrophobic sequence, *e.g.*, a sequence above the dashed line, *e.g.*, the sequence from about amino acid 111 to 128, from about 274 to 295, and from about 641 to 664 of SEQ ID NO:2 or SEQ ID NO:23; all or part of a hydrophilic sequence, *e.g.*, a sequence below the dashed line, *e.g.*, the sequence of from about amino acid 28 to 36, from about 134 to 142, and from about 301 to 316 of SEQ ID NO:2 or SEQ ID NO:23; a sequence which includes a Cys, or a glycosylation site.

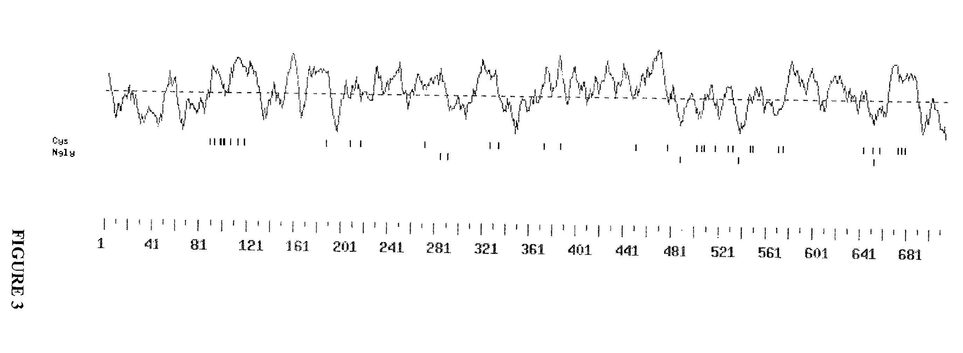

*Figure* 2 depicts a hydropathy plot of human 57301. Relatively hydrophobic residues are shown above the dashed horizontal line, and relatively hydrophilic residues are below the dashed horizontal line. The cysteine residues (cys) are indicated by short vertical lines just below the hydropathy trace. The numbers corresponding to the amino acid sequence of human 57301 are indicated. Polypeptides of the invention include fragments which include: all or part of a hydrophobic sequence, *e.g.*, a sequence above the dashed line, *e.g.*, the sequence from about amino acid 151 to 167, from about 263 to 279, and from about 352 to 369 of SEQ ID NO:5; all or part of a hydrophilic sequence, *e.g.*, a sequence below the dashed line, *e.g.*, the sequence of from about amino acid 82 to 95, from about 325 to 332, and from about 528 to 537 of SEQ ID NO:5; a sequence which includes a Cys, or a glycosylation site.

*Figure 3* depicts a hydropathy plot of human 58324. Relatively hydrophobic residues are shown above the dashed horizontal line, and relatively hydrophilic residues are below the dashed horizontal line. The cysteine residues (cys) are indicated by short vertical lines just below the hydropathy trace. The numbers corresponding to the amino acid sequence of human 58324 are indicated. Polypeptides of the invention include fragments which include: all or part of a hydrophobic sequence, *e.g.*, a sequence above the dashed line, *e.g.,* the sequence from about amino acid 173 to 193, from about 314 to 335, and from about 667 to 691 of SEQ ID NO: 8; all or part of a hydrophilic sequence, *e.g.*, a sequence below the dashed line, *e.g.*, the sequence of from about amino acid 40 to 50, from about 194 to 201, and from about 538 to 546 of SEQ ID NO:8; a sequence which includes a Cys, or a glycosylation site.

### Human 38554

As used herein, "38554" refers to the molecules derived from Fbh38554FL (SEQ ID NO:1, 2, and 3) and to molecules derived from Fbh38554Fla (SEQ ID NO:22, 23, and 24). The human 38554 sequence (SEQ ID NO:1 or SEQ ID NO:22), which is approximately 3220 and 3227 nucleotides long, respectively, including untranslated regions, contains a predicted methionine-initiated coding sequence of about 2139 nucleotides, including the termination codon (nucleotides indicated as coding of SEQ ID NO:1; SEQ ID NO:3 or coding of SEQ ID NO:22; SEQ ID NO:24). The coding sequence encodes a 712 amino acid protein (SEQ ID NO:2 or SEQ ID NO:23).

Human 38554 contains the following regions or other structural features (for general information regarding PFAM identifiers, PS prefix and PF prefix domain identification numbers, refer to Sonnhammer *et al.* (1997) *Protein* 28:405-420 and http://www.pscedu/general/ software/packages/pfam/pfam.html): twelve transmembrane domains, thirteen non-transmembrane regions, a kazal domain (PFAM Accession Number PF00050, SEQ ID NO:10) located at about amino acid residues 476 to 523 of SEQ ID NO:2 or SEQ ID NO:23; and one peroxisomal targeting signal (PSORT PTS2, SEQ ID NO:19) at about amino acids 154 to 162 of SEQ ID NO:2 (not in SEQ ID NO:23). The transmembrane domains (predicted by MEMSAT, Jones et al., (1994) *Biochemistry* 33:3038-3049) are located at about amino acids 42 to 58, 80 to 102, 111 to 128, 190 to 212, 221 to 245, 274 to 295, 354 to 373, 393 to 414, 427 to 446, 553 to 577, 588 to 612, and 641 to 664 of SEQ ID NO:2 or SEQ ID NO:23; and the non-transmembrane regions are located at about amino acids 1 to 41, 59 to 79, 103 to 110, 129 to 189, 213 to 220, 246 to 273, 296 to 353, 374 to 392, 415 to 426, 447 to 552, 578 to 587, 613 to 640, and 665 to 712 of SEQ ID NO:2 or SEQ ID NO:23. The hydropathy plots of 38554 are depicted in Figures 1A and 1B.

Human 38554 also contains the following regions or other structural features: one tyrosine kinase phosphorylation site (Prosite PS00007) at about amino acids 378 to 384 of SEQ ID NO:2 or SEQ ID NO:23; thirteen protein kinase C phosphorylation sites (Prosite PS00005) at about amino acids 4 to 6, 24 to 26, 152 to 154, 264 to 266, 312 to 314, 345 to 347, 374 to 376, 388 390, 509 to 511, 512 to 514, 629 to 631, 677 to 679 and 685 to 687 of SEQ ID NO:2 or twelve protein kinase C phosphorylation sites (Prosite PS00005) at about amino acids 4 to 6, 24 to 26, 264 to 266, 312 to 314, 345 to 347, 374 to 376, 388 390, 509 to 511, 512 to 514, 629 to 631, 677 to 679 and 685 to 687 of SEQ ID NO:23; eleven casein kinase II phosphorylation sites (Prosite PS00006) located at about amino acids 4 to 7, 31 to 34, 68 to 71, 165 to 168, 264 to 267, 304 to 307, 310 to 313, 466 to 469, 485 to 488, 677 to 680, and 694 to 697 of SEQ ID NO:2 or SEQ ID NO:23; six N-glycosylation sites (Prosite PS00001) from about amino acids 146 to 149, 309 to 312, 510 to 513, 520 to 523, 533 to 536, and 692 to 695 of SEQ ID NO:2 or SEQ ID NO:23; and twelve N-myristoylation sites (Prosite PS00008) from about amino acids 82 to 87, 226 to 231, 243 to 248, 385 to 390, 406 to 411, 446 to 451, 454 to 459, 505 to 510, 525 to 530, 537 to 537, 568 to 573, and 625 to 630 of SEQ ID NO:2 or SEQ ID NO:23.

### Human 57301

The human 57301 sequence (SEQ ID NO:4), which is approximately 2866 nucleotides long including untranslated regions, contains a predicted methionine-initiated coding sequence of about 1662 nucleotides, including the termination codon (nucleotides indicated as coding of SEQ ID NO:4; SEQ ID NO:6). The coding sequence encodes a 553 amino acid protein (SEQ ID NO:5).

Human 57301 contains the following regions or other structural features: twelve transmembrane domains, thirteen non-transmembrane regions, and a sugar (and other) transporter domain (PFAM Accession Number PF00083) located at about amino acid residues 106 to 530 of SEQ ID NO:5. The transmembrane domains (predicted by MEMSAT, Jones et al., (1994) *Biochemistry* 33:3038-3049) are located at about amino acids 21 to 37, 151 to 167, 174 to 196, 204 to 222, 232 to 255, 263 to 279, 352 to 369, 378 to 400, 409 to 426, 436 to 455, 466 to 486 and 495 to 515 of SEQ ID NO:5; and the non-transmembrane regions at about amino acids 1 to 20, 38 to 150, 168 to 173, 197 to 203, 223 to 231, 256 to 262, 280 to 351, 370 to 377, 401 to 408, 427 to 435, 456 to 465, 487 to 494, and 516 to 553 of SEQ ID NO:5. The hydropathy plot of 57301 is depicted in Figure 2.

Human 57301 also contains the following regions or other structural features: four protein kinase C phosphorylation sites (Prosite PS00005) at about amino acids 46 to 48, 167 to 169, 282 to 284, and 289 to 291 of SEQ ID NO:5; four casein kinase II phosphorylation sites (Prosite PS00006) located at about amino acids 35 to 38, 107 to 110, 211 to 214, and 526 to 529 of SEQ ID NO:5; two cAMP/cGMP-dependent protein kinase phosphorylation sites (Prosite PS00004) located at about amino acids 405 to 408 and 536 to 539 of SEQ ID NO:5; three N-glycosylation sites (Prosite PS00001) from about amino acids 39 to 42, 56 to 59, and 102 to 105 of SEQ ID NO:5; two amidation sites (Prosite PS00009) from about amino acids 170 to 173 and 403 to 406 of SEQ ID NO:5; and eight N-myristoylation sites (Prosite PS00008) from about amino acids 155 to 160, 187 to 192, 246 to 251, 331 to 336, 431 to 436, 443 to 448, 472 to 477 and 541 to 546 of SEQ ID NO:5.

### Human 58324

The human 58324 sequence (SEQ ID NO:7), which is approximately 2480 nucleotides long including untranslated regions, contains a predicted methionine-initiated coding sequence of about 2160 nucleotides, including the termination codon (nucleotides indicated as coding of SEQ ID NO:7; SEQ ID NO:9). The coding sequence encodes a 719 amino acid protein (SEQ ID NO:8).

Human 58324 contains the following regions or other structural features: twelve transmembrane domains, thirteen non-transmembrane regions, and a kazal domain (PFAM Accession Number PF00050) located at about amino acid residues 502 to 549 of SEQ ID NO:8. The transmembrane domains (predicted by MEMSAT, Jones et al., (1994) *Biochemistry* 33:3038-3049) are located at about amino acids 107 to 126, 150 to 166, 173 to 193, 231 to 254, 265 to 289, 314 to 335, 372 to 391, 420 to 444, 457 to 475, 580 to 603, 614 to 635, and 667 to 691 of SEQ ID NO:8; and the non-transmembrane regions are located at about amino acids 1 to 106, 127 to 149, 167 to 172, 194 to 230, 255 to 264, 290 to 313, 336 to 371, 392 to 419, 445 to 456, 476 to 579, 604 to 613, 636 to 666, and 692 to 719 of SEQ ID NO:8. The hydropathy plot of 58324 is depicted in Figure 3.

Human 58324 also contains the following regions or other structural features: seven protein kinase C phosphorylation sites (Prosite PS00005) at about amino acids 38 to 40, 41 to 43, 75 to 77, 342 to 344, 450 to 452, 492 to 494, and 705 to 707 of SEQ ID NO:8; five casein kinase II phosphorylation sites (Prosite PS00006) located at about amino acids 11 to 14, 129 to 132, 192 to 195, 252 to 255, and 445 to 448 of SEQ ID NO:8; one tyrosine kinase site (Prosite PS00007) located at about amino acids 144 to 151 of SEQ ID NO:8; five N-glycosylation sites (Prosite PS00001) from about amino acids 294 to 297, 300 to 303, 497 to 500, 546 to 549 and 661 to 664 of SEQ ID NO:8; one amidation site (Prosite PS00009) from about amino acids 44 to 47 of SEQ ID NO:8; and eleven N-myristoylation sites (Prosite PS00008) from about amino acids 37 to 42, 92 to 97, 100 to 105, 120 to 125, 184 to 189, 216 to 221, 264 to 269, 432 to 437, 441 to 446, 531 to 536, and 553 to 558 of SEQ ID NO:8.

**Table 1:**

| **Summary of Sequence Information for 38554, 57301, and 58324** | | | |
|---|---|---|---|
| **Gene** | **cDNA** | **ORF** | **Polypeptide** |
| 38554 | SEQ ID NO:1 or 22 | SEQ ID NO:3 or 24 | SEQ ID NO:2 or 23 |
| 57301 | SEQ ID NO:4 | SEQ ID NO:6 | SEQ ID NO:5 |
| 58324 | SEQ ID NO:7 | SEQ ID NO:9 | SEQ ID NO:8 |

The 38554, 57301 and 58324 proteins contain a significant number of structural characteristics in common with members of the SLC21 or 22 transporter families. The term "family" when referring to the protein and nucleic acid molecules of the invention means two or more proteins or nucleic acid molecules having a common structural domain or motif and having sufficient amino acid or nucleotide sequence homology as defined herein. Such family members can be naturally or non-naturally occurring and can be from either the same or different species. For example, a family can contain a first protein of human origin as well as other distinct proteins of human origin, or alternatively, can contain homologs of non-human origin, *e.g.*, rat or mouse proteins. Members of a family can also have common functional characteristics.

As used herein, the terms "transporter," "organic ion transporter," "organic anion transporter," "SLC21 family, or SLC22 family" include secondary active transport proteins. Secondary active transporters couple the active transport of one molecule, *e.g.,* an ion, *e.g.*, an organic ion (*e.g.*, an organic anion or a cation, a prostaglandin, a steroidal compound (*e.g.*, estrone-3-sulfate), a bile acid, a drug, a neurotransmitter, a sulfated lipophilic metabolite, a glucuronidated lipophilic metabolite, a polyamine, a carnitine, or a choline) against its concentration gradient to the energy gained by concomitant transport of a second molecule, *e.g.*, another ion (*e.g.*, a bicarbonate ion or a dicarboxylate ion) with its concentration gradient.

The SLC21 or SLC22 families of proteins of proteins are characterized by at least one, two, three, four, five, six, seven, eight, nine, ten, eleven, preferably twelve transmembrane domains. Typically, the hydrophobic transmembrane domains anchor the transporter within a cell or organelle membrane and through coordinated allosteric movements, effect the transport function across the membrane. The non-transmembrane loops between and beyond the transmembrane domains of the transporter determine the ion binding specificity and provide the ion binding and release activity for the transporter. Some members of these families also have a transporter domain, and/or a kazal domain.

A GAP alignment of 38554 with an SLC21 family member, organic anion transporting protein 14 (OATP-F, accession number 7839587 in GenPept, corresponding to AF260704 in Genbank, SEQ ID NO:13) results in 99.7% identity between the two sequences (as determined from a matrix made by matblas from blosum62.iij). A GAP alignment of 57301 with an SLC22 family member, organic anion transporter 4 (hOAT4, SEQ ID NO:16, accession number 7707622 in GenPept, corresponding to AB026116 in Genbank) results in 51.7% identity between the two sequences (as determined from a matrix made by matblas from blosum62.iij). A GAP alignment of 57301 with an SLC22 family member, renal-specific transporter (mouse RST, SEQ ID NO:17, accession number 2696709 in GenPept, corresponding to BAA23875 in GenBank) results in 71.4% identity between the two sequences (as determined from a matrix made by matblas from blosum62.iij). A GAP alignment of 58324 with an SLC21 family member, organic anion transporter (OATP-E, SEQ ID NO:18, accession number 6683743 in GenPept, corresponding to AB026116 in Genbank) results in 30% identity between the two sequences (as determined from a matrix made by matblas from blosum62.iij).

A 38554, 57301 or 58324 polypeptide can include at least one, two, three, four, five, six, seven, eight, nine, ten, eleven, preferably twelve "transmembrane domains" or regions homologous with a "transmembrane domain".

As used herein, the term "transmembrane domain" includes an amino acid sequence of about 10 to 40 amino acid residues in length and spans the plasma membrane. Transmembrane domains are rich in hydrophobic residues, *e.g.*, at least 50%, 60%, 70%, 80%, 90%, 95% or more of the amino acids of a transmembrane domain are hydrophobic, *e.g.*, leucines, isoleucines, tyrosines, or tryptophans. Transmembrane domains typically have alpha-helical structures and are described in, for example, Zagotta, W.N. *et al.*, (1996) *Annual Rev. Neurosci.* 19:235-263, the contents of which are incorporated herein by reference.

In a preferred embodiment, a 38554, 57301 or 58324 polypeptide or protein has at least one, two, three, four, five, six, seven, eight, nine, ten, eleven, preferably twelve "transmembrane domains" or regions which include at least about 12 to 35 more preferably about 14 to 30 or 15 to 25 amino acid residues each and have at least about 60%, 70% 80% 90% 95%, 99%, or 100% homology with a "transmembrane domain," *e.g.*, the transmembrane domains of human 38554, 57301 or 58324 (*e.g.*, residues 42 to 58, 80 to 102, 111 to 128, 190 to 212, 221 to 245, 274 to 295, 354 to 373, 393 to 414, 427 to 446, 553 to 577, 588 to 612, and 641 to 664 of SEQ ID NO:2 or SEQ ID NO:23; residues 21 to 37, 151 to 167, 174 to 196, 204 to 222, 232 to 255, 263 to 279, 352 to 369, 378 to 400, 409 to 426, 436 to 455, 466 to 486 and 495 to 515 of SEQ ID NO:5; or residues 107 to 126, 150 to 166, 173 to 193, 231 to 254, 265 to 289, 314 to 335, 372 to 391, 420 to 444, 457 to 475, 580 to 603, 614 to 635, and 667 to 691 of SEQ ID NO:8). The transmembrane domains in 38554, 57301 and 58324 are shown in the hydropathy plots (Figures 1A, 1B, 2 and 3, respectively) as regions of about 15 to 25 amino acids where the hydropathy trace is mostly above the horizontal line.

To identify the presence of a "transmembrane" domain in a 38554, 57301 or 58324 protein sequence, and make the determination that a polypeptide or protein of interest has a particular profile, the amino acid sequence of the protein can be analyzed by a transmembrane prediction method that predicts the secondary structure and topology of integral membrane proteins based on the recognition of topological models (MEMSAT, Jones *et al.*, (1994) *Biochemistry* 33:3038-3049).

A 38554, 57301 or 58324 polypeptide can include at least one, two, three, four, five, six, seven, eight, nine, ten, eleven, twelve, preferably thirteen "non-transmembrane regions." As used herein, the term "non-transmembrane region" includes an amino acid sequence not identified as a transmembrane domain. The non-transmembrane regions in 38554 are located at about amino acids 1 to 41, 59 to 79, 103 to 110, 129 to 189, 213 to 220, 246 to 273, 296 to 353, 374 to 392, 415 to 426, 447 to 552, 578 to 587, 613 to 640, and 665 to 712 of SEQ ID NO:2 or SEQ ID NO:23. The non-transmembrane regions in 57301 are located at about amino acids 1 to 20, 38 to 150, 168 to 173, 197 to 203, 223 to 231, 256 to 262, 280 to 351, 370 to 377, 401 to 408, 427 to 435, 456 to 465, 487 to 494, and 516 to 553 of SEQ ID NO:5. The non-transmembrane regions in 58324 are located at about amino acids 1 to 106, 127 to 149, 167 to 172, 194 to 230, 255 to 264, 290 to 313, 336 to 371, 392 to 419, 445 to 456, 476 to 579, 604 to 613, 636 to 666, and 692 to 719 of SEQ ID NO:8.

The non-transmembrane regions of 38554, 57301 or 58324 include at least one, two, three, four, five, six, preferably seven cytoplasmic regions. When located at the N-terminus, the cytoplasmic region is referred to herein as the "N-terminal cytoplasmic domain." As used herein, an "N-terminal cytoplasmic domain" includes an amino acid sequence having about 1 to 300, preferably about 1 to 250, preferably about 1 to 200, more preferably about 1 to 150, or even more preferably about 1 to 110 amino acid residues in length and is located inside of a cell or within the cytoplasm of a cell. The C-terminal amino acid residue of an "N-terminal cytoplasmic domain" is adjacent to an N-terminal amino acid residue of a transmembrane domain in a 38554, 57301 or 58324 protein. For example, an N-terminal cytoplasmic domain is located at about amino acid residues 1 to 41 of SEQ ID NO:2 or SEQ ID NO:23, 1 to 20 of SEQ ID NO:5, and 1 to 106 of SEQ ID NO:8.

In a preferred embodiment, a 38554, 57301 or 58324 polypeptide or protein has an N-terminal cytoplasmic domain or a region which includes at least about 5, preferably about 10 to 200, and more preferably about 15 to 110 amino acid residues and has at least about 60%, 70% 80% 90% 95%, 99%, or 100% homology with an "N-terminal cytoplasmic domain," *e.g.*, the N-terminal cytoplasmic domain of human 38554, 57301 or 58324 (*e.g.*, residues 1 to 41 of SEQ ID NO:2 or SEQ ID NO:23, 1 to 20 of SEQ ID NO:5, and 1 to 106 of SEQ ID NO:8).

In another embodiment, a cytoplasmic region of a 38554, 57301 or 58324 protein can include the C-terminus and can be a "C-terminal cytoplasmic domain," also referred to herein as a "C-terminal cytoplasmic tail." As used herein, a "C-terminal cytoplasmic domain" includes an amino acid sequence having a length of at least about 15, preferably about 20 to 60, more preferably about 25 to 55 amino acid residues and is located inside of a cell or within the cytoplasm of a cell. The N-terminal amino acid residue of a "C-terminal cytoplasmic domain" is adjacent to a C-terminal amino acid residue of a transmembrane domain in a 38554, 57301 or 58324 protein. For example, a C-terminal cytoplasmic domain is located at about amino acid residues 665 to 712 of SEQ ID NO:2 or SEQ ID NO:23, 516 to 553 of SEQ ID NO:5, and 692 to 719 of SEQ ID NO:8.

In a preferred embodiment, a 38554, 57301 or 58324 polypeptide or protein has a C-terminal cytoplasmic domain or a region which includes at least about 5, preferably about 15 to 60, and more preferably about 25 to 55 amino acid residues and has at least about 60%, 70% 80% 90% 95%, 99%, or 100% homology with a C-terminal cytoplasmic domain," *e.g.*, the C-terminal cytoplasmic domain of human 38554, 57301 or 58324 *(e.g.,* residues 665 to 712 of SEQ ID NO:2 or SEQ ID NO:23, 516 to 553 of SEQ ID NO:5, and 692 to 719 of SEQ ID NO:8).

In another embodiment, a 38554, 57301 or 58324 protein includes at least one, two, three, four, preferably five cytoplasmic loops. As used herein, the term "loop" includes an amino acid sequence that resides outside of a phospholipid membrane, having a length of at least about 4, preferably about 5 to 150, more preferably about 6 to 120 amino acid residues, and has an amino acid sequence that connects two transmembrane domains within a protein or polypeptide. Accordingly, the N-terminal amino acid of a loop is adjacent to a C-terminal amino acid of a transmembrane domain in a 38554, 57301 or 58324 molecule, and the C-terminal amino acid of a loop is adjacent to an N-terminal amino acid of a transmembrane domain in a 38554, 57301, or 58324 molecule. As used herein, a "cytoplasmic loop" includes a loop located inside of a cell or within the cytoplasm of a cell. For example, a "cytoplasmic loop" can be found at about amino acid residues 103 to 110, 213 to 220, 296 to 353, 415 to 426, and 578 to 587 of SEQ ID NO:2 or SEQ ID NO:23; 168 to 173, 223 to 231, 280 to 351, 401 to 408, and 456 to 465 of SEQ ID NO:5; 167 to 172, 255 to 264, 336 to 371, 445 to 456, and 604 to 613 of SEQ ID NO:8.

In a preferred embodiment, a 38554, 57301 or 58324 polypeptide or protein has a cytoplasmic loop or a region which includes at least about 4, preferably about 5 to 100, and more preferably about 6 to 80 amino acid residues and has at least about 60%, 70% 80% 90% 95%, 99%, or 100% homology with a cytoplasmic loop," *e.g.*, a cytoplasmic loop of human 38554, 57301 or 58324 (*e.g.*, residues 103 to 110, 213 to 220, 296 to 353, 415 to 426, and 578 to 587 of SEQ ID NO:2 or SEQ ID NO:23; 168 to 173, 223 to 231, 280 to 351, 401 to 408, and 456 to 465 of SEQ ID NO:5; 167 to 172, 255 to 264, 336 to 371, 445 to 456, and 604 to 613 of SEQ ID NO:8).

In another embodiment, a 38554, 57301 or 58324 protein includes at least one, two, three, four, five, preferably six non-cytoplasmic loops. As used herein, a "non-cytoplasmic loop" includes an amino acid sequence located outside of a cell or within an intracellular organelle. Non-cytoplasmic loops include extracellular domains (*i.e.*, outside of the cell) and intracellular domains (*i.e.*, within the cell). When referring to membrane-bound proteins found in intracellular organelles (*e.g*., mitochondria, endoplasmic reticulum, peroxisomes microsomes, vesicles, endosomes, and lysosomes), non-cytoplasmic loops include those domains of the protein that reside in the lumen of the organelle or the matrix or the intermembrane space. For example, a "non-cytoplasmic loop" can be found at about amino acid residues 59 to 79, 129 to 189, 246 to 273, 374 to 392, 447 to 452, and 613 to 640 of SEQ ID NO:2 or SEQ ID NO:23; 38 to 150, 197 to 203, 256 to 262, 370 to 377, 427 to 435, and 487 to 494 of SEQ ID NO:5; 127 to 149, 194 to 230, 290 to 313, 392 to 419, 476 to 579, and 636 to 666 of SEQ ID NO:8.

In a preferred embodiment, a 38554, 57301 or 58324 polypeptide or protein has at least one non-cytoplasmic loop or a region which includes at least about 4, preferably about 5 to 150, more preferably about 6 to 120 amino acid residues and has at least about 60%, 70% 80% 90% 95%, 99%, or 100% homology with a "non-cytoplasmic loop," *e.g.*, at least one non-cytoplasmic loop of human 38554, 57301, or 58324 *(e.g.,* residues 59 to 79, 129 to 189, 246 to 273, 374 to 392, 447 to 452, and 613 to 640 of SEQ ID NO:2 or SEQ ID NO:23; 38 to 150, 197 to 203, 256 to 262, 370 to 377, 427 to 435, and 487 to 494 of SEQ ID NO:5; 127 to 149, 194 to 230, 290 to 313, 392 to 419, 476 to 579, and 636 to 666 of SEQ ID NO:8).

A 38554, 57301 or 58324 protein or polypeptide can include a "transporter domain" or a region homologous to a "transporter domain." As used herein, the term "transporter domain" includes an amino acid sequence of about 20 to 250 amino acid residues in length, resides in a non-cytoplasmic loop and participates in the transport of a molecule, *e.g.* an ion, (*e.g.*, an organic anion or cation, a hormone or a metabolite) across a membrane, *e.g.* a cell or organelle membrane and can have a bit score (PSI-BLAST) for the alignment of the sequence to a transporter domain of at least 80. Preferably, a transporter domain includes at least about 30 to 225 amino acids, more preferably about 35 to 215 amino acid residues, or about 40 to 195 amino acids and has a bit score for the alignment of the sequence to a transporter domain (PSI-BLAST) of at least 100, 120, 135 or greater. The transporter domain of 38554 and 58324 is homologous to ProDom family PD005488 ("Transporter Protein Transmembrane Transport Similar Matrin F/G Organic Anion Sodium-Independent;" SEQ ID NO:12, ProDomain Release 2000.1; http://www.toulouseinrafr/prodom.html; see also ProDomain No. PD005488, Release 1999.2). An alignment of this domain of 38554 (amino acids 476 to 667 of SEQ ID NO:2 or SEQ ID NO:23) with PD005488 resulted in 44% identity as determined by PSI-BLAST. An alignment of this domain of 58324 (amino acids 502 to 672 of SEQ ID NO:8) with PD005488 resulted in 32% identity as determined by PSI-BLAST. The transporter domain of 57301 is homologous to ProDom family PD151320 ("Organic Transporter-like Transport Protein Renal Anion Transporter Cationic Kidney-Specific Solute," SEQ ID NO:15, ProDomain Release 1999.2; http://www.toulouse.inra.fr/prodom.html). An alignment of this region (amino acids 102 to 145 of SEQ ID NO:5) with PD151320 resulted in 56% identity as determined by PSI-BLAST.

In a preferred embodiment a 38554, 57301 or 58324 polypeptide or protein has a "transporter domain" or a region which includes at least about 30 to 225 amino acids, more preferably about 35 to 215 amino acid residues, or about 40 to 195 amino acid residues and has at least about 60%, 70% 80% 90% 95%, 99%, or 100% homology with a "transporter domain," *e.g.*, the transporter domain of human 38554, 57301 or 58324, (*e.g.*, residues 476 to 667 of SEQ ID NO:2 or SEQ ID NO:23, 102 to 145 of SEQ ID NO:5, or 502 to 672 of SEQ ID NO:8).

For further identification of a transporter domain in a 38554, 57301, or 58324 protein sequence, and make the determination that a polypeptide or protein of interest has a particular profile, the amino acid sequence of the protein can be searched against a database of domains, *e.g.*, the ProDom database (Corpet *et al.* (1999), *Nucl. Acids Res.* 27:263-267). The ProDom protein domain database consists of an automatic compilation of homologous domains. Current versions of ProDom are built using recursive PSI-BLAST searches (Altschul SF *et al.* (1997) *Nucleic Acids Res.* 25:3389-3402; Gouzy *et al.* (1999) *Computers and Chemistry* 23:333-340) of the SWISS-PROT 38 and TREMBL protein databases. The database automatically generates a consensus sequence for each domain. A BLAST search was performed against the HMM database resulting in the identification of a "Organic Transporter-like Transport Protein Renal Anion Transporter Cationic Kidney-Specific Solute" domain in the amino acid sequence of human 57301 at about residues 102 to 145 of SEQ ID NO:5 and a "Transporter Protein Transmembrane Transport Similar Matrin F/G Organic Anion Sodium-Independent" domain in the amino acid sequence of human 38554 and 58324 at about residues 476 to 667 of SEQ ID NO:2 or SEQ ID NO:23, or 502 to 672 of SEQ ID NO:8.

A 57301 polypeptide can further include a "sugar (and other) transporter domain" or regions homologous with a "sugar (and other) transporter domain" (SEQ ID NO: 14, PFAM Accession Number PF00083, http;//genome.wustl.edu/Pfam/.html). As used herein, the term "sugar (and other) transporter domain" includes an amino acid sequence of about 420 to 440 amino acid residues in length and transports molecules, *e.g.*, ions, sugars or metabolites. An alignment of the sugar (and other) transporter domain (amino acids 106 to 530 of SEQ ID NO:5) of human 57301 with a consensus amino acid sequence (SEQ ID NO:14) derived from a hidden Markov model yields a bit score of -15.3.

Sugar (and other) transporter domains can have sequences similar to three Prosite signature sequences (two copies of PS00216 and one copy of PS00217). A sequence similar to copy one of the first Prosite signature sequence (PS00216, [LIVMSTAG]-[LIVMFSAG]-x(2)-[LIVMSA]-[DE]-x-[LIVMFYWA]-G-R-[RIG]-x(4,6)-[GSTA], SEQ ID NO:20), with a mismatch at only the first residue of the consensus, is located about between the second and third transmembrane domains of the human 57301 polypeptide and can be found at about amino acids 163 to 179 of SEQ ID NO:5. A sequence similar to copy two of the first Prosite signature sequence, with a mismatch of only an S instead of the [DE], is located about between the eighth and ninth transmembrane domains of the human 57301 polypeptide and can be found at about amino acids 396 to 411 of SEQ ID NO:5. These signature sequences are involved in the conformational change required for transport. A sequence similar to the second Prosite signature sequence (PS00217, [LIVMF]-x-G-[LIVMFA]-x(2)-G-x(8)-[LIFY]-x(2)-[EQ]-x(6)-[RK], SEQ ID NO:21), with a conserved substitution of an A for the first G, a one amino acid insertion after the fourth residue of the consensus, and only one amino acid between the [LIFY] and the [EQ], is located about the end of the fourth and in the loop before the fifth transmembrane domain of the human 57301 polypeptide and can be found at about amino acids 205 to 230 of SEQ ID NO:5. In the above conserved motifs, and other motifs described herein, the standard IUPAC one-letter code for the amino acids is used. Each element in the pattern is separated by a dash (-); square brackets ([ ]) indicate the particular residues that are accepted at that position; x indicates that any residue is accepted at that position; and numbers in parentheses (()) indicate the number of residues represented by the accompanying amino acid.

A 38554 or 58324 molecule can include a kazal domain or regions homologous with a "kazal domain" (PFAM Accession Number PF00050, SEQ ID NO:10 (http;//genome.wustl.edu/Pfam/.html). As used herein, the term "kazal domain" includes an amino acid sequence of about 45 to 55 amino acid residues in length and is characterized by the pattern of cysteine residues, required for disulfide bonding into a specific structure used for contact with the substrate. The kazal domain (HMM) has been assigned the SMART identifier kazal (SEQ ID NO:11, http://smart.embl-heidelberg.de/). An alignment of the kazal domain (amino acids 476 to 523 of SEQ ID NO:2 or SEQ ID NO:23, or 502 to 549 of SEQ ID NO:8) of human 38554 or 58324, respectively, with a consensus amino acid sequence (SEQ ID NO:10) derived from a hidden Markov model yields a bit score of-7.7 and -13.8, respectively. An alignment of the kazal domain (amino acids 475 to 523 of SEQ ID NO:2 or SEQ ID NO:23) of human 38554 with a SMART consensus amino acid sequence (SEQ ID NO:11) derived from modular architecture analysis yields a bit score of-1.8.

To identify the presence of a "sugar (and other) transporter" domain or a "kazal" domain in a 38554, 57301, or 58324 protein sequence, and make the determination that a polypeptide or protein of interest has a particular profile, the amino acid sequence of the protein can be searched against the Pfam database of HMMs (*e.g.*, the Pfam database, release 2.1) using the default parameters
(http://www.sanger.ac.uk/Software/Pfam/HMM_search). For example, the hmmsf program, which is available as part of the HMMER package of search programs, is a family specific default program for MILPAT0063 and a score of 15 is the default threshold score for determining a hit. Alternatively, the threshold score for determining a hit can be lowered (*e.g.*, to 8 bits). A description of the Pfam database can be found in Sonhammer *et al.* (1997) *Proteins* 28:405-420 and a detailed description of HMMs can be found, for example, in Gribskov *et al.* (1990) *Meth. Enzymol.* 183:146-159; Gribskov *et al.* (1987) *Proc. Natl. Acad. Sci. USA* 84:4355-4358; Krogh *et al.* (1994) *J. Mol. Biol.* 235:1501-1531; and Stultz *et al.* (1993) *Protein Sci.* 2:305-314, the contents of which are incorporated herein by reference. A search was performed against the HMM database resulting in the identification of a "sugar (and other) transporter" domain in the amino acid sequence of human 57301 at about residues 106 to 530 of SEQ ID NO:5; a "kazal" domain in the amino acid sequence of human 38554 at about residues 476 to 523 of SEQ ID NO:2 or SEQ ID NO:23; and a "kazal" domain in the amino acid sequence of human 58324 at about residues 502 to 549 of SEQ ID NO:8.

An additional method to identify the presence of a "kazal" domain in a 38554 protein sequence, and make the determination that a polypeptide or protein of interest has a particular profile, the amino acid sequence of the protein can be searched against a SMART database (Simple Modular Architecture Research Tool, http://smart.embl-heidelbergde/) of HMMs as described in Schultz *et al.* (1998), *Proc. Natl. Acad. Sci. USA* 95:5857 and Schultz *et al.* (2000) *Nucl. Acids Res* 28:231. The database contains domains identified by profiling with the hidden Markov models of the HMMer2 search program (R. Durbin *et al.* (1998) *Biological sequence analysis: probabilistic models of proteins and nucleic acids.* Cambridge University Press.; http://hmmer.wustl.edu/). The database also is extensively annotated and monitored by experts to enhance accuracy. A search was performed against the HMM database resulting in the identification of a "kazal" domain in the amino acid sequence of 38554 at about residues 475 to 523 of SEQ ID NO:2 or SEQ ID NO:23.

A 38554, 57301 or 58324 family member can include at least one, two, three, four, five, six, seven, eight, nine, ten, eleven, preferably twelve transmembrane domains; at least one, two, three, four, five, six, preferably seven cytoplasmic regions, including N- and C-terminal cytoplasmic domains and at least one, two, three, four, preferably five cytoplasmic loops; and at least one, two, three, four, five, preferably six non-cytoplasmic loops. Additionally, a 38554 or a 58324 family member can include at least one kazal domain and a 57301 family member can include a sugar (and other) transporter domain. A 38554 family member can further include at least one at least one peroxisomal targeting signal (PSORT PTS2).

Furthermore, a 38554 family member can include at least one, five, ten, preferably thirteen protein kinase C phosphorylation sites (PS00005); at least one, three, six, and preferably eleven casein kinase II phosphorylation sites (PS00006); at least one, two, four, and preferably six N-glycosylation sites (PS00001); at least one tyrosine kinase phosphorylation site (PS00007); and at least one, five, ten, and preferably twelve N-myristoylation sites (PS00008). A 57301 family member can include at least one, two, three, preferably four protein kinase C phosphorylation sites (PS00005); at least one, two, three, and preferably four casein kinase II phosphorylation sites (PS00006); at least one, two, preferably three N-glycosylation sites (PS00001); at least one, preferably two cAMP-and cGMP-dependent protein kinase phosphorylation sites (PS00004); at least one, preferably two amidation sites (PS00009); and at least one, two, four, and preferably eight N-myristoylation sites (PS00008). Furthermore, a 58324 family member can include at least one, two, four, preferably seven protein kinase C phosphorylation sites (PS00005); at least one, two, three, four, and preferably five casein kinase II phosphorylation sites (PS00006); at least one, two, four, and preferably five N-glycosylation sites (PS00001); at least one tyrosine kinase phosphorylation site (PS00007); at least one amidation site (PS00009); and at least one, three, seven and preferably eleven N-myristoylation sites (PS00008).

As the 38554, 57301 or 58324 polypeptides of the invention can modulate 38554-, 57301- or 58324-mediated activities, they can be useful for developing novel diagnostic and therapeutic agents for transporter-associated or other 38554-, 57301- or 58324-associated disorders, as described below.

The SLC21 and SLC22 families are polyspecific transporters of organic ions. Some members of the SLC21 family transport organic anions, others transport prostaglandins. Some members of the SLC22 family transport organic anions, others transport organic cations, and some may transport either type of ion. They participate in activities as diverse as intestinal or hepatic absorption of metabolites, renal reabsorption of cations or excretion of cations. Members of these families also transport a wide variety of drugs and xenobiotics, many of which are harmful to the body. In addition, organic ion transporters are responsible for the transport of the metabolites of most lipophilic compounds, *e.g.*, sulfate and glucuronide conjugates (Moller, J. V. and Sheikh, M. I. (1982) *Pharmacol Rev.* 34:315-358; Pritchard, J. B. and Miller, D. S. (1993) *Physiol. Rev.* 73:765-796; Ullrich, K. J. (1997) *J Membr. Biol.* 158:95-107; Ullrich, K. J. and Rumrich, G. (1993) *Clin. Investig.* 71:843-848; Petzinger, E. (1994) *Rev. Physiol. Biochem. Pharmacol.* 123:47-211).

Proper function of members of these families is important for many physiological processes. At the cellular level, aberrant or deficient organic ion transporter activity can detrimentally affect functions such as cellular proliferation, growth, differentiation, or migration. At the tissue level, aberrant or deficient organic ion transporter activity can detrimentally affect inter- or intra-cellular communication; or musculoskeletal function. At the organ level, aberrant or deficient organic ion transporter activity can detrimentally affect kidney, liver or cardiac function. At the organism level, aberrant or deficient organic ion transporter activity can detrimentally affect systemic responses, such as nervous system responses, hormonal responses (*e.g.*, insulin response), or immune responses; and protection of cells from toxic compounds (*e.g.*, carcinogens, toxins, or mutagens).

As used herein, a "38554, 57301 or 58324 activity", "biological activity of 38554, 57301 or 58324" or "functional activity of 38554, 57301 or 58324", refers to an activity exerted by a 38554, 57301 or 58324 protein, polypeptide or nucleic acid molecule on *e.g.*, a 38554-, 57301- or 58324-responsive cell or on a 38554, 57301 or 58324 substrate, *e.g.*, a protein substrate, as determined *in vivo* or *in vitro.* In one embodiment, a 38554, 57301 or 58324 activity is a direct activity, such as an association with a 38554, 57301 or 58324 target molecule. A "target molecule" or "binding partner" is a molecule with which a 38554, 57301 or 58324 protein binds or interacts in nature. In an exemplary embodiment, 38554, 57301 or 58324 is a transporter, *e.g.*, an SLC21 or 22 family organic ion transporter, and thus binds to or interacts in nature with a molecule, *e.g.*, an organic ion.

A 38554, 57301 or 58324 activity can also be an indirect activity, *e.g.*, a cellular signaling activity mediated by interaction of the 38554, 57301 or 58324 protein with a 38554, 57301 or 58324 receptor. Based on the above-described sequence structures and similarities to molecules of known function, the 38554, 57301 or 58324 molecules of the present invention have similar biological activities as SLC21 or 22 family members. For example, the 38554, 57301 or 58324 proteins of the present invention can have one or more of the following activities: (1) the ability to reside within a membrane; (2) the ability to interact with a substrate or target molecule; (3) the ability to transport a substrate or target molecule, *e.g.*, an ion, *e.g.*, an organic ion (*e.g.*, an organic anion, an organic cation, a prostaglandin, a steroidal compound (*e.g.*, estrone-3-sulfate), a bile acid, a drug, a neurotransmitter, a sulfated lipophilic metabolite, a glucuronidated lipophilic metabolite, a polyamine, a carnitine, or a choline) across a membrane; (4) the ability to transport a second substrate or target molecule, *e.g.*, an ion, (*e.g.*, a bicarbonate ion or a dicarboxylate ion), across a membrane; (5) the ability to interact with and/or modulate a second non-transporter protein; (6) the ability to modulate cellular signaling and/or gene transcription (*e.g.*, either directly or indirectly); (7) the ability to protect cells and/or tissues from organic ions; (8) the ability to protect cells and/or tissues from organic anions; (9) the ability to modulate hormonal responses; (10) the ability to modulate metabolism; and (11) the ability to modulate excretion.

The 38554, 57301 or 58324 molecules of the invention can modulate the activities of cells in tissues where they are expressed. For example, 38554 mRNA is expressed at high levels in human brain cortex and hypothalamus tissue and at medium levels in dorsal root ganglion, spinal cord, choroid plexus, and testes. In the neurological tissues, the expression is found on glial cells, with an epithelial cell similarity in choroid plexus. Expression of 38554 mRNA in monkey and rodent neurological tissues confirms the expression found in human neurological tissues. Regulation of expression is found in rodent dorsal root ganglion after axotomy. Also for example, 57301 mRNA is expressed at high levels in kidney and 58324 mRNA is expressed at small levels in hemangioma tissue. Accordingly, the 38554, 57301 or 58324 molecules of the invention can act as therapeutic or diagnostic agents for one or more of a pain disorder, a nervous system disorder, an immune, *e.g.*, inflammatory disorder, a testicular disorder, a kidney disorder, or an angiogenesis disorder, as well as disorders in tissues where 38554 molecules are expressed at lower levels as described below. Small amounts of 38554 expression were found in normal artery, human umbilical vein endothelial cells, hemangioma tissue, tissue from heart undergoing congestive heart failure, and kidney. Trace amounts of 32468 expression were found in salivary glands, normal colon, colon tumor, normal lung, normal tonsil, mammary gland and pancreas.

The 38554, 57301 or 58324 molecules of the invention can play an important role in pain disorders. In addition, the 38554 molecules of the invention can be used to treat and/or diagnose pain disorders in part because 38554 mRNA is expressed in glial cells, cells with important roles in neuropathic pain and/or because 38554 expression is regulated in the dorsal root ganglion after axotomy. Examples of pain disorders include, but are not limited to, pain response elicited during various forms of tissue injury, *e.g.*, inflammation, infection, and ischemia, usually referred to as hyperalgesia (described in, for example, Fields, H.L. (1987) *Pain,* New York:McGraw-Hill); pain associated with musculoskeletal disorders, *e.g.*, joint pain, tooth pain, headaches, pain associated with surgery, pain related to irritable bowel syndrome, chest pain, pain from rheumatoid arthritis, pain from viral infection, pain from allergic reaction, pain from asthma, chronic pain, pain from chronic pancreatitis, pain from somatoform disorders, pain from fibromyalgia syndrome; and neuropathic pain, allodynia, and hyperesthesia.

The 38554 molecules can be used to treat neurological disorders in part because the 38554 mRNA is expressed in the brain cortex, hypothalamus tissue, dorsal root ganglion, spinal cord, and choroid plexus. Examples of CNS or neurological disorders such as cognitive and neurodegenerative disorders, include, but are not limited to, autonomic function disorders such as hypertension and sleep disorders, and neuropsychiatric disorders, such as depression, schizophrenia, schizoaffective disorder, Korsakoff's psychosis, anxiety disorders, or phobic disorders; learning or memory disorders, *e.g.*, amnesia or age-related memory loss, attention deficit disorder, dysthymic disorder, major depressive disorder, mania, obsessive-compulsive disorder, psychoactive substance use disorders, anxiety, phobias, panic disorder, as well as bipolar affective disorder, *e.g.*, severe bipolar affective (mood) disorder (BP-1), and bipolar affective neurological disorders, *e.g.*, migraine and obesity. Such neurological disorders include, for example, disorders involving neurons, and disorders involving glia, such as astrocytes, oligodendrocytes, ependymal cells, and microglia; cerebral edema, raised intracranial pressure and herniation, and hydrocephalus; malformations and developmental diseases, such as neural tube defects, forebrain anomalies, posterior fossa anomalies, and syringomyelia and hydromyelia; perinatal brain injury; cerebrovascular diseases, such as those related to hypoxia, ischemia, and infarction, including hypotension, hypoperfusion, and low-flow states--global cerebral ischemia and focal cerebral ischemia--infarction from obstruction of local blood supply, intracranial hemorrhage, including intracerebral (intraparenchymal) hemorrhage, subarachnoid hemorrhage and ruptured berry aneurysms, and vascular malformations, hypertensive cerebrovascular disease, including lacunar infarcts, slit hemorrhages, and hypertensive encephalopathy; infections, such as acute meningitis, including acute pyogenic (bacterial) meningitis and acute aseptic (viral) meningitis, acute focal suppurative infections, including brain abscess, subdural empyema, and extradural abscess, chronic bacterial meningoencephalitis, including tuberculosis and mycobacterioses, neurosyphilis, and neuroborreliosis (Lyme disease), viral meningoencephalitis, including arthropod-borne (Arbo) viral encephalitis, *Herpes simplex* virus Type 1, *Herpes simplex* virus Type 2, *Varicella-zoster* virus (*Herpes zoster*), cytomegalovirus, poliomyelitis, rabies, and human immunodeficiency virus 1, including HIV-1 meningoencephalitis (subacute encephalitis), vacuolar myelopathy, AIDS-associated myopathy, peripheral neuropathy, and AIDS in children, progressive multifocal leukoencephalopathy, subacute sclerosing panencephalitis, fungal meningoencephalitis, other infectious diseases of the nervous system; transmissible spongiform encephalopathies (prion diseases); demyelinating diseases, including multiple sclerosis, multiple sclerosis variants, acute disseminated encephalomyelitis and acute necrotizing hemorrhagic encephalomyelitis, and other diseases with demyelination; degenerative diseases, such as degenerative diseases affecting the cerebral cortex, including Alzheimer's disease and Pick's disease, degenerative diseases of basal ganglia and brain stem, including Parkinsonism, idiopathic Parkinson's disease (paralysis agitans) and other Lewy diffuse body diseases, progressive supranuclear palsy, corticobasal degenration, multiple system atrophy, including striatonigral degenration, Shy-Drager syndrome, and olivopontocerebellar atrophy, and Huntington's disease, senile dementia, Gilles de la Tourette's syndrome, epilepsy, and Jakob-Creutzfieldt disease; spinocerebellar degenerations, including spinocerebellar ataxias, including Friedreich ataxia, and ataxiatelanglectasia, degenerative diseases affecting motor neurons, including amyotrophic lateral sclerosis (motor neuron disease), bulbospinal atrophy (Kennedy syndrome), and spinal muscular atrophy; inborn errors of metabolism, such as leukodystrophies, including Krabbe disease, metachromatic leukodystrophy, adrenoleukodystrophy, Pelizaeus-Merzbacher disease, and Canavan disease, mitochondrial encephalomyopathies, including Leigh disease and other mitochondrial encephalomyopathies; toxic and acquired metabolic diseases, including vitamin deficiencies such as thiamine (vitamin B₁) deficiency and vitamin B₁₂ deficiency, neurologic sequelae of metabolic disturbances, including hypoglycemia, hyperglycemia, and hepatic encephatopathy, toxic disorders, including carbon monoxide, methanol, ethanol, and radiation, including combined methotrexate and radiation-induced injury; tumors, such as gliomas, including astrocytoma, including fibrillary (diffuse) astrocytoma and glioblastoma multiforme, pilocytic astrocytoma, pleomorphic xanthoastrocytoma, and brain stem glioma, oligodendroglioma, and ependymoma and related paraventricular mass lesions, neuronal tumors, poorly differentiated neoplasms, including medulloblastoma, other parenchymal tumors, including primary brain lymphoma, germ cell tumors, and pineal parenchymal tumors, meningiomas, metastatic tumors, paraneoplastic syndromes, peripheral nerve sheath tumors, including schwannoma, neurofibroma, and malignant peripheral nerve sheath tumor (malignant schwannoma), and neurocutaneous syndromes (phakomatoses), including neurofibromotosis, including Type 1 neurofibromatosis (NF1) and TYPE 2 neurofibromatosis (NF2), tuberous sclerosis, and Von Hippel-Lindau disease. Further CNS-related disorders include, for example, those listed in the American Psychiatric Association's Diagnostic and Statistical manual of Mental Disorders (DSM), the most current version of which is incorporated herein by reference in its entirety.

The 38554 molecules of the invention can be used to treat and/or diagnose a variety of immune, *e.g.*, inflammatory disorders in part because the 38554 mRNA is expressed in the choroid plexus. The choroid plexus is responsible for the secretion of the cerebral fluid and is involved in inflammatory responses. Examples of immune disorders or diseases include, but are not limited to, autoimmune diseases (including, for example, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, autoimmune thyroiditis, dermatitis (including atopic dermatitis and eczematous dermatitis), psoriasis, Sjögren's Syndrome, inflammatory bowel disease, *e.g.* Crohn's disease and ulcerative colitis, aphthous ulcer, iritis, conjunctivitis, keratoconjunctivitis, asthma, allergic asthma, chronic obstructive pulmonary disease, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis), graft-versus-host disease, cases of transplantation, and allergy such as, atopic allergy.

The 38554 molecules can be used to treat testicular disorders in part because the 38554 mRNA is expressed in the testis. The blood-testis barrier is analogous to the blood-brain barrier in the physiology of seminiferous tubules and maturation of spermatozoa as they develop into spermatids. Transporter molecules, such as 38554, can play a role in the maintenance of this barrier and supply of ions to the developing spermatids. Disorders involving the testis and epididymis include, but are not limited to, congenital anomalies such as cryptorchidism, regressive changes such as atrophy, inflammations such as nonspecific epididymitis and orchitis, granulomatous (autoimmune) orchitis, and specific inflammations including, but not limited to, gonorrhea, mumps, tuberculosis, and syphilis, vascular disturbances including torsion, testicular tumors including germ cell tumors that include, but are not limited to, seminoma, spermatocytic seminoma, embryonal carcinoma, yolk sac tumor choriocarcinoma, teratoma, and mixed tumors, tumore of sex cord-gonadal stroma including, but not limited to, Leydig (interstitial) cell tumors and sertoli cell tumors (androblastoma), and testicular lymphoma, and miscellaneous lesions of tunica vaginalis.

The 57301 molecules can be used to treat renal disorders in part because the 57301 mRNA is expressed in the kidney. Disorders involving the kidney include, but are not limited to, congenital anomalies including, but not limited to, cystic diseases of the kidney, that include but are not limited to, cystic renal dysplasia, polycystic kidney diseases, and cystic diseases of renal medulla; glomerular diseases including pathologies of glomerular injury that include, but are not limited to, in situ immune complex deposition, that includes, but is not limited to, anti-GBM nephritis, Heymann nephritis and other nephritis conditions, glomerulonephritis conditions, minimal change disease (lipoid nephrosis), focal segmental glomerulosclerosis, IgA nephropathy (Berger disease); glomerular lesions associated with systemic disease, including but not limited to, systemic lupus erythematosus, Henoch-Schonlein purpura, bacterial endocarditis, diabetic glomerulosclerosis, amyloidosis, fibrillary and immunotactoid glomerulonephritis, and other systemic disorders; diseases affecting tubules and interstitium, including acute tubular necrosis and tubulointerstitial nephritis, including but not limited to, pyelonephritis and urinary tract infection, acute pyelonephritis, chronic pyelonephritis and reflux nephropathy, and tubulointerstitial nephritis induced by drugs and toxins, and other tubulointerstitial diseases including, but not limited to, urate nephropathy, hypercalcemia and nephrocalcinosis, and multiple myeloma; diseases of blood vessels including benign nephrosclerosis, malignant hypertension and accelerated nephrosclerosis, renal artery stenosis, and thrombotic microangiopathies including, but not limited to, hemolytic-uremic syndromes, and other vascular disorders including, but not limited to, atherosclerotic ischemic renal disease, atheroembolic renal disease, sickle cell disease nephropathy, diffuse cortical necrosis, and renal infarcts; urinary tract obstruction (obstructive uropathy); urolithiasis (renal calculi, stones); and tumors of the kidney including, but not limited to, benign tumors, such as renal papillary adenoma, renal fibroma or hamartoma (renomedullary interstitial cell tumor), angiomyolipoma, and oncocytoma, and malignant tumors, including renal cell carcinoma (hypernephroma, adenocarcinoma of kidney), which includes urothelial carcinomas of renal pelvis.

The 38554 and 58324 molecules can be used to treat angiogenic disorders in part because the 38554 mRNA is expressed in normal artery, human umbilical vein endothelial cells, hemangioma tissue, and tissue from heart undergoing congestive heart failure, and 58324 mRNA is expressed in hemangioma tissue. As used herein, an "angiogenic disorder" includes a disease or disorder which affects or is caused by aberrant or deficient angiogenesis. Disorders involving angiogenesis include, but are not limited to, aberrant or excess angiogenesis in tumors such as hemangiomas and Kaposi's sarcoma, von Hippel-Lindau disease, as well as the angiogenesis associated with tumor growth; aberrant or excess angiogenesis in diseases such as a Castleman's disease or fibrodysplasia ossificans progressiva; aberrant or deficient angiogenesis associated with aging, complications of healing certain wounds and complications of diseases such as diabetes and rheumatoid arthritis; or aberrant or deficient angiogenesis associated with hereditary hemorrhagic telangiectasia, autosomal dominant polycystic kidney disease, myelodysplastic syndrome or Klippel-Trenaunay-Weber syndrome.

Thus, the 38554, 57301 or 58324 molecules can act as novel diagnostic targets and therapeutic agents for controlling one or more of a pain disorder, a nervous system disorder, an immune, *e.g.*, inflammatory disorder, a testicular disorder, a kidney disorder, an angiogenesis disorder, as described above, or other organic ion transport, organic ion absorption or excretion, inter- or intra-cellular signaling, and/or hormonal response disorders.

The 38554, 57301 or 58324 protein, fragments thereof, and derivatives and other variants of the sequence in SEQ ID NO:2 or SEQ ID NO:23, SEQ ID NO:5, or SEQ ID NO:8, respectively thereof are collectively referred to as "polypeptides or proteins of the invention" or "38554, 57301 or 58324 polypeptides or proteins". Nucleic acid molecules encoding such polypeptides or proteins are collectively referred to as "nucleic acids of the invention" or "38554, 57301 or 58324 nucleic acids."

As used herein, the term "nucleic acid molecule" includes DNA molecules (*e.g.*, a cDNA or genomic DNA) and RNA molecules (*e.g.*, an mRNA) and analogs of the DNA or RNA generated, *e.g.*, by the use of nucleotide analogs. The nucleic acid molecule can be single-stranded or double-stranded, but preferably is double-stranded DNA.

The term "isolated or purified nucleic acid molecule" includes nucleic acid molecules which are separated from other nucleic acid molecules which are present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (i.e., sequences located at the 5' and/or 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb or 0.1 kb of 5' and/or 3' nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material or culture medium when produced by recombinant techniques, or substantially free of chemical precursors or other chemicals when chemically synthesized.

As used herein, the term "hybridizes under low stringency, medium stringency, high stringency, or very high stringency conditions" describes conditions for hybridization and washing. Guidance for performing hybridization reactions can be found in *Current Protocols in Molecular Biology* (1989) John Wiley & Sons, N.Y., 6.3.1-6.3.6, which is incorporated by reference. Aqueous and nonaqueous methods are described in that reference and either can be used. Specific hybridization conditions referred to herein are as follows: 1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); 2) medium stringency hybridization conditions in 6X SSC at about 45°C, followed by one or more washes in 0.2X SSC, 0.1% SDS at 60°C; 3) high stringency hybridization conditions in 6X SSC at about 45°C, followed by one or more washes in 0.2X SSC, 0.1% SDS at 65°C; and preferably 4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

As used herein, a "naturally-occurring" nucleic acid molecule refers to an RNA or DNA molecule having a nucleotide sequence that occurs in nature (*e.g.*, encodes a natural protein).

As used herein, the terms "gene" and "recombinant gene" refer to nucleic acid molecules which include an open reading frame encoding a 38554, 57301 or 58324 protein, preferably a mammalian 38554, 57301 or 58324 protein, and can further include non-coding regulatory sequences, and introns.

An "isolated" or "purified" polypeptide or protein is substantially free of cellular material or other contaminating proteins from the cell or tissue source from which the protein is derived, or substantially free from chemical precursors or other chemicals when chemically synthesized. In one embodiment, the language "substantially free" means preparation of 38554, 57301 or 58324 protein having less than about 30%, 20%, 10% and more preferably 5% (by dry weight), of non-38554, 57301 or 58324 protein (also referred to herein as a "contaminating protein"), or of chemical precursors or non-38554, 57301 or 58324 chemicals. When the 38554, 57301 or 58324 protein or biologically active portion thereof is recombinantly produced, it is also preferably substantially free of culture medium, i.e., culture medium represents less than about 20%, more preferably less than about 10%, and most preferably less than about 5% of the volume of the protein preparation. The invention includes isolated or purified preparations of at least 0.01, 0.1, 1.0, and 10 milligrams in dry weight.

A "non-essential" amino acid residue is a residue that can be altered from the wild-type sequence of 38554, 57301 or 58324 (*e.g.*, the sequence of SEQ ID NO:1, 3, 22, 24, 4, 6, 7, or 9) without abolishing or more preferably, without substantially altering a biological activity, whereas an "essential" amino acid residue results in such a change. For example, amino acid residues that are conserved among the polypeptides of the present invention, *e.g.*, those present in the sugar (and other) transporter domain, are predicted to be particularly unamenable to alteration.

A "conservative amino acid substitution" is one in which the amino acid residue is replaced with an amino acid residue having a similar side chain. Families of amino acid residues having similar side chains have been defined in the art. These families include amino acids with basic side chains (*e.g.*, lysine, arginine, histidine), acidic side chains (*e.g.*, aspartic acid, glutamic acid), uncharged polar side chains (*e.g.*, glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.*, threonine, valine, isoleucine) and aromatic side chains *(e.g.,* tyrosine, phenylalanine, tryptophan, histidine). Thus, a predicted nonessential amino acid residue in a 38554, 57301 or 58324 protein is preferably replaced with another amino acid residue from the same side chain family. Alternatively, in another embodiment, mutations can be introduced randomly along all or part of a 38554, 57301 or 58324 coding sequence, such as by saturation mutagenesis, and the resultant mutants can be screened for 38554, 57301 or 58324 biological activity to identify mutants that retain activity. Following mutagenesis of SEQ ID NO:1, SEQ ID NO:3, SEQ ID NO:4, SEQ ID NO:6, SEQ ID NO:7, SEQ ID NO:9, SEQ ID NO:22, or SEQ ID NO:24 the encoded protein can be expressed recombinantly and the activity of the protein can be determined.

As used herein, a "biologically active portion" of a 38554, 57301 or 58324 protein includes a fragment of a 38554, 57301 or 58324 protein which participates in an interaction between a 38554, 57301 or 58324 molecule and a non-38554, 57301 or 58324 molecule. Biologically active portions of a 38554, 57301 or 58324 protein include peptides comprising amino acid sequences sufficiently homologous to or derived from the amino acid sequence of the 38554, 57301 or 58324 protein, *e.g.*, the amino acid sequence shown in SEQ ID NO:2 or SEQ ID NO:23, SEQ ID NO:5, or SEQ ID NO:8, which include fewer amino acids than the full length 38554, 57301 or 58324 protein, and exhibit at least one activity of a 38554, 57301 or 58324 protein. Typically, biologically active portions comprise a domain or motif with at least one activity of the 38554, 57301 or 58324 protein, *e.g.*, organic ion transporter activity. A biologically active portion of a 38554, 57301 or 58324 protein can be a polypeptide which is, for example, 10, 25, 50, 100, 200 or more amino acids in length. Biologically active portions of a 38554, 57301 or 58324 protein can be used as targets for developing agents which modulate a 38554-, 57301- or 58324-mediated activity, *e.g.*, organic ion transporter activity.

Calculations of homology or sequence identity (the terms "homology" and "identity" are used interchangeably herein) between sequences are performed as follows:

To determine the percent identity of two amino acid sequences, or of two nucleic acid sequences, the sequences are aligned for optimal comparison purposes (*e.g.*, gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence (*e.g.*, when aligning a second sequence to the 58324 amino acid sequence of SEQ ID NO:8 having 719 amino acid residues, at least [30%] 215, preferably at least [40%] 287 , more preferably at least [50%] 359, even more preferably at least [60%] 431, and even more preferably at least [70%] 503, [80%] 575, or [90%] 647 amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch (1970) *J. Mol. Biol.* 48:444-453 algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used if the practitioner is uncertain about what parameters should be applied to determine if a molecule is within a sequence identity or homology limitation of the invention) are a Blossum 62 scoring matrix with a gap penalty of 12, a gap extend penalty of 4, and a frameshift gap penalty of 5.

The percent identity between two amino acid or nucleotide sequences can be determined using the algorithm of E. Meyers and W. Miller ((1989) CABIOS, 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, *et al.* (1990) *J*. *Mol. Biol.* 215:403-10. BLAST nucleotide searches can be performed with the NBLAST program, score = 100, wordlength = 12 to obtain nucleotide sequences homologous to 38554, 57301 or 58324 nucleic acid molecules of the invention. BLAST protein searches can be performed with the XBLAST program, score = 50, wordlength = 3 to obtain amino acid sequences homologous to 38554, 57301 or 58324 protein molecules of the invention. To obtain gapped alignments for comparison purposes, Gapped BLAST can be utilized as described in Altschul *et al.*, (1997) *Nucleic Acids Res.* 25:3389-3402. When utilizing BLAST and Gapped BLAST programs, the default parameters of the respective programs (*e.g.*, XBLAST and NBLAST) can be used. See http://www.ncbi.nlm.nih.gov.

Particular 38554, 57301 or 58324 polypeptides of the present invention have an amino acid sequence substantially identical to the amino acid sequence of SEQ ID NO:2 or SEQ ID NO:23, SEQ ID NO:5, or SEQ ID NO:8. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 60%, or 65% identity, likely 75% identity, more likely 85%, 86%, 87%, 88%, 89%, 90%, 91%, 92%, 93%, 94%, 95%, 96%, 97%, 98% or 99% identity to SEQ ID NO:2 or SEQ ID NO:23, SEQ ID NO:5, or SEQ ID NO:8 are termed substantially identical.

"Misexpression or aberrant expression", as used herein, refers to a non-wild type pattern of gene expression, at the RNA or protein level. It includes: expression at non-wild type levels, i.e., over or under expression; a pattern of expression that differs from wild type in terms of the time or stage at which the gene is expressed, *e.g.*, increased or decreased expression (as compared with wild type) at a predetermined developmental period or stage; a pattern of expression that differs from wild type in terms of decreased expression (as compared with wild type) in a predetermined cell type or tissue type; a pattern of expression that differs from wild type in terms of the splicing size, amino acid sequence, post-transitional modification, or biological activity of the expressed polypeptide; a pattern of expression that differs from wild type in terms of the effect of an environmental stimulus or extracellular stimulus on expression of the gene, *e.g.,* a pattern of increased or decreased expression (as compared with wild type) in the presence of an increase or decrease in the strength of the stimulus.

"Subject", as used herein, can refer to a mammal, *e.g.*, a human, or to an experimental or animal or disease model. The subject can also be a non-human animal, *e.g.*, a horse, cow, goat, or other domestic animal.

A "purified preparation of cells", as used herein, refers to, in the case of plant or animal cells, an *in vitro* preparation of cells and not an entire intact plant or animal. In the case of cultured cells or microbial cells, it consists of a preparation of at least 10% and more preferably 50% of the subject cells.

Various aspects of the invention are described in further detail below.

### Isolated Nucleic Acid Molecules

In one aspect, the invention provides, an isolated or purified, nucleic acid molecule that encodes a 38554, 57301 or 58324 polypeptide described herein, *e.g.*, a full length 38554, 57301 or 58324 protein or a fragment thereof, *e.g.*, a biologically active portion of 38554, 57301 or 58324 protein. Also included is a nucleic acid fragment suitable for use as a hybridization probe, which can be used, *e.g.*, to identify a nucleic acid molecule encoding a polypeptide of the invention, 38554, 57301 or 58324 mRNA, and fragments suitable for use as primers, *e.g.*, PCR primers for the amplification or mutation of nucleic acid molecules.

In one embodiment, an isolated nucleic acid molecule of the invention includes the nucleotide sequence shown in SEQ ID NO:1, SEQ ID NO:22, or a portion of any of these nucleotide sequences. In one embodiment, the nucleic acid molecule includes sequences encoding the human 38554 protein (i.e., "the coding region" of SEQ ID NO:1, as shown in SEQ ID NO:3), as well as 5' untranslated sequences (nucleotides 1 to 337 of SEQ ID NO:1) and 3' untranslated sequences (nucleotides 2477 to 3220 of SEQ ID NO:1). In another embodiment, the nucleic acid molecule includes sequences encoding the human 38554 protein (i.e., "the coding region" of SEQ ID NO:22, as shown in SEQ ID NO:24), as well as 5' untranslated sequences (nucleotides 1 to 344 of SEQ ID NO:1) and 3' untranslated sequences (nucleotides 2484 to 3227 of SEQ ID NO: 1). Alternatively, the nucleic acid molecule can include only the coding region of SEQ ID NO: 1 or SEQ ID NO:22 (*e.g.*, SEQ ID NO:3 or SEQ ID NO:24, respectively) and, *e.g.*, no flanking sequences which normally accompany the subject sequences. In another embodiment, the nucleic acid molecule encodes a sequence corresponding to a fragment of the protein from about amino acid 476 to 667 of SEQ ID NO:2 or SEQ ID NO:23.

In one embodiment, an isolated nucleic acid molecule of the invention includes the nucleotide sequence shown in SEQ ID NO:4, or a portion of any of this nucleotide sequence. In one embodiment, the nucleic acid molecule includes sequences encoding the human 57301 protein (i.e., "the coding region" of SEQ ID NO:4, as shown in SEQ ID NO:6), as well as 5' untranslated sequences (nucleotides 1 to 364 of SEQ ID NO:4) and 3' untranslated sequences (nucleotides 2027 to 2866 of SEQ ID NO:4). Alternatively, the nucleic acid molecule can include only the coding region of SEQ ID NO:4 (*e.g.*, SEQ ID NO:6) and, *e.g.*, no flanking sequences which normally accompany the subject sequence. In another embodiment, the nucleic acid molecule encodes a sequence corresponding to a fragment of the protein from about amino acid 102 to 145 of SEQ ID NO:5.

In one embodiment, an isolated nucleic acid molecule of the invention includes the nucleotide sequence shown in SEQ ID NO:7, or a portion of any of these nucleotide sequences. In one embodiment, the nucleic acid molecule includes sequences encoding the human 58324 protein (i.e., "the coding region" of SEQ ID NO:7, as shown in SEQ ID NO:9), as well as 5' untranslated sequences (nucleotides 1 to 147 of SEQ ID NO:7) and 3' untranslated sequences (nucleotides 2308 to 2480 of SEQ ID NO:7). Alternatively, the nucleic acid molecule can include only the coding region of SEQ ID NO:7 (*e.g.*, SEQ ID NO:9) and, *e.g.*, no flanking sequences which normally accompany the subject sequence. In another embodiment, the nucleic acid molecule encodes a sequence corresponding to a fragment of the protein from about amino acid 502 to 672 of SEQ ID NO:8.

In another embodiment, an isolated nucleic acid molecule of the invention includes a nucleic acid molecule which is a complement of the nucleotide sequence shown in SEQ ID NO:1, SEQ ID NO:3, SEQ ID NO:22, SEQ ID NO:24, SEQ ID NO:4, SEQ ID NO:6, SEQ ID NO:7, or SEQ ID NO:9, or a portion of any of these nucleotide sequences. In other embodiments, the nucleic acid molecule of the invention is sufficiently complementary to the nucleotide sequence shown in SEQ ID NO:1, SEQ ID NO:3, SEQ ID NO:22, SEQ ID NO:24, SEQ ID NO:4, SEQ ID NO:6, SEQ ID NO:7, or SEQ ID NO:9, such that it can hybridize to the nucleotide sequence shown in SEQ ID NO:1, SEQ ID NO:3, SEQ ID NO:22, SEQ ID NO:24, SEQ ID NO:4, SEQ ID NO:6, SEQ ID NO:7, or SEQ ID NO:9, thereby forming a stable duplex.

In one embodiment, an isolated nucleic acid molecule of the present invention includes a nucleotide sequence which is at least about: 60%, 65%, 70%, 75%, 80%, 85%, %86%, 87%, 88%, 89%, 90%, 91%, 92%, 93%, 94%, 95%, 96%, 97%, 98%, 99%, or more homologous to the entire length of the nucleotide sequence shown in SEQ ID NO:1, SEQ ID NO:3, SEQ ID NO:22, SEQ ID NO:24, SEQ ID NO:4, SEQ ID NO:6, SEQ ID NO:7, or SEQ ID NO:9, or a portion, preferably of the same length, of any of these nucleotide sequences.

### 38554, 57301 or 58324 Nucleic Acid Fragments

**[00102]** A nucleic acid molecule of the invention can include only a portion of the nucleic acid sequence of SEQ ID NO:1, SEQ ID NO:3, SEQ ID NO:22, SEQ ID NO:24, SEQ ID NO:4, SEQ ID NO:6, SEQ ID NO:7, or SEQ ID NO:9. For example, such a nucleic acid molecule can include a fragment which can be used as a probe or primer or a fragment encoding a portion of a 38554, 57301 or 58324 protein, *e.g.*, an immunogenic or biologically active portion of a 38554, 57301 or 58324 protein. A fragment can comprise those nucleotides of SEQ ID NO:1, SEQ ID NO:22, SEQ ID NO:4 or SEQ ID NO:7, which encode a transporter domain of human 38554, 57301 or 58324. The nucleotide sequence determined from the cloning of the 38554, 57301 or 58324 gene allows for the generation of probes and primers designed for use in identifying and/or cloning other 38554, 57301 or 58324 family members, or fragments thereof, as well as 38554, 57301 or 58324 homologs, or fragments thereof, from other species.

[00103] In another embodiment, a nucleic acid includes a nucleotide sequence that includes part, or all, of the coding region and extends into either (or both) the 5' or 3' noncoding region. Other embodiments include a fragment which includes a nucleotide sequence encoding an amino acid fragment described herein. Nucleic acid fragments can encode a specific domain or site described herein or fragments thereof, particularly fragments thereof which are at least 40 amino acids in length. Fragments also include nucleic acid sequences corresponding to specific amino acid sequences described above or fragments thereof. For example, a nucleic acid fragment can include a sequence which encodes at least 138 amino acids of SEQ ID NO:8. Nucleic acid fragments should not to be construed as encompassing those fragments that may have been disclosed prior to the invention.

[00104] A nucleic acid fragment can include a sequence corresponding to a domain, region, or functional site described herein. A nucleic acid fragment can also include one or more domain, region, or functional site described herein. Thus, for example, a 38554, 57301 or 58324 nucleic acid fragment can include a sequence corresponding to a transporter domain.

**[00105]** 38554, 57301 or 58324 probes and primers are provided. Typically a probe/primer is an isolated or purified oligonucleotide. The oligonucleotide typically includes a region of nucleotide sequence that hybridizes under stringent conditions to at least about 7, 12 or 15, preferably about 20 or 25, more preferably about 30, 35, 40, 45, 50, 55, 60, 65, or 75 consecutive nucleotides of a sense or antisense sequence of SEQ ID NO:1, SEQ ID NO:3, SEQ ID NO:22, SEQ ID NO:24, SEQ ID NO:4, SEQ ID NO:6, SEQ ID NO:7, or SEQ ID NO:9, or of a naturally occurring allelic variant or mutant of SEQ ID NO:1, SEQ ID NO:3, SEQ ID NO:22, SEQ ID NO:24, SEQ ID NO:4, SEQ ID NO:6, SEQ ID NO:7, or SEQ ID NO:9.

In a preferred embodiment the nucleic acid is a probe which is at least 5 or 10, and less than 200, more preferably less than 100, or less than 50, base pairs in length. It should be identical, or differ by 1, or less than in 5 or 10 bases, from a sequence disclosed herein. If alignment is needed for this comparison the sequences should be aligned for maximum homology. "Looped" out sequences from deletions or insertions, or mismatches, are considered differences.

A probe or primer can be derived from the sense or anti-sense strand of a nucleic acid which encodes: a transmembrane domain at about amino acids 42 to 58, 80 to 102, 111 to 128, 190 to 212, 221 to 245, 274 to 295, 354 to 373, 393 to 414, 427 to 446, 553 to 577, 588 to 612, or 641 to 664 of SEQ ID NO:2 or SEQ ID NO:23; at about amino acids 21 to 37, 151 to 167, 174 to 196, 204 to 222, 232 to 255, 263 to 279, 352 to 369, 378 to 400, 409 to 426, 436 to 455, 466 to 486 or 495 to 515 of SEQ ID NO:5; or at about amino acids 107 to 126, 150 to 166, 173 to 193, 231 to 254, 265 to 289, 314 to 335, 372 to 391, 420 to 444, 457 to 475, 580 to 603, 614 to 635, or 667 to 691 of SEQ ID NO:8; a transporter domain at about amino acid residues 476 to 677 of SEQ ID NO:2 or SEQ ID NO:23, 102 to 145 of SEQ ID NO:5, or at about 502 to 672 of SEQ ID NO:8; a kazal domain at about amino acid residues 476 to 523 of SEQ ID NO:2 or SEQ ID NO:23 or 502 to 549 of SEQ ID NO:8; or a sugar (and other) transporter domain at about amino acid residues 106 to 530 of SEQ ID NO:5.

In another embodiment, a set of primers is provided, *e.g.*, primers suitable for use in a PCR, which can be used to amplify a selected region of a 38554, 57301 or 58324 sequence, *e.g.*, a domain, region, site or other sequence described herein. The primers should be at least 5, 10, or 50 base pairs in length and less than 100, or less than 200, base pairs in length. The primers should be identical, or differ by one base from a sequence disclosed herein or from a naturally occurring variant. For example, primers suitable for amplifying all or a portion of any of the following regions are provided: a transmembrane domain at about amino acids 42 to 58, 80 to 102, 111 to 128, 190 to 212, 221 to 245, 274 to 295, 354 to 373, 393 to 414, 427 to 446, 553 to 577, 588 to 612, or 641 to 664 of SEQ ID NO:2 or SEQ ID NO:23; at about amino acids 21 to 37, 151 to 167, 174 to 196, 204 to 222, 232 to 255, 263 to 279, 352 to 369, 378 to 400, 409 to 426, 436 to 455, 466 to 486 or 495 to 515 of SEQ ID NO:5; or at about amino acids 107 to 126, 150 to 166, 173 to 193, 231 to 254, 265 to 289, 314 to 335, 372 to 391, 420 to 444, 457 to 475, 580 to 603, 614 to 635, or 667 to 691 of SEQ ID NO:8; a transporter domain at about amino acid residues 476 to 677 of SEQ ID NO:2 or SEQ ID NO:23, 102 to 145 of SEQ ID NO:5, or at about 502 to 672 of SEQ ID NO:8; a kazal domain at about amino acid residues 476 to 523 of SEQ ID NO:2 or SEQ ID NO:23, or 502 to 549 of SEQ ID NO:8; or a sugar (and other) transporter domain at about amino acid residues 106 to 530 of SEQ ID NO:5.

A nucleic acid fragment can encode an epitope-bearing region of a polypeptide described herein.

A nucleic acid fragment encoding a "biologically active portion of a 38554, 57301 or 58324 polypeptide" can be prepared by isolating a portion of the nucleotide sequence of SEQ ID NO:1, 3, 22, 24, 4, 6, 7, or 9, which encodes a polypeptide having a 38554, 57301 or 58324 biological activity (*e.g.*, the biological activities of the 38554, 57301 or 58324 proteins are described herein), expressing the encoded portion of the 38554, 57301 or 58324 protein (*e.g.*, by recombinant expression *in vitro)* and assessing the activity of the encoded portion of the 38554, 57301 or 58324 protein. For example, a nucleic acid fragment encoding a biologically active portion of 38554, 57301 or 58324 includes a transporter domain at about amino acid residues 476 to 677 of SEQ ID NO:2 or SEQ ID NO:23, 102 to 145 of SEQ ID NO:5, or at about 502 to 672 of SEQ ID NO:8. A nucleic acid fragment encoding a biologically active portion of a 38554, 57301 or 58324 polypeptide, can comprise a nucleotide sequence which is greater than 120 or more nucleotides in length.

In preferred embodiments, a nucleic acid includes a nucleotide sequence which is about 300, 600, 900, 1200, 1500, 1800, 2100, 2200, 2300, 2400, 2500, 2600, 2700, 2800, 2900, 3000, 3100, 3200 or more nucleotides in length and hybridizes under stringent hybridization conditions to a nucleic acid molecule of SEQ ID NO: 1, SEQ ID NO:3, SEQ ID NO:22, SEQ ID NO:24, SEQ ID NO:4, SEQ ID NO:6, SEQ ID NO:7, or SEQ ID NO:9.

### 38554, 57301 or 58324 Nucleic Acid Variants

The invention further encompasses nucleic acid molecules that differ from the nucleotide sequence shown in SEQ ID NO:1, SEQ ID NO:3, SEQ ID NO:22, SEQ ID NO:24, SEQ ID NO:4, SEQ ID NO:6, SEQ ID NO:7, or SEQ ID NO:9. Such differences can be due to degeneracy of the genetic code (and result in a nucleic acid which encodes the same 38554, 57301 or 58324 proteins as those encoded by the nucleotide sequence disclosed herein. In another embodiment, an isolated nucleic acid molecule of the invention has a nucleotide sequence encoding a protein having an amino acid sequence which differs, by at least 1, but less than 5, 10, 20, 50, or 100 amino acid residues that shown in SEQ ID NO:2, SEQ ID NO:23, SEQ ID NO:5, or SEQ ID NO:8. If alignment is needed for this comparison the sequences should be aligned for maximum homology. "Looped" out sequences from deletions or insertions, or mismatches, are considered differences.

Nucleic acids of the inventor can be chosen for having codons, which are preferred, or non-preferred, for a particular expression system. *E.g.*, the nucleic acid can be one in which at least one codon, at preferably at least 10%, or 20% of the codons has been altered such that the sequence is optimized for expression in *E. coli,* yeast, human, insect, or CHO cells.

Nucleic acid variants can be naturally occurring, such as allelic variants (same locus), homologs (different locus), and orthologs (different organism) or can be non naturally occurring. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product).

In a preferred embodiment, the nucleic acid differs from that of SEQ ID NO:1, SEQ ID NO:3, SEQ ID NO:22, SEQ ID NO:24, SEQ ID NO:4, SEQ ID NO:6, SEQ ID NO:7, or SEQ ID NO:9, *e.g.*, as follows: by at least one but less than 10, 20, 30, or 40 nucleotides; at least one but less than 1%, 5%, 10% or 20% of the nucleotides in the subject nucleic acid. If necessary for this analysis the sequences should be aligned for maximum homology. "Looped" out sequences from deletions or insertions, or mismatches, are considered differences.

Orthologs, homologs, and allelic variants can be identified using methods known in the art. These variants comprise a nucleotide sequence encoding a polypeptide that is 50%, at least about 55%, typically at least about 70-75%, more typically at least about 80-85%, or 86-89% and most typically at least about 90-95% or 96-99% or more identical to the nucleotide sequence shown in SEQ ID NO:2, SEQ ID NO:23, SEQ ID NO:5, or SEQ ID NO:8 or a fragment of this sequence. Such nucleic acid molecules can readily be identified as being able to hybridize under stringent conditions, to the nucleotide sequence shown in SEQ ID NO:2, SEQ ID NO:23, SEQ ID NO:5, or SEQ ID NO:8 or a fragment of the sequence. Nucleic acid molecules corresponding to orthologs, homologs, and allelic variants of the 38554, 57301 or 58324 cDNAs of the invention can further be isolated by mapping to the same chromosome or locus as the 38554, 57301 or 58324 gene.

Preferred variants include those that are correlated with organic ion transporter activity.

Allelic variants of 38554, 57301 or 58324, *e.g.*, human 38554, 57301 or 58324, include both functional and non-functional proteins. Functional allelic variants are naturally occurring amino acid sequence variants of the 38554, 57301 or 58324 protein within a population that maintain the ability to reside within a membrane, *e.g.,* a cell or organelle membrane, transport a substrate or target molecule, *e.g.*, an ion, *e.g.*, an organic ion (*e.g.*, an organic anion, cation, a prostaglandin, or a metabolite) across the membrane, or transport a second substrate or target molecule, *e.g.*, an ion, (*e.g.*, a bicarbonate ion or a dicarboxylate ion), across the membrane. Functional allelic variants will typically contain only conservative substitution of one or more amino acids of SEQ ID NO:2, SEQ ID NO:23, SEQ ID NO:5, or SEQ ID NO:8, or substitution, deletion or insertion of non-critical residues in non-critical regions of the protein. Non-functional allelic variants are naturally-occurring amino acid sequence variants of the 38554, 57301 or 58324, *e.g.*, human 38554, 57301 or 58324, protein within a population that do not have the ability to reside within a membrane, *e.g.,* a cell or organelle membrane, transport a substrate or target molecule, *e.g.*, an ion, *e.g.*, an organic ion (*e.g.*, an organic anion, cation, a prostaglandin, or a metabolite) across the membrane, or transport a second substrate or target molecule, *e.g.*, an ion, (*e.g.*, a bicarbonate ion or a dicarboxylate ion), across the membrane. Non-functional allelic variants will typically contain a non-conservative substitution, a deletion, or insertion, or premature truncation of the amino acid sequence of SEQ ID NO:2, SEQ ID NO:23, SEQ ID NO:5, or SEQ ID NO:8, or a substitution, insertion, or deletion in critical residues or critical regions of the protein.

Moreover, nucleic acid molecules encoding other 38554, 57301 or 58324 family members and, thus, which have a nucleotide sequence which differs from the 38554, 57301 or 58324 sequences of SEQ ID NO:1, SEQ ID NO:3, SEQ ID NO:22, SEQ ID NO:24, SEQ ID NO:4, SEQ ID NO:6, SEQ ID NO:7, or SEQ ID NO:9 are intended to be within the scope of the invention.

### Antisense Nucleic Acid Molecules, Ribozymes and Modified 38554, 57301 or 58324 Nucleic Acid Molecules

In another aspect, the invention features, an isolated nucleic acid molecule which is antisense to 38554, 57301 or 58324. An "antisense" nucleic acid can include a nucleotide sequence which is complementary to a "sense" nucleic acid encoding a protein, *e.g.*, complementary to the coding strand of a double-stranded cDNA molecule or complementary to an mRNA sequence. The antisense nucleic acid can be complementary to an entire 38554, 57301 or 58324 coding strand, or to only a portion thereof (*e.g.*, the coding region of human 38554, 57301 or 58324 corresponding to SEQ ID NO:3, SEQ ID NO:24, SEQ ID NO:6, or SEQ ID NO:9). In another embodiment, the antisense nucleic acid molecule is antisense to a "noncoding region" of the coding strand of a nucleotide sequence encoding 38554, 57301 or 58324 (*e.g.*, the 5' and 3' untranslated regions).

An antisense nucleic acid can be designed such that it is complementary to the entire coding region of 38554, 57301 or 58324 mRNA, but more preferably is an oligonucleotide which is antisense to only a portion of the coding or noncoding region of 38554, 57301 or 58324 mRNA. For example, the antisense oligonucleotide can be complementary to the region surrounding the translation start site of 38554, 57301 or 58324 mRNA, *e.g.*, between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length.

An antisense nucleic acid of the invention can be constructed using chemical synthesis and enzymatic ligation reactions using procedures known in the art. For example, an antisense nucleic acid (*e.g.*, an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g.*, phosphorothioate derivatives and acridine substituted nucleotides can be used. The antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject (*e.g.*, by direct injection at a tissue site), or generated *in situ* such that they hybridize with or bind to cellular mRNA and/or genomic DNA encoding a 38554, 57301 or 58324 protein to thereby inhibit expression of the protein, *e.g.*, by inhibiting transcription and/or translation. Alternatively, antisense nucleic acid molecules can be modified to target selected cells and then administered systemically. For systemic administration, antisense molecules can be modified such that they specifically or selectively bind to receptors or antigens expressed on a selected cell surface, *e.g*., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier *et al.* (1987) *Nucleic Acids. Res.* 15:6625-6641). The antisense nucleic acid molecule can also comprise a 2'-o-methylribonucleotide (Inoue *et al.* (1987) *Nucleic Acids Res.* 15:6131-6148) or a chimeric RNA-DNA analogue (Inoue *et al.* (1987) *FEBS Lett.* 215:327-330).

In still another embodiment, an antisense nucleic acid of the invention is a ribozyme. A ribozyme having specificity for a 38554, 57301 or 58324-encoding nucleic acid can include one or more sequences complementary to the nucleotide sequence of a 38554, 57301 or 58324 cDNA disclosed herein (i.e., SEQ ID NO:1, SEQ ID NO:3, SEQ ID NO:22, SEQ ID NO:24, SEQ ID NO:4, SEQ ID NO:6, SEQ ID NO:7, or SEQ ID NO:9), and a sequence having known catalytic sequence responsible for mRNA cleavage (see U.S. Pat. No. 5,093,246 or Haselhoff and Gerlach (1988) *Nature* 334:585-591). For example, a derivative of a *Tetrahymena* L-19 IVS RNA can be constructed in which the nucleotide sequence of the active site is complementary to the nucleotide sequence to be cleaved in a 38554, 57301 or 58324-encoding mRNA. See, *e.g.*, Cech *et al.* U.S. Patent No. 4,987,071; and Cech *et al.* U.S. Patent No. 5,116,742. Alternatively, 38554, 57301 or 58324 mRNA can be used to select a catalytic RNA having a specific ribonuclease activity from a pool of RNA molecules. See, *e.g.*, Bartel, D. and Szostak, J.W. (1993) *Science* 261:1411-1418.

38554, 57301 or 58324 gene expression can be inhibited by targeting nucleotide sequences complementary to the regulatory region of the 38554, 57301 or 58324 (*e.g.*, the 38554, 57301 or 58324 promoter and/or enhancers) to form triple helical structures that prevent transcription of the 38554, 57301 or 58324 gene in target cells. See generally, Helene, C. (1991) *Anticancer Drug Des.* 6:569-84; Helene, C. (1992) *Ann. N.Y. Acad. Sci.* 660:27-36; and Maher, L.J. (1992) *Bioassays* 14:807-15. The potential sequences that can be targeted for triple helix formation can be increased by creating a so called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

The invention also provides detectably labeled oligonucleotide primer and probe molecules. Typically, such labels are chemiluminescent, fluorescent, radioactive, or colorimetric.

A 38554, 57301 or 58324 nucleic acid molecule can be modified at the base moiety, sugar moiety or phosphate backbone to improve, *e.g.*, the stability, hybridization, or solubility of the molecule. For example, the deoxyribose phosphate backbone of the nucleic acid molecules can be modified to generate peptide nucleic acids (see Hyrup B. *et al.* (1996) *Bioorganic & Medicinal Chemistry* 4: 5-23). As used herein, the terms "peptide nucleic acid" or "PNA" refers to a nucleic acid mimic, *e.g.*, a DNA mimic, in which the deoxyribose phosphate backbone is replaced by a pseudopeptide backbone and only the four natural nucleobases are retained. The neutral backbone of a PNA can allow for specific hybridization to DNA and RNA under conditions of low ionic strength. The synthesis of PNA oligomers can be performed using standard solid phase peptide synthesis protocols as described in Hyrup B. *et al.* (1996) *supra;* Perry-O'Keefe *et a.l* (1996) *Proc. Natl*. *Acad. Sci.* 93: 14670-675.

PNAs of 38554, 57301 or 58324 nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of 38554, 57301 or 58324 nucleic acid molecules can also be used in the analysis of single base pair mutations in a gene, (*e.g.*, by PNA-directed PCR clamping); as 'artificial restriction enzymes' when used in combination with other enzymes, (*e.g.*, S1 nucleases (Hyrup B. *et al.* (1996) *supra));* or as probes or primers for DNA sequencing or hybridization (Hyrup B. *et al.* (1996) *supra;* Perry-O'Keefe *supra).*

In other embodiments, the oligonucleotide can include other appended groups such as peptides (*e.g.*, for targeting host cell receptors *in vivo*), or agents facilitating transport across the cell membrane (see, *e.g.*, Letsinger *et al.* (1989) *Proc. Natl. Acad. Sci. USA* 86:6553-6556; Lemaitre *et al.* (1987) *Proc. Natl. Acad. Sci. USA* 84:648-652; PCT Publication No. W088/09810) or the blood-brain barrier (see, *e.g.*, PCT Publication No. W089/10134). In addition, oligonucleotides can be modified with hybridization-triggered cleavage agents (see, *e.g.*, Krol *et al.* (1988) *Bio-Techniques* 6:958-976) or intercalating agents. (see, *e.g.*, Zon (1988) *Pharm. Res.* 5:539-549). To this end, the oligonucleotide can be conjugated to another molecule, (*e.g.*, a peptide, hybridization triggered cross-linking agent, transport agent, or hybridization-triggered cleavage agent).

The invention also includes molecular beacon oligonucleotide primer and probe molecules having at least one region which is complementary to a 38554, 57301 or 58324 nucleic acid of the invention, two complementary regions one having a fluorophore and one a quencher such that the molecular beacon is useful for quantitating the presence of the 38554, 57301 or 58324 nucleic acid of the invention in a sample. Molecular beacon nucleic acids are described, for example, in Lizardi *et al.*, U.S. Patent No. 5,854,033; Nazarenko *et al.*, U.S. Patent No. 5,866,336, and Livak *et al.*, U.S. Patent 5,876,930.

### Isolated 38554, 57301 or 58324 Polypeptides

In another aspect, the invention features, an isolated 38554, 57301 or 58324 protein, or fragment, *e.g.*, a biologically active portion, for use as immunogens or antigens to raise or test (or more generally to bind) anti-38554, 57301 or 58324 antibodies. 38554, 57301 or 58324 protein can be isolated from cells or tissue sources using standard protein purification techniques. 38554, 57301 or 58324 protein or fragments thereof can be produced by recombinant DNA techniques or synthesized chemically.

Polypeptides of the invention include those which arise as a result of the existence of multiple genes, alternative transcription events, alternative RNA splicing events, and alternative translational and post-translational events. The polypeptide can be expressed in systems, *e.g.*, cultured cells, which result in substantially the same post-translational modifications present when expressed the polypeptide is expressed in a native cell, or in systems which result in the alteration or omission of post-translational modifications, *e.g.,* glycosylation or cleavage, present in a native cell.

In a preferred embodiment, a 38554 polypeptide has one or more of the following characteristics:

it has the ability to transport a substrate or molecule, *e.g.*, an ion, *e.g.*, an organic ion, (*e.g.*, an organic anion, an organic cation, a prostaglandin, a steroidal compound (*e.g.*, estrone-3-sulfate), a bile acid, a drug, a neurotransmitter, a sulfated lipophilic metabolite, a glucuronidated lipophilic metabolite, a polyamine, a carnitine, or a choline), across a membrane;

it has the ability to transport a second substrate or molecule, *e.g.*, an ion, (*e.g.*, a bicarbonate ion or a dicarboxylate ion), across a membrane;

it has a molecular weight, *e.g.*, a deduced molecular weight, preferably ignoring any contribution of post translational modifications, amino acid composition or other physical characteristic of a 38554 polypeptide, *e.g.*, a polypeptide of SEQ ID NO:2 or SEQ ID NO:23;

it has an overall sequence similarity of at least 60%, preferably at least 70%, more preferably at least 80, 90, 95, 96, 97, 98, or 99%, with a polypeptide of SEQ ID NO:2 or SEQ ID NO:23;

it can be found in a membrane or brain cortex, hypothalamus tissue, dorsal root ganglion, spinal cord, choroid plexus, and testis;

it can have a transporter domain which has a sequence similarity preferably about 70%, 80%, 90% 95, 96, 97, 98, or 99% with amino acid residues about 476 to 667 of SEQ ID NO:2 or SEQ ID NO:23;

it can have a kazal domain which has a sequence similarity preferably about 70%, 80%, 90%, 95, 96, 97, 98, or 99% with amino acid residues about 476 to 523 of SEQ ID NO:2 or SEQ ID NO:23; and

its expression can be regulated in the dorsal root ganglion after axotomy.

In a preferred embodiment, a 57301 polypeptide has one or more of the following characteristics:

it has the ability to transport a substrate or molecule, *e.g.*, an ion, *e.g.*, an organic ion, (*e.g.*, an organic anion, an organic cation, a prostaglandin, a steroidal compound (*e.g.*, estrone-3-sulfate), a bile acid, a drug, a neurotransmitter, a sulfated lipophilic metabolite, a glucuronidated lipophilic metabolite, a polyamine, a carnitine, or a choline), across a membrane;

it has the ability to transport a second substrate or molecule, *e.g.*, an ion, (*e.g.*, a bicarbonate ion or a dicarboxylate ion), across a membrane;

it has a molecular weight, *e.g.*, a deduced molecular weight, preferably ignoring any contribution of post translational modifications, amino acid composition or other physical characteristic of a 57301 polypeptide, *e.g.*, a polypeptide of SEQ ID NO:5;

it has an overall sequence similarity of at least 60%, preferably at least 70%, more preferably at least 80, 90, 95, 96, 97, 98, or 99%, with a polypeptide of SEQ ID NO:5;

it can be found in a membrane or in the kidney;

it can have a transporter domain which has a sequence similarity preferably about 70%, 80%, 90%, 95% 96%, 97%, 98%, or 99% with amino acid residues about 102 to 145 of SEQ ID NO:5;

it can have a sugar (and others) transporter domain which has a sequence similarity preferably about 70%, 80%, 90% or 95% with amino acid residues about 106 to 530 of SEQ ID NO:5.

In a preferred embodiment, a 58324 polypeptide has one or more of the following characteristics:

it has the ability to transport a substrate or molecule, *e.g.*, an ion, *e.g.*, an organic ion, *e.g.*, an organic anion, an organic cation, a prostaglandin, a steroidal compound (*e.g.*, estrone-3-sulfate), a bile acid, a drug, a neurotransmitter, a sulfated lipophilic metabolite, a glucuronidated lipophilic metabolite, a polyamine, a carnitine, or a choline, across a membrane;

it has the ability to transport a second substrate or molecule, *e.g.*, an ion, (*e.g.*, a bicarbonate ion or a dicarboxylate ion), across a membrane;

it has a molecular weight, *e.g.*, a deduced molecular weight, preferably ignoring any contribution of post translational modifications, amino acid composition or other physical characteristic of a 58324 polypeptide, *e.g.*, a polypeptide of SEQ ID NO:8;

it has an overall sequence similarity of at least 60%, preferably at least 70%, more preferably at least 80, 90, or 95%, with a polypeptide of SEQ ID NO:8;

it can be found in a membrane or in hemangioma tissue;

it can have a transporter domain which has a sequence similarity preferably about 70%, 80%, 90% or 95% with amino acid residues about 502 to 672 of SEQ ID NO:8;

it can have a kazal domain which has a sequence similarity preferably about 70%, 80%, 90% or 95% with amino acid residues about 502 to 549 of SEQ ID NO:8.

In a preferred embodiment the 38554, 57301 or 58324 protein, or fragment thereof, differs from the corresponding sequence in SEQ ID NO:2, SEQ ID NO:23, SEQ ID NO:5, or SEQ ID NO:8, respectively. In one embodiment it differs by at least one but by less than 15, 10 or 5 amino acid residues. In another it differs from the corresponding sequence in SEQ ID NO:2, SEQ ID NO:23, SEQ ID NO:5, or SEQ ID NO:8 by at least one residue but less than 20%, 15%, 10% or 5% of the residues in it differ from the corresponding sequence in SEQ ID NO:2, SEQ ID NO:23, SEQ ID NO:5, or SEQ ID NO:8. (If this comparison requires alignment the sequences should be aligned for maximum homology. "Looped" out sequences from deletions or insertions, or mismatches, are considered differences.) The differences are, preferably, differences or changes at a non-essential residue or a conservative substitution. In a preferred embodiment the differences are not in the transporter domain located at about amino acid residues 476 to 667 of SEQ ID NO:2 or SEQ ID NO:23, 102 to 145 of SEQ ID NO:5 or 502 to 672 of SEQ ID NO:8. In another embodiment one or more differences are in the transporter domain located at about amino acid residues 476 to 667 of SEQ ID NO:2 or SEQ ID NO:23, 102 to 145 of SEQ ID NO:5 or 502 to 672 of SEQ ID NO:8.

Other embodiments include a protein that contains one or more changes in amino acid sequence, *e.g.*, a change in an amino acid residue which is not essential for activity. Such 38554, 57301, 58324 proteins differ in amino acid sequence from SEQ ID NO:2 or SEQ ID NO:23, SEQ ID NO:5, or SEQ ID NO:8, yet retain biological activity.

In one embodiment, the protein includes an amino acid sequence at least about 60%, 65%, 70%, 75%, 80%, 83%, 84%, 85%, 86%, 87%, 90%, 95%, 96%, 97%, 98%, 99% or more homologous to SEQ ID NO:2, SEQ ID NO:23, SEQ ID NO:5, or SEQ ID NO:8. In another embodiment, the protein includes fragments or regions homologous to fragments, at least about 70%, 80%, 85%, 86%, 87%, 88%, 89%, 90%, 95%, 96%, 97%, 98%, 99% or more homologous to a fragment of SEQ ID NO:2, SEQ ID NO:23, SEQ ID NO:5, or SEQ ID NO:8. A fragment of a 38554, 57301, or 58324 protein can be an antigenic fragment of SEQ ID NO:2, SEQ ID NO:23, SEQ ID NO:5, or SEQ ID NO:8 of at least about 15 amino acids in length. A fragment of a 58324 protein can be at least about 139 amino acids of SEQ ID NO:8 or a fragment thereof, or a domain, *e.g.* a transmembrane domain or a fragment thereof, *e.g.* about amino acid residues 107 to 126, 150 to 166, 173 to 193, 231 to 254, 265 to 289, 314 to 335, 372 to 391, 420 to 444, 457 to 475, 580 to 603, 614 to 635, and 667 to 691 of SEQ ID NO:8.

A 38554, 57301, 58324 protein or fragment is provided which varies from the sequence of SEQ ID NO:2, SEQ ID NO:23, SEQ ID NO:5, or SEQ ID NO:8 in regions defined by amino acids about 476 to 667 of SEQ ID NO:2 or SEQ ID NO:23, 102 to 145 of SEQ ID NO:5 or 502 to 672 of SEQ ID NO:8 by at least one but by less than 15, 10 or 5 amino acid residues in the protein or fragment but which does not differ from SEQ ID NO:2, SEQ ID NO:23, SEQ ID NO:5, or SEQ ID NO:8 in regions defined by amino acids about 476 to 667 of SEQ ID NO:2 or SEQ ID NO:23, 102 to 145 of SEQ ID NO:5 or 502 to 672 of SEQ ID NO:8. (If this comparison requires alignment the sequences should be aligned for maximum homology. "Looped" out sequences from deletions or insertions, or mismatches, are considered differences.) In some embodiments the difference is at a non-essential residue or is a conservative substitution, while in others the difference is at an essential residue or is a non-conservative substitution.

In one embodiment, a biologically active portion of a 38554, 57301 or 58324 protein includes a sugar (and others) transporter domain. Moreover, other biologically active portions, in which other regions of the protein are deleted, can be prepared by recombinant techniques and evaluated for one or more of the functional activities of a native 38554, 57301 or 58324 protein.

In a preferred embodiment, the 38554, 57301 or 58324 protein has an amino acid sequence shown in SEQ ID NO:2, SEQ ID NO:23, SEQ ID NO:5, or SEQ ID NO:8, respectively. In other embodiments, the 38554, 57301 or 58324 protein is sufficiently or substantially identical to SEQ ID NO:2, SEQ ID NO:23, SEQ ID NO:5, or SEQ ID NO:8, respectively. In yet another embodiment, the 38554, 57301 or 58324 protein is sufficiently or substantially identical to SEQ ID NO:2, SEQ ID NO:23, SEQ ID NO:5, or SEQ ID NO:8, and retains the functional activity of the protein of SEQ ID NO:2, SEQ ID NO:23, SEQ ID NO:5, or SEQ ID NO:8, as described in detail in the subsections above.

### 38554, 57301 or 58324 Chimeric or Fusion Proteins

In another aspect, the invention provides 38554, 57301 or 58324 chimeric or fusion proteins. As used herein, a 38554, 57301 or 58324 "chimeric protein" or "fusion protein" includes a 38554, 57301 or 58324 polypeptide linked to a non-38554, 57301 or 58324 polypeptide. A "non-38554, 57301 or 58324 polypeptide" refers to a polypeptide having an amino acid sequence corresponding to a protein which is not substantially homologous to the 38554, 57301 or 58324 protein, *e.g.*, a protein which is different from the 38554, 57301 or 58324 protein and which is derived from the same or a different organism. The 38554, 57301 or 58324 polypeptide of the fusion protein can correspond to all or a portion *e.g.*, a fragment described herein of a 38554, 57301 or 58324 amino acid sequence. In a preferred embodiment, a 38554, 57301 or 58324 fusion protein includes at least one (or two) biologically active portion of a 38554, 57301 or 58324 protein. The non-38554, 57301 or 58324 polypeptide can be fused to the N-terminus or C-terminus of the 38554, 57301 or 58324 polypeptide.

The fusion protein can include a moiety which has a high affinity for a ligand. For example, the fusion protein can be a GST-38554, 57301 or 58324 fusion protein in which the 38554, 57301 or 58324 sequences are fused to the C-terminus of the GST sequences. Such fusion proteins can facilitate the purification of recombinant 38554, 57301 or 58324. Alternatively, the fusion protein can be a 38554, 57301 or 58324 protein containing a heterologous signal sequence at its N-terminus. In certain host cells (*e.g.*, mammalian host cells), expression and/or secretion of 38554, 57301 or 58324 can be increased through use of a heterologous signal sequence.

Fusion proteins can include all or a part of a serum protein, *e.g.*, a portion of an immunoglobulin (*e.g.*, IgG, IgA, or IgE), *e.g.*, an Fc region and/or the hinge C1 and C2 sequences of an immunoglobulin or human serum albumin.

The 38554, 57301 or 58324 fusion proteins of the invention can be incorporated into pharmaceutical compositions and administered to a subject *in vivo*. The 38554, 57301 or 58324 fusion proteins can be used to affect the bioavailability of a 38554, 57301 or 58324 substrate. 38554, 57301 or 58324 fusion proteins can be useful therapeutically for the treatment of disorders caused by, for example, (i) aberrant modification or mutation of a gene encoding a 38554, 57301 or 58324 protein; (ii) misregulation of the 38554, 57301 or 58324 gene; and (iii) aberrant post-translational modification of a 38554, 57301 or 58324 protein.

Moreover, the 38554, 57301 or 58324-fusion proteins of the invention can be used as immunogens to produce anti-38554, 57301 or 58324 antibodies in a subject, to purify 38554, 57301 or 58324 ligands and in screening assays to identify molecules which inhibit the interaction of 38554, 57301 or 58324 with a 38554, 57301 or 58324 substrate.

Expression vectors are commercially available that already encode a fusion moiety (*e.g.*, a GST polypeptide). A 38554, 57301 or 58324-encoding nucleic acid can be cloned into such an expression vector such that the fusion moiety is linked in-frame to the 38554, 57301 or 58324 protein.

### Variants of 38554, 57301 or 58324 Proteins

In another aspect, the invention also features a variant of a 38554, 57301 or 58324 polypeptide, *e.g.*, which functions as an agonist (mimetics) or as an antagonist. Variants of the 38554, 57301 or 58324 proteins can be generated by mutagenesis, *e.g.*, discrete point mutation, the insertion or deletion of sequences or the truncation of a 38554, 57301 or 58324 protein. An agonist of the 38554, 57301 or 58324 proteins can retain substantially the same, or a subset, of the biological activities of the naturally occurring form of a 38554, 57301 or 58324 protein. An antagonist of a 38554, 57301 or 58324 protein can inhibit one or more of the activities of the naturally occurring form of the 38554, 57301 or 58324 protein by, for example, competitively modulating a 38554, 57301 or 58324-mediated activity of a 38554, 57301 or 58324 protein. Thus, specific biological effects can be elicited by treatment with a variant of limited function. Preferably, treatment of a subject with a variant having a subset of the biological activities of the naturally occurring form of the protein has fewer side effects in a subject relative to treatment with the naturally occurring form of the 38554, 57301 or 58324 protein.

Variants of a 38554, 57301 or 58324 protein can be identified by screening combinatorial libraries of mutants, *e.g.*, truncation mutants, of a 38554, 57301 or 58324 protein for agonist or antagonist activity.

Libraries of fragments *e.g.*, N terminal, C terminal, or internal fragments, of a 38554, 57301 or 58324 protein coding sequence can be used to generate a variegated population of fragments for screening and subsequent selection of variants of a 38554, 57301 or 58324 protein.

Variants in which a cysteine residues is added or deleted or in which a residue which is glycosylated is added or deleted are particularly preferred.

Methods for screening gene products of combinatorial libraries made by point mutations or truncation, and for screening cDNA libraries for gene products having a selected property. Recursive ensemble mutagenesis (REM), a new technique which enhances the frequency of functional mutants in the libraries, can be used in combination with the screening assays to identify 38554, 57301 or 58324 variants (Arkin and Yourvan (1992) *Proc. Natl. Acad. Sci. USA* 89:7811-7815; Delgrave *et al.* (1993) *Protein Engineering* 6:327-331).

Cell based assays can be exploited to analyze a variegated 38554, 57301 or 58324 library. For example, a library of expression vectors can be transfected into a cell line, *e.g.*, a cell line, which ordinarily responds to 38554, 57301 or 58324 in a substrate-dependent manner. The transfected cells are then contacted with 38554, 57301 or 58324 and the effect of the expression of the mutant on signaling by the 38554, 57301 or 58324 substrate can be detected, *e.g.*, by measuring organic ion transporter activity, substrate or target molecule binding activity, intra- or inter-cellular signal modulating activity, gene expression modulating activity, hormonal response modulating acitivity, and/or the ability to protect cells and/or tissues from organic anions. Plasmid DNA can then be recovered from the cells which score for inhibition, or alternatively, potentiation of signaling by the 38554, 57301 or 58324 substrate, and the individual clones further characterized.

In another aspect, the invention features a method of making a 38554, 57301 or 58324 polypeptide, *e.g.*, a peptide having a non-wild type activity, *e.g.*, an antagonist, agonist, or super agonist of a naturally occurring 38554, 57301 or 58324 polypeptide, *e.g.*, a naturally occurring 38554, 57301 or 58324 polypeptide. The method includes altering the sequence of a 38554, 57301 or 58324 polypeptide, *e.g.*, altering the sequence, *e.g.*, by substitution or deletion of one or more residues of a non-conserved region, a domain or residue disclosed herein, and testing the altered polypeptide for the desired activity.

In another aspect, the invention features a method of making a fragment or analog of a 38554, 57301 or 58324 polypeptide a biological activity of a naturally occurring 38554, 57301 or 58324 polypeptide. The method includes altering the sequence, *e.g.*, by substitution or deletion of one or more residues, of a 38554, 57301 or 58324 polypeptide, *e.g.*, altering the sequence of a non-conserved region, or a domain or residue described herein, and testing the altered polypeptide for the desired activity.

### Anti-38554, 57301 or 58324 Antibodies

In another aspect, the invention provides an anti-38554, 57301 or 58324 antibody. The term "antibody" as used herein refers to an immunoglobulin molecule or immunologically active portion thereof, i.e., an antigen-binding portion. Examples of immunologically active portions of immunoglobulin molecules include scFV and dcFV fragments, Fab and F(ab')₂ fragments which can be generated by treating the antibody with an enzyme such as papain or pepsin, respectively.

The antibody can be a polyclonal, monoclonal, recombinant, *e.g.*, a chimeric or humanized, fully human, non-human, *e.g.*, murine, or single chain antibody. In a preferred embodiment it has effector function and can fix complement. The antibody can be coupled to a toxin or imaging agent.

A full-length 38554, 57301 or 58324 protein or, antigenic peptide fragment of 38554, 57301 or 58324 can be used as an immunogen or can be used to identify anti-38554, 57301 or 58324 antibodies made with other immunogens, *e.g.*, cells, membrane preparations, and the like. The antigenic peptide of 38554, 57301 or 58324 should include at least 8 amino acid residues of the amino acid sequence shown in SEQ ID NO:2, SEQ ID NO:23, SEQ ID NO:5, or SEQ ID NO:8 and encompasses an epitope of 38554, 57301 or 58324. Preferably, the antigenic peptide includes at least 10 amino acid residues, more preferably at least 15 amino acid residues, even more preferably at least 20 amino acid residues, and most preferably at least 30 amino acid residues.

Fragments of 38554, 57301 or 58324 which include residues about 28 to 36, from about 134 to 142, and from about 301 to 316 of SEQ ID NO:2 or SEQ ID NO:23 can be used to make, *e.g.*, used as immunogens or used to characterize the specificity of an antibody, antibodies against hydrophilic regions of the 38554 protein (see Figures 1A and 1B), about 82 to 95, from about 325 to 332, and from about 528 to 537 of SEQ ID NO:5 can be used to make, *e.g.*, used as immunogens or used to characterize the specificity of an antibody, antibodies against hydrophilic regions of the 57301 protein (see Figure 2) or about 40 to 50, from about 194 to 201, and from about 538 to 546 of SEQ ID NO:8 can be used to make, *e.g.*, used as immunogens or used to characterize the specificity of an antibody, antibodies against hydrophilic regions of the 58324 protein (see Figure 3. Similarly, fragments of 38554, 57301 or 58324 which include residues about 111 to 128, from about 274 to 295, and from about 641 to 664 of SEQ ID NO:2 or SEQ ID NO:23 can be used to make an antibody against a hydrophobic region of the 38554, about 151 to 167, from about 263 to 279, and from about 352 to 369 of SEQ ID NO:5 can be used to make an antibody against a hydrophobic region of the 57301 or about 173 to 193, from about 314 to 335, and from about 667 to 691 of SEQ ID NO:8 can be used to make an antibody against a hydrophobic region of the 58324 protein; fragments of 38554, 57301 or 58324 which include residues about 60 to 75, about 140 to 160, or about 450 to 500 of SEQ ID NO:2 or SEQ ID NO:23 can be used to make an antibody against a non-cytoplasmic region of the 38554 protein, about40 to 60, about 115 to 140, or about 427 to 435 of SEQ ID NO:5 can be used to make an antibody against a non-cytoplasmic region of the 57301 protein, about 127 to 149, about 194 to 230, or about 392 to 419 of SEQ ID NO:8 can be used to make an antibody against a non-cytoplasmic region of the 58324 protein; fragments of 38554, 57301, or 58324 which include residues about 1 to 30, about 300 to 352, or about 670 to 700 of SEQ ID NO:2 or SEQ ID NO:23 can be used to make an antibody against an intracellular region of the 38554 protein, about 1 to 20, about 223 to 231, or about 280 to 351 of SEQ ID NO:5 can be used to make an antibody against an intracellular region of the 57301 protein, about 1 to 106, about 255 to 264, or about 336 to371 of SEQ ID NO:8 can be used to make an antibody against an intracellular region of the 58324 protein; a fragment of 38554, 57301 or 58324 which includes residues about 476 to 490, about 500 to 525, or about 506 to 523 of SEQ ID NO:2 or SEQ ID NO:23; about 102 to 112, about 113 to 125, or about 130 to 140 of SEQ ID NO:5; or which includes residues about 502 to 522, 535 to 555 or about 570 to 600 of SEQ ID NO:8 can be used to make an antibody against the transporter domain of the 38554, 57301 or 58324 protein, respectively.

Antibodies reactive with, or specific or selective for, any of these regions, or other regions or domains described herein are provided.

Preferred epitopes encompassed by the antigenic peptide are regions of 38554, 57301 or 58324 are located on the surface of the protein, *e.g.*, hydrophilic regions, as well as regions with high antigenicity. For example, an Emini surface probability analysis of the human 38554, 57301 or 58324 protein sequence can be used to indicate the regions that have a particularly high probability of being localized to the surface of the 38554, 57301 or 58324 protein and are thus likely to constitute surface residues useful for targeting antibody production.

In a preferred embodiment the antibody can bind to the non-cytoplasmic portion of the 38554, 57301 or 58324 protein, *e.g.*, it can bind to a whole cell which expresses the 38554, 57301 or 58324 protein. In another embodiment, the antibody binds an intracellular portion of the 38554, 57301 or 58324 protein.

In a preferred embodiment the antibody binds an epitope on any domain or region on 38554, 57301 or 58324 proteins described herein.

Additionally, chimeric, humanized, and completely human antibodies are also within the scope of the invention. Chimeric, humanized, but most preferably, completely human antibodies are desirable for applications which include repeated administration, *e.g.*, therapeutic treatment of human patients, and some diagnostic applications.

Chimeric and humanized monoclonal antibodies, comprising both human and non-human portions, can be made using standard recombinant DNA techniques. Such chimeric and humanized monoclonal antibodies can be produced by recombinant DNA techniques known in the art, for example using methods described in Robinson *et al.* International Application No. PCT/US86/02269; Akira, *et al.* European Patent Application 184,187; Taniguchi, M., European Patent Application 171,496; Morrison *et al.* European Patent Application 173,494; Neuberger *et al.* PCT International Publication No. WO 86/01533; Cabilly *et al.* U.S. Patent No. 4,816,567; Cabilly *et al.* European Patent Application 125,023; Better *et al.* (1988) *Science* 240:1041-1043; Liu *et al.* (1987) *Proc. Natl. Acad. Sci. USA* 84:3439-3443; Liu *et al.* (1987) *J. Immunol.* 139:3521-3526; Sun *et al.* (1987) *Proc. Natl. Acad. Sci. USA* 84:214-218; Nishimura *et al.* (1987) *Canc. Res.* 47:999-1005; Wood *et al.* (1985) *Nature* 314:446-449; and Shaw *et al.* (1988) *J. Natl. Cancer Inst.* 80:1553-1559); Morrison, S. L. (1985) *Science* 229:1202-1207; Oi *et al.* (1986) *BioTechniques* 4:214; Winter U.S. Patent 5,225,539; Jones *et al.* (1986) *Nature* 321:552-525; Verhoeyan *et al.* (1988) *Science* 239:1534; and Beidler *et al.* (1988) *J. Immunol.* 141:4053-4060.

Completely human antibodies are particularly desirable for therapeutic treatment of human patients. Such antibodies can be produced using transgenic mice that are incapable of expressing endogenous immunoglobulin heavy and light chains genes, but which can express human heavy and light chain genes. *See,* for example, Lonberg and Huszar (1995) *Int. Rev. Immunol. 13*:65-93); and U.S. Patent Nos. 5,625,126; 5,633,425; 5,569,825; 5,661,016; and 5,545,806. In addition, companies such as Abgenix, Inc. (Fremont, CA) and Medarex, Inc. (Princeton, NJ), can be engaged to provide human antibodies directed against a selected antigen using technology similar to that described above.

Completely human antibodies that recognize a selected epitope can be generated using a technique referred to as "guided selection." In this approach a selected non-human monoclonal antibody, *e.g.*, a murine antibody, is used to guide the selection of a completely human antibody recognizing the same epitope. This technology is described by Jespers *et al.* (1994) *Bio*/*Technology 12*:899-903).

The anti-38554, 57301 or 58324 antibody can be a single chain antibody. A single-chain antibody (scFV) can be engineered (see, for example, Colcher, D. *et al.* (1999) *Ann. N Y Acad. Sci.* 880:263-80; and Reiter, Y. (1996) *Clin. Cancer Res.* 2:245-52). The single chain antibody can be dimerized or multimerized to generate multivalent antibodies having specificities for different epitopes of the same target 38554, 57301 or 58324 protein.

In a preferred embodiment, the antibody has reduced or no ability to bind an Fc receptor. For example, it is an isotype or subtype, fragment or other mutant, which does not support binding to an Fc receptor, *e.g.*, it has a mutagenized or deleted Fc receptor binding region.

An antibody (or fragment thereof) may be conjugated to a therapeutic moiety such as a cytotoxin, a therapeutic agent or a radioactive ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, maytansinoids, *e.g.*, maytansinol (see US Patent No. 5,208,020), CC-1065 (see US Patent Nos. 5,475,092, 5,585,499, 5,846,545) and analogs or homologs thereof. Therapeutic agents include, but are not limited to, antimetabolites (*e.g.*, methotrexate, 6-mercaptopurine, 6-thioguanine, cytarabine, 5-fluorouracil decarbazine), alkylating agents (*e.g.*, mechlorethamine, thioepa chlorambucil, CC-1065, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g.*, daunorubicin (formerly daunomycin) and doxorubicin), antibiotics (*e.g.*, dactinomycin (formerly actinomycin), bleomycin, mithramycin, and anthramycin (AMC)), and anti-mitotic agents (*e.g.*, vincristine, vinblastine, taxol and maytansinoids). Radioactive ions include, but are not limited to iodine, yttrium and praseodymium.

The conjugates of the invention can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophase colony stimulating factor ("GM-CSF"), granulocyte colony stimulating factor ("G-CSF"), or other growth factors.

Alternatively, an antibody can be conjugated to a second antibody to form an antibody heteroconjugate as described by Segal in U.S. Patent No. 4,676,980.

An anti-38554, 57301 or 58324 antibody (*e.g.*, monoclonal antibody) can be used to isolate 38554, 57301 or 58324 by standard techniques, such as affinity chromatography or immunoprecipitation. Moreover, an anti-38554, 57301 or 58324 antibody can be used to detect 38554, 57301 or 58324 protein *(e.g.,* in a cellular lysate or cell supernatant) in order to evaluate the abundance and pattern of expression of the protein. Anti-38554, 57301 or 58324 antibodies can be used diagnostically to monitor protein levels in tissue as part of a clinical testing procedure, *e.g.*, to determine the efficacy of a given treatment regimen. Detection can be facilitated by coupling (i.e., physically linking) the antibody to a detectable substance (i.e., antibody labelling). Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

In preferred embodiments, an antibody can be made by immunizing with a purified 38554, 57301 or 58324 antigen, or a fragment thereof, *e.g.*, a fragment described herein, a membrane associated antigen, tissues, *e.g.*, crude tissue preparations, whole cells, preferably living cells, lysed cells, or cell fractions, *e.g.*, membrane fractions.

Antibodies which bind only a native 38554, 57301 or 58324 protein, only denatured or otherwise non-native 38554, 57301 or 58324 protein, or which bind both, are within the invention. Antibodies with linear or conformational epitopes are within the invention. Conformational epitopes sometimes can be identified by identifying antibodies which bind to native but not denatured 38554, 57301 or 58324 protein.

### Recombinant Expression Vectors, Host Cells and Genetically Engineered Cells

In another aspect, the invention includes, vectors, preferably expression vectors, containing a nucleic acid encoding a polypeptide described herein. As used herein, the term "vector" refers to a nucleic acid molecule capable of transporting another nucleic acid to which it has been linked and can include a plasmid, cosmid or viral vector. The vector can be capable of autonomous replication or it can integrate into a host DNA. Viral vectors include, *e.g.*, replication defective retroviruses, adenoviruses and adeno-associated viruses.

A vector can include a 38554, 57301 or 58324 nucleic acid in a form suitable for expression of the nucleic acid in a host cell. Preferably the recombinant expression vector includes one or more regulatory sequences operatively linked to the nucleic acid sequence to be expressed. The term "regulatory sequence" includes promoters, enhancers and other expression control elements (*e.g.*, polyadenylation signals). Regulatory sequences include those which direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vectors of the invention can be introduced into host cells to thereby produce proteins or polypeptides, including fusion proteins or polypeptides, encoded by nucleic acids as described herein (*e.g.*, 38554, 57301 or 58324 proteins, mutant forms of 38554, 57301 or 58324 proteins, fusion proteins, and the like).

The recombinant expression vectors of the invention can be designed for expression of 38554, 57301 or 58324 proteins in prokaryotic or eukaryotic cells. For example, polypeptides of the invention can be expressed in *E. coli,* insect cells (*e.g.*, using baculovirus expression vectors), yeast cells or mammalian cells. Suitable host cells are discussed further in Goeddel, (1990) *Gene Expression Technology: Methods in Enzymology* 185, Academic Press, San Diego, CA . Alternatively, the recombinant expression vector can be transcribed and translated *in vitro*, for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D.B. and Johnson, K.S. (1988) *Gene* 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be used in 38554, 57301 or 58324 activity assays, (*e.g.*, direct assays or competitive assays described in detail below), or to generate antibodies specific or selective for 38554, 57301 or 58324 proteins. In a preferred embodiment, a fusion protein expressed in a retroviral expression vector of the present invention can be used to infect bone marrow cells which are subsequently transplanted into irradiated recipients. The pathology of the subject recipient is then examined after sufficient time has passed (*e.g.*, six weeks).

To maximize recombinant protein expression in *E. coli* is to express the protein in a host bacteria with an impaired capacity to proteolytically cleave the recombinant protein (Gottesman, S., (1990) *Gene Expression Technology: Methods in Enzymology* 185, Academic Press, San Diego, California 119-128). Another strategy is to alter the nucleic acid sequence of the nucleic acid to be inserted into an expression vector so that the individual codons for each amino acid are those preferentially utilized in *E. coli* (Wada *et al.*, (1992) *Nucleic Acids Res.* 20:2111-2118). Such alteration of nucleic acid sequences of the invention can be carried out by standard DNA synthesis techniques.

The 38554, 57301 or 58324 expression vector can be a yeast expression vector, a vector for expression in insect cells, *e.g.*, a baculovirus expression vector or a vector suitable for expression in mammalian cells.

When used in mammalian cells, the expression vector's control functions are often provided by viral regulatory elements. For example, commonly used promoters are derived from polyoma, Adenovirus 2, cytomegalovirus and Simian Virus 40.

In another embodiment, the recombinant mammalian expression vector is capable of directing expression of the nucleic acid preferentially in a particular cell type (*e.g.*, tissue-specific regulatory elements are used to express the nucleic acid). Non-limiting examples of suitable tissue-specific promoters include the albumin promoter (liver-specific; Pinkert *et al.* (1987) *Genes Dev.* 1:268-277), lymphoid-specific promoters (Calame and Eaton (1988) *Adv. Immunol.* 43:235-275), in particular promoters of T cell receptors (Winoto and Baltimore (1989) *EMBO J* 8:729-733) and immunoglobulins (Banerji *et al.* (1983) *Cell* 33:729-740; Queen and Baltimore (1983) *Cell* 33:741-748), neuron-specific promoters (*e.g.*, the neurofilament promoter; Byrne and Ruddle (1989) *Proc. Natl. Acad. Sci. USA* 86:5473-5477), pancreas-specific promoters (Edlund *et al.* (1985) *Science* 230:912-916), and mammary gland-specific promoters (*e.g.*, milk whey promoter; U.S. Patent No. 4,873,316 and European Application Publication No. 264,166). Developmentally-regulated promoters are also encompassed, for example, the murine hox promoters (Kessel and Gruss (1990) *Science* 249:374-379) and the α-fetoprotein promoter (Campes and Tilghman (1989) *Genes Dev.* 3:537-546).

The invention further provides a recombinant expression vector comprising a DNA molecule of the invention cloned into the expression vector in an antisense orientation. Regulatory sequences (*e.g.*, viral promoters and/or enhancers) operatively linked to a nucleic acid cloned in the antisense orientation can be chosen which direct the constitutive, tissue specific or cell type specific expression of antisense RNA in a variety of cell types. The antisense expression vector can be in the form of a recombinant plasmid, phagemid or attenuated virus. For a discussion of the regulation of gene expression using antisense genes see Weintraub, H. *et al.*, (1986) *Reviews - Trends in Genetics* 1:1.

Another aspect the invention provides a host cell which includes a nucleic acid molecule described herein, *e.g.*, a 38554, 57301 or 58324 nucleic acid molecule within a recombinant expression vector or a 38554, 57301 or 58324 nucleic acid molecule containing sequences which allow it to homologously recombine into a specific site of the host cell's genome. The terms "host cell" and "recombinant host cell" are used interchangeably herein. Such terms refer not only to the particular subject cell but to the progeny or potential progeny of such a cell. Because certain modifications can occur in succeeding generations due to either mutation or environmental influences, such progeny may not, in fact, be identical to the parent cell, but are still included within the scope of the term as used herein.

A host cell can be any prokaryotic or eukaryotic cell. For example, a 38554, 57301 or 58324 protein can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into host cells via conventional transformation or transfection techniques. As used herein, the terms "transformation" and "transfection" are intended to refer to a variety of art-recognized techniques for introducing foreign nucleic acid (*e.g.*, DNA) into a host cell, including calcium phosphate or calcium chloride co-precipitation, DEAE-dextran-mediated transfection, lipofection, or electroporation.

A host cell of the invention can be used to produce (i.e., express) a 38554, 57301 or 58324 protein. Accordingly, the invention further provides methods for producing a 38554, 57301 or 58324 protein using the host cells of the invention. In one embodiment, the method includes culturing the host cell of the invention (into which a recombinant expression vector encoding a 38554, 57301 or 58324 protein has been introduced) in a suitable medium such that a 38554, 57301 or 58324 protein is produced. In another embodiment, the method further includes isolating a 38554, 57301 or 58324 protein from the medium or the host cell.

In another aspect, the invention features, a cell or purified preparation of cells which include a 38554, 57301 or 58324 transgene, or which otherwise misexpress 38554, 57301 or 58324. The cell preparation can consist of human or non human cells, *e.g.*, rodent cells, *e.g.*, mouse or rat cells, rabbit cells, or pig cells. In preferred embodiments, the cell or cells include a 38554, 57301 or 58324 transgene, *e.g.*, a heterologous form of a 38554, 57301 or 58324, *e.g.*, a gene derived from humans (in the case of a non-human cell). The 38554, 57301 or 58324 transgene can be misexpressed, *e.g.*, overexpressed or underexpressed. In other preferred embodiments, the cell or cells include a gene which misexpress an endogenous 38554, 57301 or 58324, *e.g.*, a gene the expression of which is disrupted, *e.g.*, a knockout. Such cells can serve as a model for studying disorders which are related to mutated or mis-expressed 38554, 57301 or 58324 alleles or for use in drug screening.

In another aspect, the invention features, a human cell, *e.g.,* a hematopoietic stem cell, transformed with nucleic acid which encodes a subject 38554, 57301 or 58324 polypeptide.

Also provided are cells, preferably human cells, *e.g.*, human hematopoietic or fibroblast cells, in which an endogenous 38554, 57301 or 58324 is under the control of a regulatory sequence that does not normally control the expression of the endogenous 38554, 57301 or 58324 gene. The expression characteristics of an endogenous gene within a cell, *e.g.*, a cell line or microorganism, can be modified by inserting a heterologous DNA regulatory element into the genome of the cell such that the inserted regulatory element is operably linked to the endogenous 38554, 57301 or 58324 gene. For example, an endogenous 38554, 57301 or 58324 gene which is "transcriptionally silent," *e.g.*, not normally expressed, or expressed only at very low levels, can be activated by inserting a regulatory element which is capable of promoting the expression of a normally expressed gene product in that cell. Techniques such as targeted homologous recombinations, can be used to insert the heterologous DNA as described in, *e.g.*, Chappel, US 5,272,071; WO 91/06667, published in May 16, 1991.

### Transgenic Animals

The invention provides non-human transgenic animals. Such animals are useful for studying the function and/or activity of a 38554, 57301 or 58324 protein and for identifying and/or evaluating modulators of 38554, 57301 or 58324 activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, and the like. A transgene is exogenous DNA or a rearrangement, *e.g.*, a deletion of endogenous chromosomal DNA, which preferably is integrated into or occurs in the genome of the cells of a transgenic animal. A transgene can direct the expression of an encoded gene product in one or more cell types or tissues of the transgenic animal, other transgenes, *e.g.*, a knockout, reduce expression. Thus, a transgenic animal can be one in which an endogenous 38554, 57301 or 58324 gene has been altered by, *e.g.*, by homologous recombination between the endogenous gene and an exogenous DNA molecule introduced into a cell of the animal, *e.g.*, an embryonic cell of the animal, prior to development of the animal.

Intronic sequences and polyadenylation signals can also be included in the transgene to increase the efficiency of expression of the transgene. A tissue-specific regulatory sequence(s) can be operably linked to a transgene of the invention to direct expression of a 38554, 57301 or 58324 protein to particular cells. A transgenic founder animal can be identified based upon the presence of a 38554, 57301 or 58324 transgene in its genome and/or expression of 38554, 57301 or 58324 mRNA in tissues or cells of the animals. A transgenic founder animal can then be used to breed additional animals carrying the transgene. Moreover, transgenic animals carrying a transgene encoding a 38554, 57301 or 58324 protein can further be bred to other transgenic animals carrying other transgenes. 38554, 57301 or 58324 proteins or polypeptides can be expressed in transgenic animals or plants, *e.g.*, a nucleic acid encoding the protein or polypeptide can be introduced into the genome of an animal. In preferred embodiments the nucleic acid is placed under the control of a tissue specific promoter, *e.g.*, a milk or egg specific promoter, and recovered from the milk or eggs produced by the animal. Suitable animals are mice, pigs, cows, goats, and sheep.

The invention also includes a population of cells from a transgenic animal, as discussed, *e.g.*, below.

### Uses

The nucleic acid molecules, proteins, protein homologs, and antibodies described herein can be used in one or more of the following methods: a) screening assays; b) predictive medicine (*e.g.,* diagnostic assays, prognostic assays, monitoring clinical trials, and pharmacogenetics); and c) methods of treatment (*e.g.*, therapeutic and prophylactic).

The isolated nucleic acid molecules of the invention can be used, for example, to express a 38554, 57301 or 58324 protein (*e.g.*, via a recombinant expression vector in a host cell in gene therapy applications), to detect a 38554, 57301 or 58324 mRNA (*e.g.*, in a biological sample) or a genetic alteration in a 38554, 57301 or 58324 gene, and to modulate 38554, 57301 or 58324 activity, as described further below. The 38554, 57301 or 58324 proteins can be used to treat disorders characterized by insufficient or excessive production of a 38554, 57301 or 58324 substrate or production of 38554, 57301 or 58324 inhibitors. In addition, the 38554, 57301 or 58324 proteins can be used to screen for naturally occurring 38554, 57301 or 58324 substrates, to screen for drugs or compounds which modulate 38554, 57301 or 58324 activity, as well as to treat disorders characterized by insufficient or excessive production of 38554, 57301 or 58324 protein or production of 38554, 57301 or 58324 protein forms which have decreased, aberrant or unwanted activity compared to 38554, 57301 or 58324 wild type protein (*e.g.*, organic ion transport, organic ion absorption or excretion, inter- or intra-cellular signaling, and/or hormonal responses disorders). Moreover, the anti-38554, 57301 or 58324 antibodies of the invention can be used to detect and isolate 38554, 57301 or 58324 proteins, regulate the bioavailability of 38554, 57301 or 58324 proteins, and modulate 38554, 57301 or 58324 activity.

A method of evaluating a compound for the ability to interact with, *e.g.*, bind, a subject 38554, 57301 or 58324 polypeptide is provided. The method includes: contacting the compound with the subject 38554, 57301 or 58324 polypeptide; and evaluating ability of the compound to interact with, *e.g.*, to bind or form a complex with the subject 38554, 57301 or 58324 polypeptide. This method can be performed *in vitro, e.g.,* in a cell free system, or *in vivo, e.g.*, in a two-hybrid interaction trap assay. This method can be used to identify naturally occurring molecules which interact with subject 38554, 57301 or 58324 polypeptide. It can also be used to find natural or synthetic inhibitors of subject 38554, 57301 or 58324 polypeptide. Screening methods are discussed in more detail below.

### Screening Assays:

The invention provides methods (also referred to herein as "screening assays") for identifying modulators, i.e., candidate or test compounds or agents (*e.g.*, proteins, peptides, peptidomimetics, peptoids, small molecules or other drugs) which bind to 38554, 57301 or 58324 proteins, have a stimulatory or inhibitory effect on, for example, 38554, 57301 or 58324 expression or 38554, 57301 or 58324 activity, or have a stimulatory or inhibitory effect on, for example, the expression or activity of a 38554, 57301 or 58324 substrate. Compounds thus identified can be used to modulate the activity of target gene products (*e.g.*, 38554, 57301 or 58324 genes) in a therapeutic protocol, to elaborate the biological function of the target gene product, or to identify compounds that disrupt normal target gene interactions.

In one embodiment, the invention provides assays for screening candidate or test compounds which are substrates of a 38554, 57301 or 58324 protein or polypeptide or a biologically active portion thereof. In another embodiment, the invention provides assays for screening candidate or test compounds which bind to or modulate the activity of a 38554, 57301 or 58324 protein or polypeptide or a biologically active portion thereof.

The test compounds of the present invention can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, *e.g.*, Zuckermann, R.N. *et al.* (1994) *J. Med. Chem.* 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam, K.S. (1997) *Anticancer Drug Des.* 12:145).

Examples of methods for the synthesis of molecular libraries can be found in the art, for example in: DeWitt *et al.* (1993) *Proc. Natl. Acad. Sci. U.S.A.* 90:6909-13; Erb *et al.* (1994) *Proc. Natl. Acad. Sci. USA* 91:11422-426; Zuckermann *et al.* (1994). *J. Med. Chem.* 37:2678-85; Cho *et al.* (1993) *Science* 261:1303; Carrell *et al.* (1994) *Angew. Chem. Int. Ed. Engl.* 33:2059; Carell *et al.* (1994) *Angew. Chem. Int. Ed. Engl.* 33:2061; and in Gallop *et al.* (1994) *J. Med. Chem.* 37:1233-51.

Libraries of compounds can be presented in solution (*e.g.*, Houghten (1992) *Biotechniques* 13:412-421), or on beads (Lam (1991) *Nature* 354:82-84), chips (Fodor (1993) *Nature* 364:555-556), bacteria (Ladner, USP 5,223,409), spores (Ladner USP '409), plasmids (Cull *et al.* (1992) *Proc Natl Acad Sci USA* 89:1865-1869) or on phage (Scott and Smith (1990) *Science* 249:386-390; Devlin (1990) *Science* 249:404-406; Cwirla *et al.* (1990) *Proc. Natl. Acad. Sci.* 87:6378-6382; Felici (1991) *J. Mol. Biol.* 222:301-310; Ladner *supra.*).

In one embodiment, an assay is a cell-based assay in which a cell which expresses a 38554, 57301 or 58324 protein or biologically active portion thereof is contacted with a test compound, and the ability of the test compound to modulate 38554, 57301 or 58324 activity is determined. Determining the ability of the test compound to modulate 38554, 57301 or 58324 activity can be accomplished by monitoring, for example, organic ion transporter activity. The cell, for example, can be of mammalian origin, *e.g.*, human.

The ability of the test compound to modulate 38554, 57301 or 58324 binding to a compound, *e.g.*, a 38554, 57301 or 58324 substrate, or to bind to 38554, 57301 or 58324 can also be evaluated. This can be accomplished, for example, by coupling the compound, *e.g.*, the substrate, with a radioisotope or enzymatic label such that binding of the compound, *e.g.*, the substrate, to 38554, 57301 or 58324 can be determined by detecting the labeled compound, *e.g.*, substrate, in a complex. Alternatively, 38554, 57301 or 58324 could be coupled with a radioisotope or enzymatic label to monitor the ability of a test compound to modulate 38554, 57301 or 58324 binding to a 38554, 57301 or 58324 substrate in a complex. For example, compounds (*e.g.*, 38554, 57301 or 58324 substrates) can be labeled with ¹²⁵I, ³⁵S, ¹⁴C, or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (*e.g.*, a 38554, 57301 or 58324 substrate) to interact with 38554, 57301 or 58324 with or without the labeling of any of the interactants can be evaluated. For example, a microphysiometer can be used to detect the interaction of a compound with 38554, 57301 or 58324 without the labeling of either the compound or the 38554, 57301 or 58324. McConnell, H. M. *et al.* (1992) *Science* 257:1906-1912. As used herein, a "microphysiometer" (*e.g.*, Cytosensor) is an analytical instrument that measures the rate at which a cell acidifies its environment using a light-addressable potentiometric sensor (LAPS). Changes in this acidification rate can be used as an indicator of the interaction between a compound and 38554, 57301 or 58324.

In yet another embodiment, a cell-free assay is provided in which a 38554, 57301 or 58324 protein or biologically active portion thereof is contacted with a test compound and the ability of the test compound to bind to the 38554, 57301 or 58324 protein or biologically active portion thereof is evaluated. Preferred biologically active portions of the 38554, 57301 or 58324 proteins to be used in assays of the present invention include fragments which participate in interactions with non-38554, 57301 or 58324 molecules, *e.g.,* fragments with high surface probability scores.

Soluble and/or membrane-bound forms of isolated proteins (*e.g.*, 38554, 57301 or 58324 proteins or biologically active portions thereof) can be used in the cell-free assays of the invention. When membrane-bound forms of the protein are used, it may be desirable to utilize a solubilizing agent. Examples of such solubilizing agents include nonionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

Cell-free assays involve preparing a reaction mixture of the target gene protein and the test compound under conditions and for a time sufficient to allow the two components to interact and bind, thus forming a complex that can be removed and/or detected.

The interaction between two molecules can also be detected, *e.g.*, using fluorescence energy transfer (FET) (see, for example, Lakowicz *et al.*, U.S. Patent No. 5,631,169; Stavrianopoulos, *et al.*, U.S. Patent No. 4,868,103). A fluorophore label on the first, 'donor' molecule is selected such that its emitted fluorescent energy will be absorbed by a fluorescent label on a second, 'acceptor' molecule, which in turn is able to fluoresce due to the absorbed energy. Alternately, the 'donor' protein molecule can simply utilize the natural fluorescent energy of tryptophan residues. Labels are chosen that emit different wavelengths of light, such that the 'acceptor' molecule label can be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (*e.g.*, using a fluorimeter).

In another embodiment, determining the ability of the 38554, 57301 or 58324 protein to bind to a target molecule can be accomplished using real-time Biomolecular Interaction Analysis (BIA) (see, *e.g.*, Sjolander, S. and Urbaniczky, C. (1991) *Anal. Chem.* 63:2338-2345 and Szabo *et al.* (1995) *Curr. Opin. Struct. Biol.* 5:699-705). "Surface plasmon resonance" or "BIA" detects biospecific interactions in real time, without labeling any of the interactants (*e.g.*, BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In one embodiment, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize either 38554, 57301 or 58324, an anti-38554, 57301 or 58324 antibody or its target molecule to facilitate separation of complexed from uncomplexed forms of one or both of the proteins, as well as to accommodate automation of the assay. Binding of a test compound to a 38554, 57301 or 58324 protein, or interaction of a 38554, 57301 or 58324 protein with a target molecule in the presence and absence of a candidate compound, can be accomplished in any vessel suitable for containing the reactants. Examples of such vessels include microtiter plates, test tubes, and microcentrifuge tubes. In one embodiment, a fusion protein can be provided which adds a domain that allows one or both of the proteins to be bound to a matrix. For example, glutathione-S-transferase/38554, 57301 or 58324 fusion proteins or glutathione-S-transferase/target fusion proteins can be adsorbed onto glutathione sepharose beads (Sigma Chemical, St. Louis, MO) or glutathione derivatized microtiter plates, which are then combined with the test compound or the test compound and either the non-adsorbed target protein or 38554, 57301 or 58324 protein, and the mixture incubated under conditions conducive to complex formation (*e.g.*, at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of 38554, 57301 or 58324 binding or activity determined using standard techniques.

Other techniques for immobilizing either a 38554, 57301 or 58324 protein or a target molecule on matrices include using conjugation of biotin and streptavidin. Biotinylated 38554, 57301 or 58324 protein or target molecules can be prepared from biotin-NHS (N-hydroxy-succinimide) using techniques known in the art (*e.g.*, biotinylation kit, Pierce Chemicals, Rockford, IL), and immobilized in the wells of streptavidin-coated 96 well plates (Pierce Chemical).

In order to conduct the assay, the non-immobilized component is added to the coated surface containing the anchored component. After the reaction is complete, unreacted components are removed (*e.g.*, by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; *e.g.*, using a labeled antibody specific or selective for the immobilized component (the antibody, in turn, can be directly labeled or indirectly labeled with, *e.g.*, a labeled anti-Ig antibody).

In one embodiment, this assay is performed utilizing antibodies reactive with 38554, 57301 or 58324 protein or target molecules but which do not interfere with binding of the 38554, 57301 or 58324 protein to its target molecule. Such antibodies can be derivatized to the wells of the plate, and unbound target or 38554, 57301 or 58324 protein trapped in the wells by antibody conjugation. Methods for detecting such complexes, in addition to those described above for the GST-immobilized complexes, include immunodetection of complexes using antibodies reactive with the 38554, 57301 or 58324 protein or target molecule, as well as enzyme-linked assays which rely on detecting an enzymatic activity associated with the 38554, 57301 or 58324 protein or target molecule.

Alternatively, cell free assays can be conducted in a liquid phase. In such an assay, the reaction products are separated from unreacted components, by any of a number of standard techniques, including but not limited to: differential centrifugation (see, for example, Rivas, G., and Minton, A.P., (1993) *Trends Biochem Sci* 18:284-7); chromatography (gel filtration chromatography, ion-exchange chromatography); electrophoresis (see, *e.g.,* Ausubel, F. *et al.*, eds. (1999) *Current Protocols in Molecular Biology, J.* Wiley, New York.); and immunoprecipitation (see, for example, Ausubel, F. *et al.*, eds. (1999) *Current Protocols in Molecular Biology*, J. Wiley, New York). Such resins and chromatographic techniques are known to one skilled in the art (see, *e.g.*, Heegaard, N.H., (1998) *J Mol Recognit* 11:141-8; Hage, D.S., and Tweed, S.A. (1997) *J Chromatogr B Biomed Sci Appl.* 699:499-525). Further, fluorescence energy transfer can also be conveniently utilized, as described herein, to detect binding without further purification of the complex from solution.

In a preferred embodiment, the assay includes contacting the 38554, 57301 or 58324 protein or biologically active portion thereof with a known compound which binds 38554, 57301 or 58324 to form an assay mixture, contacting the assay mixture with a test compound, and determining the ability of the test compound to interact with a 38554, 57301 or 58324 protein, wherein determining the ability of the test compound to interact with a 38554, 57301 or 58324 protein includes determining the ability of the test compound to preferentially bind to 38554, 57301 or 58324 or biologically active portion thereof, or to modulate the activity of a target molecule, as compared to the known compound.

The target gene products of the invention can, *in vivo*, interact with one or more cellular or extracellular macromolecules, such as proteins. For the purposes of this discussion, such cellular and extracellular macromolecules are referred to herein as "binding partners." Compounds that disrupt such interactions can be useful in regulating the activity of the target gene product. Such compounds can include, but are not limited to molecules such as antibodies, peptides, and small molecules. The preferred target genes/products for use in this embodiment are the 38554, 57301 or 58324 genes herein identified. In an alternative embodiment, the invention provides methods for determining the ability of the test compound to modulate the activity of a 38554, 57301 or 58324 protein through modulation of the activity of a downstream effector of a 38554, 57301 or 58324 target molecule. For example, the activity of the effector molecule on an appropriate target can be determined, or the binding of the effector to an appropriate target can be determined, as previously described.

To identify compounds that interfere with the interaction between the target gene product and its cellular or extracellular binding partner(s), a reaction mixture containing the target gene product and the binding partner is prepared, under conditions and for a time sufficient, to allow the two products to form complex. In order to test an inhibitory agent, the reaction mixture is provided in the presence and absence of the test compound. The test compound can be initially included in the reaction mixture, or can be added at a time subsequent to the addition of the target gene and its cellular or extracellular binding partner. Control reaction mixtures are incubated without the test compound or with a placebo. The formation of any complexes between the target gene product and the cellular or extracellular binding partner is then detected. The formation of a complex in the control reaction, but not in the reaction mixture containing the test compound, indicates that the compound interferes with the interaction of the target gene product and the interactive binding partner. Additionally, complex formation within reaction mixtures containing the test compound and normal target gene product can also be compared to complex formation within reaction mixtures containing the test compound and mutant target gene product. This comparison can be important in those cases wherein it is desirable to identify compounds that disrupt interactions of mutant but not normal target gene products.

These assays can be conducted in a heterogeneous or homogeneous format. Heterogeneous assays involve anchoring either the target gene product or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the target gene products and the binding partners, *e.g.*, by competition, can be identified by conducting the reaction in the presence of the test substance. Alternatively, test compounds that disrupt preformed complexes, *e.g.*, compounds with higher binding constants that displace one of the components from the complex, can be tested by adding the test compound to the reaction mixture after complexes have been formed. The various formats are briefly described below.

In a heterogeneous assay system, either the target gene product or the interactive cellular or extracellular binding partner, is anchored onto a solid surface (*e.g.*, a microtiter plate), while the non-anchored species is labeled, either directly or indirectly. The anchored species can be immobilized by non-covalent or covalent attachments. Alternatively, an immobilized antibody specific or selective for the species to be anchored can be used to anchor the species to the solid surface.

In order to conduct the assay, the partner of the immobilized species is exposed to the coated surface with or without the test compound. After the reaction is complete, unreacted components are removed (*e.g.*, by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface; *e.g.*, using a labeled antibody specific or selective for the initially non-immobilized species (the antibody, in turn, can be directly labeled or indirectly labeled with, *e.g.*, a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; *e.g.*, using an immobilized antibody specific or selective for one of the binding components to anchor any complexes formed in solution, and a labeled antibody specific or selective for the other partner to detect anchored complexes. Again, depending upon the order of addition of reactants to the liquid phase, test compounds that inhibit complex or that disrupt preformed complexes can be identified.

In an alternate embodiment of the invention, a homogeneous assay can be used. For example, a preformed complex of the target gene product and the interactive cellular or extracellular binding partner product is prepared in that either the target gene products or their binding partners are labeled, but the signal generated by the label is quenched due to complex formation (see, *e.g.*, U.S. Patent No. 4,109,496 that utilizes this approach for immunoassays). The addition of a test substance that competes with and displaces one of the species from the preformed complex will result in the generation of a signal above background. In this way, test substances that disrupt target gene product-binding partner interaction can be identified.

In yet another aspect, the 38554, 57301 or 58324 proteins can be used as "bait proteins" in a two-hybrid assay or three-hybrid assay (see, *e.g.*, U.S. Patent No. 5,283,317; Zervos *et al.* (1993) *Cell* 72:223-232; Madura *et al.* (1993) *J. Biol*. *Chem.* 268:12046-12054; Bartel *et al.* (1993) *Biotechniques* 14:920-924; Iwabuchi *et al.* (1993) *Oncogene* 8:1693-1696; and Brent WO94/10300), to identify other proteins, which bind to or interact with 38554, 57301 or 58324 ("38554, 57301 or 58324-binding proteins" or "38554, 57301 or 58324-bp") and are involved in 38554, 57301 or 58324 activity. Such 38554, 57301 or 58324-bps can be activators or inhibitors of signals by the 38554, 57301 or 58324 proteins or 38554, 57301 or 58324 targets as, for example, downstream elements of a 38554, 57301 or 58324-mediated signaling pathway.

The two-hybrid system is based on the modular nature of most transcription factors, which consist of separable DNA-binding and activation domains. Briefly, the assay utilizes two different DNA constructs. In one construct, the gene that codes for a 38554, 57301 or 58324 protein is fused to a gene encoding the DNA binding domain of a known transcription factor (*e.g.*, GAL-4). In the other construct, a DNA sequence, from a library of DNA sequences, that encodes an unidentified protein ("prey" or "sample") is fused to a gene that codes for the activation domain of the known transcription factor. (Alternatively the: 38554, 57301 or 58324 protein can be the fused to the activator domain.) If the "bait" and the "prey" proteins are able to interact, *in vivo*, forming a 38554, 57301 or 58324-dependent complex, the DNA-binding and activation domains of the transcription factor are brought into close proximity. This proximity allows transcription of a reporter gene (*e.g.*, lacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the 38554, 57301 or 58324 protein.

In another embodiment, modulators of 38554, 57301 or 58324 expression are identified. For example, a cell or cell free mixture is contacted with a candidate compound and the expression of 38554, 57301 or 58324 mRNA or protein evaluated relative to the level of expression of 38554, 57301 or 58324 mRNA or protein in the absence of the candidate compound. When expression of 38554, 57301 or 58324 mRNA or protein is greater in the presence of the candidate compound than in its absence, the candidate compound is identified as a stimulator of 38554, 57301 or 58324 mRNA or protein expression. Alternatively, when expression of 38554, 57301 or 58324 mRNA or protein is less (statistically significantly less) in the presence of the candidate compound than in its absence, the candidate compound is identified as an inhibitor of 38554, 57301 or 58324 mRNA or protein expression. The level of 38554, 57301 or 58324 mRNA or protein expression can be determined by methods described herein for detecting 38554, 57301 or 58324 mRNA or protein.

In another aspect, the invention pertains to a combination of two or more of the assays described herein. For example, a modulating agent can be identified using a cell-based or a cell free assay, and the ability of the agent to modulate the activity of a 38554, 57301 or 58324 protein can be confirmed *in vivo, e.g.*, in an animal such as an animal model for organic ion transport, organic ion absorption or excretion, inter- or intra-cellular signaling, and/or hormonal responses disorders.

This invention further pertains to novel agents identified by the above-described screening assays. Accordingly, it is within the scope of this invention to further use an agent identified as described herein (*e.g.*, a 38554, 57301 or 58324 modulating agent, an antisense 38554, 57301 or 58324 nucleic acid molecule, a 38554, 57301 or 58324-specific antibody, or a 38554, 57301 or 58324-binding partner) in an appropriate animal model to determine the efficacy, toxicity, side effects, or mechanism of action, of treatment with such an agent. Furthermore, novel agents identified by the above-described screening assays can be used for treatments as described herein.

### Detection Assays

Portions or fragments of the nucleic acid sequences identified herein can be used as polynucleotide reagents. For example, these sequences can be used to: (i) map their respective genes on a chromosome *e.g.*, to locate gene regions associated with genetic disease or to associate 38554, 57301 or 58324 with a disease; (ii) identify an individual from a minute biological sample (tissue typing); and (iii) aid in forensic identification of a biological sample. These applications are described in the subsections below.

### Chromosome Mapping

The 38554, 57301 or 58324 nucleotide sequences or portions thereof can be used to map the location of the 38554, 57301 or 58324 genes on a chromosome. This process is called chromosome mapping. Chromosome mapping is useful in correlating the 38554, 57301 or 58324 sequences with genes associated with disease.

Briefly, 38554, 57301 or 58324 genes can be mapped to chromosomes by preparing PCR primers (preferably 15-25 bp in length) from the 38554, 57301 or 58324 nucleotide sequences. These primers can then be used for PCR screening of somatic cell hybrids containing individual human chromosomes. Only those hybrids containing the human gene corresponding to the 38554, 57301 or 58324 sequences will yield an amplified fragment.

A panel of somatic cell hybrids in which each cell line contains either a single human chromosome or a small number of human chromosomes, and a full set of mouse chromosomes, can allow easy mapping of individual genes to specific human chromosomes. (D'Eustachio P. *et al.* (1983) *Science* 220:919-924).

Other mapping strategies *e.g.*, in situ hybridization (described in Fan, Y. *et al.* (1990) *Proc. Natl. Acad. Sci. USA*, 87:6223-27), pre-screening with labeled flow-sorted chromosomes, and pre-selection by hybridization to chromosome specific cDNA libraries can be used to map 38554, 57301 or 58324 to a chromosomal location.

Fluorescence *in situ* hybridization (FISH) of a DNA sequence to a metaphase chromosomal spread can further be used to provide a precise chromosomal location in one step. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases will suffice to get good results at a reasonable amount of time. For a review of this technique, see Verma *et al.* (1988) Human Chromosomes: A Manual of Basic Techniques, Pergamon Press, New York).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. (Such data are found, for example, in V. McKusick, *Mendelian Inheritance in Man,* available on-line through Johns Hopkins University Welch Medical Library). The relationship between a gene and a disease, mapped to the same chromosomal region, can then be identified through linkage analysis (co-inheritance of physically adjacent genes), described in, for example, Egeland, J. *et al.* (1987) *Nature,* 325:783-787.

Moreover, differences in the DNA sequences between individuals affected and unaffected with a disease associated with the 38554, 57301 or 58324 gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### Tissue Typing

38554, 57301 or 58324 sequences can be used to identify individuals from biological samples using, *e.g.*, restriction fragment length polymorphism (RFLP). In this technique, an individual's genomic DNA is digested with one or more restriction enzymes, the fragments separated, *e.g.*, in a Southern blot, and probed to yield bands for identification. The sequences of the present invention are useful as additional DNA markers for RFLP (described in U.S. Patent 5,272,057).

Furthermore, the sequences of the present invention can also be used to determine the actual base-by-base DNA sequence of selected portions of an individual's genome. Thus, the 38554, 57301 or 58324 nucleotide sequences described herein can be used to prepare two PCR primers from the 5' and 3' ends of the sequences. These primers can then be used to amplify an individual's DNA and subsequently sequence it. Panels of corresponding DNA sequences from individuals, prepared in this manner, can provide unique individual identifications, as each individual will have a unique set of such DNA sequences due to allelic differences.

Allelic variation occurs to some degree in the coding regions of these sequences, and to a greater degree in the noncoding regions. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO:1, SEQ ID NO:22, SEQ ID NO:4, or SEQ ID NO:7 can provide positive individual identification with a panel of perhaps 10 to 1,000 primers which each yield a noncoding amplified sequence of 100 bases. If predicted coding sequences, such as those in SEQ ID NO:3, SEQ ID NO:24, SEQ ID NO:6, or SEQ ID NO:9 are used, a more appropriate number of primers for positive individual identification would be 500-2,000.

If a panel of reagents from 38554, 57301 or 58324 nucleotide sequences described herein is used to generate a unique identification database for an individual, those same reagents can later be used to identify tissue from that individual. Using the unique identification database, positive identification of the individual, living or dead, can be made from extremely small tissue samples.

### Use of Partial 38554, 57301 or 58324 Sequences in Forensic Biology

DNA-based identification techniques can also be used in forensic biology. To make such an identification, PCR technology can be used to amplify DNA sequences taken from very small biological samples such as tissues, *e.g.*, hair or skin, or body fluids, *e.g.*, blood, saliva, or semen found at a crime scene. The amplified sequence can then be compared to a standard, thereby allowing identification of the origin of the biological sample.

The sequences of the present invention can be used to provide polynucleotide reagents, *e.g.*, PCR primers, targeted to specific loci in the human genome, which can enhance the reliability of DNA-based forensic identifications by, for example, providing another "identification marker" (i.e. another DNA sequence that is unique to a particular individual). As mentioned above, actual base sequence information can be used for identification as an accurate alternative to patterns formed by restriction enzyme generated fragments. Sequences targeted to noncoding regions of SEQ ID NO: 1, SEQ ID NO:22, SEQ ID NO:4, or SEQ ID NO:7 (*e.g.*, fragments derived from the noncoding regions of SEQ ID NO:1, SEQ ID NO:22, SEQ ID NO:4, or SEQ ID NO:7 having a length of at least 20 bases, preferably at least 30 bases) are particularly appropriate for this use.

The 38554, 57301 or 58324 nucleotide sequences described herein can further be used to provide polynucleotide reagents, *e.g.*, labeled or labelable probes which can be used in, for example, an *in situ* hybridization technique, to identify a specific tissue. This can be very useful in cases where a forensic pathologist is presented with a tissue of unknown origin. Panels of such 38554, 57301 or 58324 probes can be used to identify tissue by species and/or by organ type.

In a similar fashion, these reagents, *e.g.*, 38554, 57301 or 58324 primers or probes can be used to screen tissue culture for contamination (i.e. screen for the presence of a mixture of different types of cells in a culture).

### Predictive Medicine

The present invention also pertains to the field of predictive medicine in which diagnostic assays, prognostic assays, and monitoring clinical trials are used for prognostic (predictive) purposes to thereby treat an individual.

Generally, the invention provides, a method of determining if a subject is at risk for a disorder related to a lesion in or the misexpression of a gene which encodes 38554, 57301 or 58324.

Such disorders include, *e.g.*, a disorder associated with the misexpression of 38554, 57301 or 58324 gene; a disorder of the organic ion transport system.

The method includes one or more of the following:

detecting, in a tissue of the subject, the presence or absence of a mutation which affects the expression of the 38554, 57301 or 58324 gene, or detecting the presence or absence of a mutation in a region which controls the expression of the gene, *e.g.*, a mutation in the 5' control region;

detecting, in a tissue of the subject, the presence or absence of a mutation which alters the structure of the 38554, 57301 or 58324 gene;

detecting, in a tissue of the subject, the misexpression of the 38554, 57301 or 58324 gene, at the mRNA level, *e.g.*, detecting a non-wild type level of a mRNA ;

detecting, in a tissue of the subject, the misexpression of the gene, at the protein level, *e.g.*, detecting a non-wild type level of a 38554, 57301 or 58324 polypeptide.

In preferred embodiments the method includes: ascertaining the existence of at least one of: a deletion of one or more nucleotides from the 38554, 57301 or 58324 gene; an insertion of one or more nucleotides into the gene, a point mutation, *e.g.*, a substitution of one or more nucleotides of the gene, a gross chromosomal rearrangement of the gene, *e.g.*, a translocation, inversion, or deletion.

For example, detecting the genetic lesion can include: (i) providing a probe/primer including an oligonucleotide containing a region of nucleotide sequence which hybridizes to a sense or antisense sequence from SEQ ID NO:1, SEQ ID NO:22, SEQ ID NO:4, or SEQ ID NO:7, or naturally occurring mutants thereof or 5' or 3' flanking sequences naturally associated with the 38554, 57301 or 58324 gene; (ii) exposing the probe/primer to nucleic acid of the tissue; and detecting, by hybridization, *e.g.*, *in situ* hybridization, of the probe/primer to the nucleic acid, the presence or absence of the genetic lesion.

In preferred embodiments detecting the misexpression includes ascertaining the existence of at least one of: an alteration in the level of a messenger RNA transcript of the 38554, 57301 or 58324 gene; the presence of a non-wild type splicing pattern of a messenger RNA transcript of the gene; or a non-wild type level of 38554, 57301 or 58324.

Methods of the invention can be used prenatally or to determine if a subject's offspring will be at risk for a disorder.

In preferred embodiments the method includes determining the structure of a 38554, 57301 or 58324 gene, an abnormal structure being indicative of risk for the disorder.

In preferred embodiments the method includes contacting a sample from the subject with an antibody to the 38554, 57301 or 58324 protein or a nucleic acid, which hybridizes specifically with the gene. These and other embodiments are discussed below.

### Diagnostic and Prognostic Assays

The presence, level, or absence of 38554, 57301 or 58324 protein or nucleic acid in a biological sample can be evaluated by obtaining a biological sample from a test subject and contacting the biological sample with a compound or an agent capable of detecting 38554, 57301 or 58324 protein or nucleic acid (*e.g.*, mRNA, genomic DNA) that encodes 38554, 57301 or 58324 protein such that the presence of 38554, 57301 or 58324 protein or nucleic acid is detected in the biological sample. The term "biological sample" includes tissues, cells and biological fluids isolated from a subject, as well as tissues, cells and fluids present within a subject. A preferred biological sample is serum. The level of expression of the 38554, 57301 or 58324 gene can be measured in a number of ways, including, but not limited to: measuring the mRNA encoded by the 38554, 57301 or 58324 genes; measuring the amount of protein encoded by the 38554, 57301 or 58324 genes; or measuring the activity of the protein encoded by the 38554, 57301 or 58324 genes.

The level of mRNA corresponding to the 38554, 57301 or 58324 gene in a cell can be determined both by *in situ* and by *in vitro* formats.

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length 38554, 57301 or 58324 nucleic acid, such as the nucleic acid of SEQ ID NO:1, SEQ ID NO:22, SEQ ID NO:4, or SEQ ID NO:7, or a portion thereof, such as an oligonucleotide of at least 7, 15, 30, 50, 100, 250 or 500 nucleotides in length and sufficient to specifically hybridize under stringent conditions to 38554, 57301 or 58324 mRNA or genomic DNA. Other suitable probes for use in the diagnostic assays are described herein.

In one format, mRNA (or cDNA) is immobilized on a surface and contacted with the probes, for example by running the isolated mRNA on an agarose gel and transferring the mRNA from the gel to a membrane, such as nitrocellulose. In an alternative format, the probes are immobilized on a surface and the mRNA (or cDNA) is contacted with the probes, for example, in a two-dimensional gene chip array. A skilled artisan can adapt known mRNA detection methods for use in detecting the level of mRNA encoded by the 38554, 57301 or 58324 genes.

The level of mRNA in a sample that is encoded by one of 38554, 57301 or 58324 can be evaluated with nucleic acid amplification, *e.g.*, by rtPCR (Mullis (1987) U.S. Patent No. 4,683,202), ligase chain reaction (Barany (1991) *Proc. Natl. Acad. Sci. USA* 88:189-193), self sustained sequence replication (Guatelli *et al.*, (1990) *Proc. Natl. Acad. Sci. USA* 87:1874-1878), transcriptional amplification system (Kwoh *et al.*, (1989), *Proc. Natl. Acad. Sci. USA* 86:1173-1177), Q-Beta Replicase (Lizardi *et al.*, (1988) *Bio*/*Technology* 6:1197), rolling circle replication (Lizardi *et al.*, U.S. Patent No. 5,854,033) or any other nucleic acid amplification method, followed by the detection of the amplified molecules using techniques known in the art. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, a cell or tissue sample can be prepared/processed and immobilized on a support, typically a glass slide, and then contacted with a probe that can hybridize to mRNA that encodes the 38554, 57301 or 58324 gene being analyzed.

In another embodiment, the methods further contacting a control sample with a compound or agent capable of detecting 38554, 57301 or 58324 mRNA, or genomic DNA, and comparing the presence of 38554, 57301 or 58324 mRNA or genomic DNA in the control sample with the presence of 38554, 57301 or 58324 mRNA or genomic DNA in the test sample.

A variety of methods can be used to determine the level of protein encoded by 38554, 57301 or 58324. In general, these methods include contacting an agent that selectively binds to the protein, such as an antibody with a sample, to evaluate the level of protein in the sample. In a preferred embodiment, the antibody bears a detectable label. Antibodies can be polyclonal, or more preferably, monoclonal. An intact antibody, or a fragment thereof (*e.g.*, Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with a detectable substance. Examples of detectable substances are provided herein.

The detection methods can be used to detect 38554, 57301 or 58324 protein in a biological sample *in vitro* as well as *in vivo*. *In vitro* techniques for detection of 38554, 57301 or 58324 protein include enzyme linked immunosorbent assays (ELISAs), immunoprecipitations, immunofluorescence, enzyme immunoassay (EIA), radioimmunoassay (RIA), and Western blot analysis. *In vivo* techniques for detection of 38554, 57301 or 58324 protein include introducing into a subject a labeled anti-38554, 57301 or 58324 antibody. For example, the antibody can be labeled with a radioactive marker whose presence and location in a subject can be detected by standard imaging techniques.

In another embodiment, the methods further include contacting the control sample with a compound or agent capable of detecting 38554, 57301 or 58324 protein, and comparing the presence of 38554, 57301 or 58324 protein in the control sample with the presence of 38554, 57301 or 58324 protein in the test sample.

The invention also includes kits for detecting the presence of 38554, 57301 or 58324 in a biological sample. For example, the kit can include a compound or agent capable of detecting 38554, 57301 or 58324 protein or mRNA in a biological sample; and a standard. The compound or agent can be packaged in a suitable container. The kit can further comprise instructions for using the kit to detect 38554, 57301 or 58324 protein or nucleic acid.

For antibody-based kits, the kit can include: (1) a first antibody (*e.g.*, attached to a solid support) which binds to a polypeptide corresponding to a marker of the invention; and, optionally, (2) a second, different antibody which binds to either the polypeptide or the first antibody and is conjugated to a detectable agent.

For oligonucleotide-based kits, the kit can include: (1) an oligonucleotide, *e.g.*, a detectably labeled oligonucleotide, which hybridizes to a nucleic acid sequence encoding a polypeptide corresponding to a marker of the invention or (2) a pair of primers useful for amplifying a nucleic acid molecule corresponding to a marker of the invention. The kit can also includes a buffering agent, a preservative, or a protein stabilizing agent. The kit can also includes components necessary for detecting the detectable agent (*e.g.*, an enzyme or a substrate). The kit can also contain a control sample or a series of control samples which can be assayed and compared to the test sample contained. Each component of the kit can be enclosed within an individual container and all of the various containers can be within a single package, along with instructions for interpreting the results of the assays performed using the kit.

The diagnostic methods described herein can identify subjects having, or at risk of developing, a disease or disorder associated with misexpressed or aberrant or unwanted 38554, 57301 or 58324 expression or activity. As used herein, the term "unwanted" includes an unwanted phenomenon involved in a biological response such as pain or deregulated cell proliferation.

In one embodiment, a disease or disorder associated with aberrant or unwanted 38554, 57301 or 58324 expression or activity is identified. A test sample is obtained from a subject and 38554, 57301 or 58324 protein or nucleic acid (*e.g.*, mRNA or genomic DNA) is evaluated, wherein the level, *e.g.*, the presence or absence, of 38554, 57301 or 58324 protein or nucleic acid is diagnostic for a subject having or at risk of developing a disease or disorder associated with aberrant or unwanted 38554, 57301 or 58324 expression or activity. As used herein, a "test sample" refers to a biological sample obtained from a subject of interest, including a biological fluid (*e.g.*, serum), cell sample, or tissue.

The prognostic assays described herein can be used to determine whether a subject can be administered an agent (*e.g.*, an agonist, antagonist, peptidomimetic, protein, peptide, nucleic acid, small molecule, or other drug candidate) to treat a disease or disorder associated with aberrant or unwanted 38554, 57301 or 58324 expression or activity. For example, such methods can be used to determine whether a subject can be effectively treated with an agent for a cell organic ion transporter-associated disorder.

The methods of the invention can also be used to detect genetic alterations in a 38554, 57301 or 58324 gene, thereby determining if a subject with the altered gene is at risk for a disorder characterized by misregulation in 38554, 57301 or 58324 protein activity or nucleic acid expression, such as an organic ion transporter-associated disorder. In preferred embodiments, the methods include detecting, in a sample from the subject, the presence or absence of a genetic alteration characterized by at least one of an alteration affecting the integrity of a gene encoding a 38554, 57301 or 58324-protein, or the misexpression of the 38554, 57301 or 58324 gene. For example, such genetic alterations can be detected by ascertaining the existence of at least one of 1) a deletion of one or more nucleotides from a 38554, 57301 or 58324 gene; 2) an addition of one or more nucleotides to a 38554, 57301 or 58324 gene; 3) a substitution of one or more nucleotides of a 38554, 57301 or 58324 gene, 4) a chromosomal rearrangement of a 38554, 57301 or 58324 gene; 5) an alteration in the level of a messenger RNA transcript of a 38554, 57301 or 58324 gene, 6) aberrant modification of a 38554, 57301 or 58324 gene, such as of the methylation pattern of the genomic DNA, 7) the presence of a non-wild type splicing pattern of a messenger RNA transcript of a 38554, 57301 or 58324 gene, 8) a non-wild type level of a 38554, 57301 or 58324-protein, 9) allelic loss of a 38554, 57301 or 58324 gene, and 10) inappropriate post-translational modification of a 38554, 57301 or 58324-protein.

An alteration can be detected without a probe/primer in a polymerase chain reaction, such as anchor PCR or RACE PCR, or, alternatively, in a ligation chain reaction (LCR), the latter of which can be particularly useful for detecting point mutations in the 38554, 57301 or 58324 gene. This method can include the steps of collecting a sample of cells from a subject, isolating nucleic acid (*e.g.*, genomic, mRNA or both) from the sample, contacting the nucleic acid sample with one or more primers which specifically hybridize to a 38554, 57301 or 58324 gene under conditions such that hybridization and amplification of the 38554, 57301 or 58324-gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein. Alternatively, other amplification methods described herein or known in the art can be used.

In another embodiment, mutations in a 38554, 57301 or 58324 gene from a sample cell can be identified by detecting alterations in restriction enzyme cleavage patterns. For example, sample and control DNA is isolated, amplified (optionally), digested with one or more restriction endonucleases, and fragment length sizes are determined, *e.g.*, by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in 38554, 57301 or 58324 can be identified by hybridizing a sample and control nucleic acids, *e.g.*, DNA or RNA, two dimensional arrays, *e.g.*, chip based arrays. Such arrays include a plurality of addresses, each of which is positionally distinguishable from the other. A different probe is located at each address of the plurality. The arrays can have a high density of addresses, *e.g.*, can contain hundreds or thousands of oligonucleotides probes (Cronin, M.T. *et al.* (1996) *Human Mutation* 7: 244-255; Kozal, M.J. *et al.* (1996) *Nature Medicine* 2: 753-759). For example, genetic mutations in 38554, 57301 or 58324 can be identified in two dimensional arrays containing light-generated DNA probes as described in Cronin, M.T. *et al. supra.* Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the 38554, 57301 or 58324 gene and detect mutations by comparing the sequence of the sample 38554, 57301 or 58324 with the corresponding wild-type (control) sequence. Automated sequencing procedures can be utilized when performing the diagnostic assays (Naeve C.W. *et al.* (1995) *Biotechniques* 19:448-53), including sequencing by mass spectrometry.

Other methods for detecting mutations in the 38554, 57301 or 58324 gene include methods in which protection from cleavage agents is used to detect mismatched bases in RNA/RNA or RNA/DNA heteroduplexes (Myers *et al.* (1985) *Science* 230:1242; Cotton *et al.* (1988) *Proc. Natl Acad Sci USA* 85:4397; Saleeba *et al.* (1992) *Methods Enzymol.* 217:286-295).

In still another embodiment, the mismatch cleavage reaction employs one or more proteins that recognize mismatched base pairs in double-stranded DNA (so called "DNA mismatch repair" enzymes) in defined systems for detecting and mapping point mutations in 38554, 57301 or 58324 cDNAs obtained from samples of cells. For example, the mutY enzyme of *E. coli* cleaves A at G/A mismatches and the thymidine DNA glycosylase from HeLa cells cleaves T at G/T mismatches (Hsu *et al.* (1994) *Carcinogenesis* 15:1657-1662; U.S. Patent No. 5,459,039).

In other embodiments, alterations in electrophoretic mobility will be used to identify mutations in 38554, 57301 or 58324 genes. For example, single strand conformation polymorphism (SSCP) can be used to detect differences in electrophoretic mobility between mutant and wild type nucleic acids (Orita *et al.* (1989) *Proc Natl. Acad. Sci USA:* 86:2766, see also Cotton (1993) *Mutat. Res.* 285:125-144; and Hayashi (1992) *Genet. Anal. Tech. Appl.* 9:73-79). Single-stranded DNA fragments of sample and control 38554, 57301 or 58324 nucleic acids will be denatured and allowed to renature. The secondary structure of single-stranded nucleic acids varies according to sequence, the resulting alteration in electrophoretic mobility enables the detection of even a single base change. The DNA fragments can be labeled or detected with labeled probes. The sensitivity of the assay can be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen *et al.* (1991) *Trends Genet 7:5).*

In yet another embodiment, the movement of mutant or wild-type fragments in polyacrylamide gels containing a gradient of denaturant is assayed using denaturing gradient gel electrophoresis (DGGE) (Myers *et al.* (1985) *Nature* 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) *Biophys Chem* 265:12753).

Examples of other techniques for detecting point mutations include, but are not limited to, selective oligonucleotide hybridization, selective amplification, or selective primer extension (Saiki *et al.* (1986) *Nature* 324:163); Saiki *et al.* (1989) *Proc. Natl Acad. Sci USA* 86:6230).

Alternatively, allele specific amplification technology which depends on selective PCR amplification can be used in conjunction with the instant invention. Oligonucleotides used as primers for specific amplification can carry the mutation of interest in the center of the molecule (so that amplification depends on differential hybridization) (Gibbs *et al.* (1989) *Nucleic Acids Res.* 17:2437-2448) or at the extreme 3' end of one primer where, under appropriate conditions, mismatch can prevent, or reduce polymerase extension (Prossner (1993) *Tibtech* 11:238). In addition it may be desirable to introduce a novel restriction site in the region of the mutation to create cleavage-based detection (Gasparini *et al.* (1992) *Mol. Cell Probes* 6:1). It is anticipated that in certain embodiments amplification can also be performed using Taq ligase for amplification (Barany (1991) *Proc. Natl. Acad. Sci USA* 88:189-93). In such cases, ligation will occur only if there is a perfect match at the 3' end of the 5' sequence making it possible to detect the presence of a known mutation at a specific site by looking for the presence or absence of amplification.

The methods described herein can be performed, for example, by utilizing pre-packaged diagnostic kits comprising at least one probe nucleic acid or antibody reagent described herein, which can be conveniently used, *e.g.*, in clinical settings to diagnose patients exhibiting symptoms or family history of a disease or illness involving a 38554, 57301 or 58324 gene.

### Use of 38554, 57301 or 58324 Molecules as Surrogate Markers

The 38554, 57301 or 58324 molecules of the invention are also useful as markers of disorders or disease states, as markers for precursors of disease states, as markers for predisposition of disease states, as markers of drug activity, or as markers of the pharmacogenomic profile of a subject. Using the methods described herein, the presence, absence and/or quantity of the 38554, 57301 or 58324 molecules of the invention can be detected, and can be correlated with one or more biological states *in vivo*. For example, the 38554, 57301 or 58324 molecules of the invention can serve as surrogate markers for one or more disorders or disease states or for conditions leading up to disease states. As used herein, a "surrogate marker" is an objective biochemical marker which correlates with the absence or presence of a disease or disorder, or with the progression of a disease or disorder (*e.g.*, with the presence or absence of a tumor). The presence or quantity of such markers is independent of the disease. Therefore, these markers can serve to indicate whether a particular course of treatment is effective in lessening a disease state or disorder. Surrogate markers are of particular use when the presence or extent of a disease state or disorder is difficult to assess through standard methodologies (*e.g.*, early stage tumors), or when an assessment of disease progression is desired before a potentially dangerous clinical endpoint is reached (*e.g.*, an assessment of cardiovascular disease can be made using cholesterol levels as a surrogate marker, and an analysis of HIV infection can be made using HIV RNA levels as a surrogate marker, well in advance of the undesirable clinical outcomes of myocardial infarction or fully-developed AIDS). Examples of the use of surrogate markers in the art include: Koomen *et al.* (2000) *J Mass. Spectrom.* 35: 258-264; and James (1994) *AIDS Treatment News Archive* 209.

The 38554, 57301 or 58324 molecules of the invention are also useful as pharmacodynamic markers. As used herein, a "pharmacodynamic marker" is an objective biochemical marker which correlates specifically with drug effects. The presence or quantity of a pharmacodynamic marker is not related to the disease state or disorder for which the drug is being administered; therefore, the presence or quantity of the marker is indicative of the presence or activity of the drug in a subject. For example, a pharmacodynamic marker can be indicative of the concentration of the drug in a biological tissue, in that the marker is either expressed or transcribed or not expressed or transcribed in that tissue in relationship to the level of the drug. In this fashion, the distribution or uptake of the drug can be monitored by the pharmacodynamic marker. Similarly, the presence or quantity of the pharmacodynamic marker can be related to the presence or quantity of the metabolic product of a drug, such that the presence or quantity of the marker is indicative of the relative breakdown rate of the drug *in vivo*. Pharmacodynamic markers are of particular use in increasing the sensitivity of detection of drug effects, particularly when the drug is administered in low doses. Since even a small amount of a drug can be sufficient to activate multiple rounds of marker (*e.g.*, a 38554, 57301 or 58324 marker) transcription or expression, the amplified marker can be in a quantity which is more readily detectable than the drug itself. Also, the marker can be more easily detected due to the nature of the marker itself; for example, using the methods described herein, anti-38554, 57301 or 58324 antibodies can be employed in an immune-based detection system for a 38554, 57301 or 58324 protein marker, or 38554, 57301 or 58324-specific radiolabeled probes can be used to detect a 38554, 57301 or 58324 mRNA marker. Furthermore, the use of a pharmacodynamic marker can offer mechanism-based prediction of risk due to drug treatment beyond the range of possible direct observations. Examples of the use of pharmacodynamic markers in the art include: Matsuda *et al.* US 6,033,862; Hattis *et al.* (1991) *Env. Health Perspect.* 90: 229-238; Schentag (1999) *Am. J Health-Syst. Pharm.* 56 Suppl. 3: S21-S24; and Nicolau (1999) *Am. J Health-Syst. Pharm.* 56 Suppl. 3: S16-S20.

The 38554, 57301 or 58324 molecules of the invention are also useful as pharmacogenomic markers. As used herein, a "pharmacogenomic marker" is an objective biochemical marker which correlates with a specific clinical drug response or susceptibility in a subject (see, *e.g.*, McLeod *et al.* (1999) *Eur. J Cancer* 35:1650-1652). The presence or quantity of the pharmacogenomic marker is related to the predicted response of the subject to a specific drug or class of drugs prior to administration of the drug. By assessing the presence or quantity of one or more pharmacogenomic markers in a subject, a drug therapy which is most appropriate for the subject, or which is predicted to have a greater degree of success, can be selected. For example, based on the presence or quantity of RNA, or protein (*e.g.*, 38554, 57301 or 58324 protein or RNA) for specific tumor markers in a subject, a drug or course of treatment can be selected that is optimized for the treatment of the specific tumor likely to be present in the subject. Similarly, the presence or absence of a specific sequence mutation in 38554, 57301 or 58324 DNA can correlate with a 38554, 57301 or 58324 drug response. The use of pharmacogenomic markers therefore permits the application of the most appropriate treatment for each subject without having to administer the therapy.

### Pharmaceutical Compositions

The nucleic acid and polypeptides, fragments thereof, as well as anti-38554, 57301 or 58324 antibodies (also referred to herein as "active compounds") of the invention can be incorporated into pharmaceutical compositions. Such compositions typically include the nucleic acid molecule, protein, or antibody and a pharmaceutically acceptable carrier. As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, *e.g.*, intravenous, intradermal, subcutaneous, oral (*e.g.*, inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, *e.g.*, gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g.*, a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g.*, with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of such compounds can be determined by standard pharmaceutical procedures in cell cultures or experimental animals, *e.g.*, for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit high therapeutic indices are preferred. While compounds that exhibit toxic side effects can be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma can be measured, for example, by high performance liquid chromatography.

As defined herein, a therapeutically effective amount of protein or polypeptide (i.e., an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The protein or polypeptide can be administered one time per week for between about 1 to 10 weeks, preferably between 2 to 8 weeks, more preferably between about 3 to 7 weeks, and even more preferably for about 4, 5, or 6 weeks. The skilled artisan will appreciate that certain factors can influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments.

For antibodies, the preferred dosage is 0.1 mg/kg of body weight (generally 10 mg/kg to 20 mg/kg). If the antibody is to act in the brain, a dosage of 50 mg/kg to 100 mg/kg is usually appropriate. Generally, partially human antibodies and fully human antibodies have a longer half-life within the human body than other antibodies. Accordingly, lower dosages and less frequent administration is often possible. Modifications such as lipidation can be used to stabilize antibodies and to enhance uptake and tissue penetration (*e.g.*, into the brain). A method for lipidation of antibodies is described by Cruikshank *et al.* ((1997) *J Acquired Immune Deficiency Syndromes and Human Retrovirology* 14:193).

The present invention encompasses agents which modulate expression or activity. An agent can, for example, be a small molecule. For example, such small molecules include, but are not limited to, peptides, peptidomimetics (*e.g.*, peptoids), amino acids, amino acid analogs, polynucleotides, polynucleotide analogs, nucleotides, nucleotide analogs, organic or inorganic compounds (i.e.,. including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

Exemplary doses include milligram or microgram amounts of the small molecule per kilogram of subject or sample weight (*e.g.*, about 1 microgram per kilogram to about 500 milligrams per kilogram, about 100 micrograms per kilogram to about 5 milligrams per kilogram, or about 1 microgram per kilogram to about 50 micrograms per kilogram. It is furthermore understood that appropriate doses of a small molecule depend upon the potency of the small molecule with respect to the expression or activity to be modulated. When one or more of these small molecules is to be administered to an animal (*e.g.*, a human) in order to modulate expression or activity of a polypeptide or nucleic acid of the invention, a physician, veterinarian, or researcher can, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

An antibody (or fragment thereof) may be conjugated to a therapeutic moiety such as a cytotoxin, a therapeutic agent or a radioactive ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, maytansinoids, *e.g.*, maytansinol (see US Patent No. 5,208,020), CC-1065 (see US Patent Nos. 5,475,092, 5,585,499, 5,846,545) and analogs or homologs thereof. Therapeutic agents include, but are not limited to, antimetabolites (*e.g.*, methotrexate, 6-mercaptopurine, 6-thioguanine, cytarabine, 5-fluorouracil decarbazine), alkylating agents (*e.g.*, mechlorethamine, thioepa chlorambucil, CC-1065, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g.*, daunorubicin (formerly daunomycin) and doxorubicin), antibiotics (*e.g.*, dactinomycin (formerly actinomycin), bleomycin, mithramycin, and anthramycin (AMC)), and anti-mitotic agents (*e.g.*, vincristine, vinblastine, taxol and maytansinoids). Radioactive ions include, but are not limited to iodine, yttrium and praseodymium.

The conjugates of the invention can be used for modifying a given biological response, the drug moiety is not to be construed as limited to classical chemical therapeutic agents. For example, the drug moiety can be a protein or polypeptide possessing a desired biological activity. Such proteins can include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophase colony stimulating factor ("GM-CSF"), granulocyte colony stimulating factor ("G-CSF"), or other growth factors.

Alternatively, an antibody can be conjugated to a second antibody to form an antibody heteroconjugate as described by Segal in U.S. Patent No. 4,676,980.

The nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see *e.g.,* Chen *et al.* (1994) *Proc. Natl. Acad. Sci. USA* 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.*, retroviral vectors, the pharmaceutical preparation can include one or more cells which produce the gene delivery system.

The pharmaceutical compositions can be included in a container, pack, or dispenser together with instructions for administration.

### Methods of Treatment:

The present invention provides for both prophylactic and therapeutic methods of treating a subject at risk of (or susceptible to) a disorder or having a disorder associated with aberrant or unwanted 38554, 57301 or 58324 expression or activity. As used herein, the term "treatment" is defined as the application or administration of a therapeutic agent to a patient, or application or administration of a therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease or the predisposition toward disease. A therapeutic agent includes, but is not limited to, small molecules, peptides, antibodies, ribozymes and antisense oligonucleotides.

With regards to both prophylactic and therapeutic methods of treatment, such treatments can be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and on the market. More specifically, the term refers the study of how a patient's genes determine his or her response to a drug (*e.g.*, a patient's "drug response phenotype", or "drug response genotype".) Thus, another aspect of the invention provides methods for tailoring an individual's prophylactic or therapeutic treatment with either the 38554, 57301 or 58324 molecules of the present invention or 38554, 57301 or 58324 modulators according to that individual's drug response genotype. Pharmacogenomics allows a clinician or physician to target prophylactic or therapeutic treatments to patients who will most benefit from the treatment and to avoid treatment of patients who will experience toxic drug-related side effects.

In one aspect, the invention provides a method for preventing in a subject, a disease or condition associated with an aberrant or unwanted 38554, 57301 or 58324 expression or activity, by administering to the subject a 38554, 57301 or 58324 or an agent which modulates 38554, 57301 or 58324 expression or at least one 38554, 57301 or 58324 activity. Subjects at risk for a disease which is caused or contributed to by aberrant or unwanted 38554, 57301 or 58324 expression or activity can be identified by, for example, any or a combination of diagnostic or prognostic assays as described herein. Administration of a prophylactic agent can occur prior to the manifestation of symptoms characteristic of the 38554, 57301 or 58324 aberrance, such that a disease or disorder is prevented or, alternatively, delayed in its progression. Depending on the type of 38554, 57301 or 58324 aberrance, for example, a 38554, 57301 or 58324, 38554, 57301 or 58324 agonist or 38554, 57301 or 58324 antagonist agent can be used for treating the subject. The appropriate agent can be determined based on screening assays described herein.

It is possible that some 38554, 57301 or 58324 disorders can be caused, at least in part, by an abnormal level of gene product, or by the presence of a gene product exhibiting abnormal activity. As such, the reduction in the level and/or activity of such gene products would bring about the amelioration of disorder symptoms.

The 38554, 57301 or 58324 molecules can act as novel diagnostic targets and therapeutic agents for controlling one or more of pain disorders, nervous system disorders, immune, *e.g.*, inflammatory disorders, testicular disorders, kidney disorders, or angiogenesis disorders, as described above, as well as organic ion transporter or other 38554-, 57301-, or 58324-associated disorders, *e.g.*, cellular proliferative and/or differentiative disorders, cardiovascular disorders, including endothelial disorders, metabolic disorders, liver disorders, disorders associated with bone metabolism, or viral diseases.

Aberrant expression and/or activity of 38554, 57301 or 58324 molecules can mediate cellular proliferative and/or differentiative disorders. Examples of cellular proliferative and/or differentiative disorders include cancer, *e.g.*, carcinoma, sarcoma, metastatic disorders or hematopoietic neoplastic disorders, *e.g.*, leukemias. A metastatic tumor can arise from a multitude of primary tumor types, including but not limited to those of prostate, colon, lung, breast and liver origin.

As used herein, the term "cancer" (also used interchangeably with the terms, "hyperproliferative" and "neoplastic") refers to cells having the capacity for autonomous growth, i.e., an abnormal state or condition characterized by rapidly proliferating cell growth. Cancerous disease states may be categorized as pathologic, i.e., characterizing or constituting a disease state, *e.g.*, malignant tumor growth, or may be categorized as non-pathologic, i.e., a deviation from normal but not associated with a disease state, *e.g.,* cell proliferation associated with wound repair. The term is meant to include all types of cancerous growths or oncogenic processes, metastatic tissues or malignantly transformed cells, tissues, or organs, irrespective of histopathologic type or stage of invasiveness. The term "cancer" includes malignancies of the various organ systems, such as those affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus. The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term "carcinoma" also includes carcinosarcomas, *e.g.*, which include malignant tumors composed of carcinomatous and sarcomatous tissues. An "adenocarcinoma" refers to a carcinoma derived from glandular tissue or in which the tumor cells form recognizable glandular structures. The term "sarcoma" is art recognized and refers to malignant tumors of mesenchymal derivation.

The 38554, 57301 or 58324 molecules of the invention can be used to monitor, treat and/or diagnose a variety of proliferative disorders. Such disorders include hematopoietic neoplastic disorders. As used herein, the term "hematopoietic neoplastic disorders" includes diseases involving hyperplastic/neoplastic cells of hematopoietic origin, *e.g.*, arising from myeloid, lymphoid or erythroid lineages, or precursor cells thereof. Preferably, the diseases arise from poorly differentiated acute leukemias, *e.g.*, erythroblastic leukemia and acute megakaryoblastic leukemia. Additional exemplary myeloid disorders include, but are not limited to, acute promyeloid leukemia (APML), acute myelogenous leukemia (AML) and chronic myelogenous leukemia (CML) (reviewed in Vaickus, L. (1991) *Crit Rev. in Oncol.*/*Hemotol.* 11:267-97); lymphoid malignancies include, but are not limited to acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease.

Aberrant expression and/or activity of 38554, 57301 or 58324 molecules can mediate cardiovascular disorders. Examples of disorders involving the heart or "cardiovascular disorder" include, but are not limited to, a disease, disorder, or state involving the cardiovascular system, *e.g.*, the heart, the blood vessels, and/or the blood. A cardiovascular disorder can be caused by an imbalance in arterial pressure, a malfunction of the heart, or an occlusion of a blood vessel, *e.g.*, by a thrombus. Examples of cardiovascular disorders include but are not limited to, hypertension, atherosclerosis, coronary artery spasm, coronary artery disease, arrhythmias, heart failure, including but not limited to, cardiac hypertrophy, left-sided heart failure, and right-sided heart failure; ischemic heart disease, including but not limited to angina pectoris, myocardial infarction, chronic ischemic heart disease, and sudden cardiac death; hypertensive heart disease, including but not limited to, systemic (left-sided) hypertensive heart disease and pulmonary (right-sided) hypertensive heart disease; valvular heart disease, including but not limited to, valvular degeneration caused by calcification, such as calcification of a congenitally bicuspid aortic valve, and mitral annular calcification, and myxomatous degeneration of the mitral valve (mitral valve prolapse), rheumatic fever and rheumatic heart disease, infective endocarditis, and noninfected vegetations, such as nonbacterial thrombotic endocarditis and endocarditis of systemic lupus erythematosus (Libman-Sacks disease), carcinoid heart disease, and complications of artificial valves; myocardial disease, including but not limited to dilated cardiomyopathy, hypertrophic cardiomyopathy, restrictive cardiomyopathy, and myocarditis; pericardial disease, including but not limited to, pericardial effusion and hemopericardium and pericarditis, including acute pericarditis and healed pericarditis, and rheumatoid heart disease; neoplastic heart disease, including but not limited to, primary cardiac tumors, such as myxoma, lipoma, papillary fibroelastoma, rhabdomyoma, and sarcoma, and cardiac effects of noncardiac neoplasms; congenital heart disease, including but not limited to, left-to-right shunts--late cyanosis, such as atrial septal defect, ventricular septal defect, patent ductus arteriosus, and atrioventricular septal defect, right-to-left shunts--early cyanosis, such as tetralogy of fallot, transposition of great arteries, truncus arteriosus, tricuspid atresia, and total anomalous pulmonary venous connection, obstructive congenital anomalies, such as coarctation of aorta, pulmonary stenosis and atresia, and aortic stenosis and atresia, disorders involving cardiac transplantation, and congestive heart failure. A cardiovasular disease or disorder also includes an endothelial cell disorder.

As used herein, an "endothelial cell disorder" includes a disorder characterized by aberrant, unregulated, or unwanted endothelial cell activity, *e.g.,* proliferation, migration, angiogenesis, or vascularization; or aberrant expression of cell surface adhesion molecules or genes associated with angiogenesis, *e.g.,* TIE-2, FLT and FLK. Endothelial cell disorders include tumorigenesis, tumor metastasis, psoriasis, diabetic retinopathy, endometriosis, Grave's disease, ischemic disease *(e.g.,* atherosclerosis), and chronic inflammatory diseases (*e.g.*, rheumatoid arthritis).

Organic ion transport-associated or related disorders also include hormonal disorders, such as conditions or diseases in which the production and/or regulation of hormones in an organism is aberrant. Examples of such disorders and diseases include type I and type II diabetes mellitus, pituitary disorders (*e.g.*, growth disorders), thyroid disorders (*e.g.*, hypothyroidism or hyperthyroidism), and reproductive or fertility disorders (*e.g.*, disorders which affect the organs of the reproductive system, *e.g.*, the prostate gland, the uterus, or the vagina; disorders which involve an imbalance in the levels of a reproductive hormone in a subject; disorders affecting the ability of a subject to reproduce; and disorders affecting secondary sex characteristic development, *e.g.*, adrenal hyperplasia).

Organic ion transport-associated or related disorders also include disorders in the regulation of metabolism. Diseases of metabolic imbalance include, but are not limited to, obesity, anorexia nervosa, cachexia, lipid disorders, and diabetes.

Disorders which can be treated or diagnosed by methods described herein include, but are not limited to, disorders associated with an accumulation in the liver of fibrous tissue, such as that resulting from an imbalance between production and degradation of the extracellular matrix accompanied by the collapse and condensation of preexisting fibers. The methods described herein can be used to diagnose or treat hepatocellular necrosis or injury induced by a wide variety of agents including processes which disturb homeostasis, such as an inflammatory process, tissue damage resulting from toxic injury or altered hepatic blood flow, and infections (*e.g.*, bacterial, viral and parasitic). For example, the methods can be used for the early detection of hepatic injury, such as portal hypertension or hepatic fibrosis. In addition, the methods can be employed to detect liver fibrosis attributed to inborn errors of metabolism, for example, fibrosis resulting from a storage disorder such as Gaucher's disease (lipid abnormalities) or a glycogen storage disease, A1-antitrypsin deficiency; a disorder mediating the accumulation (*e.g.*, storage) of an exogenous substance, for example, hemochromatosis (iron-overload syndrome) and copper storage diseases (Wilson's disease), disorders resulting in the accumulation of a toxic metabolite (*e.g.*, tyrosinemia, fructosemia and galactosemia) and peroxisomal disorders (*e.g.*, Zellweger syndrome). Additionally, the methods described herein can be used for the early detection and treatment of liver injury associated with the administration of various chemicals or drugs, such as for example, methotrexate, isonizaid, oxyphenisatin, methyldopa, chlorpromazine, tolbutamide or alcohol, or which represents a hepatic manifestation of a vascular disorder such as obstruction of either the intrahepatic or extrahepatic bile flow or an alteration in hepatic circulation resulting, for example, from chronic heart failure, veno-occlusive disease, portal vein thrombosis or Budd-Chiari syndrome.

Aberrant expression and/or activity of 38554, 57301 or 58324 molecules can mediate disorders associated with bone metabolism. "Bone metabolism" refers to direct or indirect effects in the formation or degeneration of bone structures, *e.g.*, bone formation, bone resorption, etc., which can ultimately affect the concentrations in serum of calcium and phosphate. This term also includes activities mediated by 38554, 57301 or 58324 molecules effects in bone cells, *e.g.* osteoclasts and osteoblasts, that can in turn result in bone formation and degeneration. For example, 38554, 57301 or 58324 molecules can support different activities of bone resorbing osteoclasts such as the stimulation of differentiation of monocytes and mononuclear phagocytes into osteoclasts. Accordingly, 38554, 57301 or 58324 molecules that modulate the production of bone cells can influence bone formation and degeneration, and thus can be used to treat bone disorders. Examples of such disorders include, but are not limited to, osteoporosis, osteodystrophy, osteomalacia, rickets, osteitis fibrosa cystica, renal osteodystrophy, osteosclerosis, anti-convulsant treatment, osteopenia, fibrogenesis-imperfecta ossium, secondary hyperparathyrodism, hypoparathyroidism, hyperparathyroidism, cirrhosis, obstructive jaundice, drug induced metabolism, medullary carcinoma, chronic renal disease, rickets, sarcoidosis, glucocorticoid antagonism, malabsorption syndrome, steatorrhea, tropical sprue, idiopathic hypercalcemia and milk fever.

Additionally, 38554, 57301 or 58324 molecules can play an important role in the etiology of certain viral diseases, including but not limited to Hepatitis B, Hepatitis C and Herpes Simplex Virus (HSV). Modulators of 38554, 57301 or 58324 activity could be used to control viral diseases. The modulators can be used in the treatment and/or diagnosis of viral infected tissue or virus-associated tissue fibrosis, especially liver and liver fibrosis. Also, 38554, 57301 or 58324 modulators can be used in the treatment and/or diagnosis of virus-associated carcinoma, especially hepatocellular cancer.

As discussed, successful treatment of 38554, 57301 or 58324 disorders can be brought about by techniques that serve to inhibit the expression or activity of target gene products. For example, compounds, *e.g.*, an agent identified using an assays described above, that proves to exhibit negative modulatory activity, can be used in accordance with the invention to prevent and/or ameliorate symptoms of 38554, 57301 or 58324 disorders. Such molecules can include, but are not limited to peptides, phosphopeptides, small organic or inorganic molecules, or antibodies (including, for example, polyclonal, monoclonal, humanized, human, anti-idiotypic, chimeric or single chain antibodies, and Fab, F(ab')₂ and Fab expression library fragments, scFV molecules, and epitope-binding fragments thereof).

Further, antisense and ribozyme molecules that inhibit expression of the target gene can also be used in accordance with the invention to reduce the level of target gene expression, thus effectively reducing the level of target gene activity. Still further, triple helix molecules can be utilized in reducing the level of target gene activity. Antisense, ribozyme and triple helix molecules are discussed above.

It is possible that the use of antisense, ribozyme, and/or triple helix molecules to reduce or inhibit mutant gene expression can also reduce or inhibit the transcription (triple helix) and/or translation (antisense, ribozyme) of mRNA produced by normal target gene alleles, such that the concentration of normal target gene product present can be lower than is necessary for a normal phenotype. In such cases, nucleic acid molecules that encode and express target gene polypeptides exhibiting normal target gene activity can be introduced into cells via gene therapy method. Alternatively, in instances in that the target gene encodes an extracellular protein, it can be preferable to co-administer normal target gene protein into the cell or tissue in order to maintain the requisite level of cellular or tissue target gene activity.

Another method by which nucleic acid molecules can be utilized in treating or preventing a disease characterized by 38554, 57301 or 58324 expression is through the use of aptamer molecules specific for 38554, 57301 or 58324 protein. Aptamers are nucleic acid molecules having a tertiary structure which permits them to specifically or selectively bind to protein ligands (see, *e.g.*, Osborne, *et al.* (1997) *Curr. Opin. Chem Biol.* 1: 5-9; and Patel, D.J. (1997) *Curr Opin Chem Biol* 1:32-46). Since nucleic acid molecules can in many cases be more conveniently introduced into target cells than therapeutic protein molecules can be, aptamers offer a method by which 38554, 57301 or 58324 protein activity can be specifically decreased without the introduction of drugs or other molecules which can have pluripotent effects.

Antibodies can be generated that are both specific for target gene product and that reduce target gene product activity. Such antibodies can, therefore, by administered in instances whereby negative modulatory techniques are appropriate for the treatment of 38554, 57301 or 58324 disorders. For a description of antibodies, see the Antibody section above.

In circumstances wherein injection of an animal or a human subject with a 38554, 57301 or 58324 protein or epitope for stimulating antibody production is harmful to the subject, it is possible to generate an immune response against 38554, 57301 or 58324 through the use of anti-idiotypic antibodies (see, for example, Herlyn, D. (1999) *Ann Med* 31:66-78; and Bhattacharya-Chatterjee, M., and Foon, K.A. (1998) *Cancer Treat Res.* 94:51-68). If an anti-idiotypic antibody is introduced into a mammal or human subject, it should stimulate the production of anti-anti-idiotypic antibodies, which should be specific to the 38554, 57301 or 58324 protein. Vaccines directed to a disease characterized by 38554, 57301 or 58324 expression can also be generated in this fashion.

In instances where the target antigen is intracellular and whole antibodies are used, internalizing antibodies can be preferred. Lipofectin or liposomes can be used to deliver the antibody or a fragment of the Fab region that binds to the target antigen into cells. Where fragments of the antibody are used, the smallest inhibitory fragment that binds to the target antigen is preferred. For example, peptides having an amino acid sequence corresponding to the Fv region of the antibody can be used. Alternatively, single chain neutralizing antibodies that bind to intracellular target antigens can also be administered. Such single chain antibodies can be administered, for example, by expressing nucleotide sequences encoding single-chain antibodies within the target cell population (see *e.g.*, Marasco *et al.* (1993) *Proc. Natl. Acad. Sci. USA* 90:7889-7893).

The identified compounds that inhibit target gene expression, synthesis and/or activity can be administered to a patient at therapeutically effective doses to prevent, treat or ameliorate 38554, 57301 or 58324 disorders. A therapeutically effective dose refers to that amount of the compound sufficient to result in amelioration of symptoms of the disorders. Toxicity and therapeutic efficacy of such compounds can be determined by standard pharmaceutical procedures as described above.

The data obtained from the cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma can be measured, for example, by high performance liquid chromatography.

Another example of determination of effective dose for an individual is the ability to directly assay levels of "free" and "bound" compound in the serum of the test subject. Such assays can utilize antibody mimics and/or "biosensors" that have been created through molecular imprinting techniques. The compound which is able to modulate 38554, 57301 or 58324 activity is used as a template, or "imprinting molecule", to spatially organize polymerizable monomers prior to their polymerization with catalytic reagents. The subsequent removal of the imprinted molecule leaves a polymer matrix which contains a repeated "negative image" of the compound and is able to selectively rebind the molecule under biological assay conditions. A detailed review of this technique can be seen in Ansell, R. J. *et al* (1996) *Current Opinion in Biotechnology* 7:89-94 and in Shea, K.J. (1994) *Trends in Polymer Science* 2:166-173. Such "imprinted" affinity matrixes are amenable to ligand-binding assays, whereby the immobilized monoclonal antibody component is replaced by an appropriately imprinted matrix. An example of the use of such matrixes in this way can be seen in Vlatakis, G. *et al* (1993) *Nature* 361:645-647. Through the use of isotope-labeling, the "free" concentration of compound which modulates the expression or activity of 38554, 57301 or 58324 can be readily monitored and used in calculations of IC₅₀.

Such "imprinted" affinity matrixes can also be designed to include fluorescent groups whose photon-emitting properties measurably change upon local and selective binding of target compound. These changes can be readily assayed in real time using appropriate fiberoptic devices, in turn allowing the dose in a test subject to be quickly optimized based on its individual IC₅₀. An rudimentary example of such a "biosensor" is discussed in Kriz, D. *et al* (1995) *Analytical Chemistry* 67:2142-2144.

Another aspect of the invention pertains to methods of modulating 38554, 57301 or 58324 expression or activity for therapeutic purposes. Accordingly, in an exemplary embodiment, the modulatory method of the invention involves contacting a cell with a 38554, 57301 or 58324 or agent that modulates one or more of the activities of 38554, 57301 or 58324 protein activity associated with the cell. An agent that modulates 38554, 57301 or 58324 protein activity can be an agent as described herein, such as a nucleic acid or a protein, a naturally-occurring target molecule of a 38554, 57301 or 58324 protein (*e.g.*, a 38554, 57301 or 58324 substrate or receptor), a 38554, 57301 or 58324 antibody, a 38554, 57301 or 58324 agonist or antagonist, a peptidomimetic of a 38554, 57301 or 58324 agonist or antagonist, or other small molecule.

In one embodiment, the agent stimulates one or 38554, 57301 or 58324 activities. Examples of such stimulatory agents include active 38554, 57301 or 58324 protein and a nucleic acid molecule encoding 38554, 57301 or 58324. In another embodiment, the agent inhibits one or more 38554, 57301 or 58324 activities. Examples of such inhibitory agents include antisense 38554, 57301 or 58324 nucleic acid molecules, anti38554, 57301 or 58324 antibodies, and 38554, 57301 or 58324 inhibitors. These modulatory methods can be performed *in vitro* (*e.g.*, by culturing the cell with the agent) or, alternatively, *in vivo* (*e.g.*, by administering the agent to a subject). As such, the present invention provides methods of treating an individual afflicted with a disease or disorder characterized by aberrant or unwanted expression or activity of a 38554, 57301 or 58324 protein or nucleic acid molecule. In one embodiment, the method involves administering an agent (*e.g.*, an agent identified by a screening assay described herein), or combination of agents that modulates (*e.g.*, up regulates or down regulates) 38554, 57301 or 58324 expression or activity. In another embodiment, the method involves administering a 38554, 57301 or 58324 protein or nucleic acid molecule as therapy to compensate for reduced, aberrant, or unwanted 38554, 57301 or 58324 expression or activity.

Stimulation of 38554, 57301 or 58324 activity is desirable in situations in which 38554, 57301 or 58324 is abnormally downregulated and/or in which increased 38554, 57301 or 58324 activity is likely to have a beneficial effect. For example, stimulation of 38554, 57301 or 58324 activity is desirable in situations in which a 38554, 57301 or 58324 is downregulated and/or in which increased 38554, 57301 or 58324 activity is likely to have a beneficial effect. Likewise, inhibition of 38554, 57301 or 58324 activity is desirable in situations in which 38554, 57301 or 58324 is abnormally upregulated and/or in which decreased 38554, 57301 or 58324 activity is likely to have a beneficial effect.

### Pharmacogenomics

The 38554, 57301 or 58324 molecules of the present invention, as well as agents, or modulators which have a stimulatory or inhibitory effect on 38554, 57301 or 58324 activity (*e.g.*, 38554, 57301 or 58324 gene expression) as identified by a screening assay described herein can be administered to individuals to treat (prophylactically or therapeutically) 38554, 57301 or 58324 associated disorders (*e.g.*, organic ion transport, organic ion absorption or excretion, inter- or intra-cellular signaling, and/or hormonal responses disorders) associated with aberrant or unwanted 38554, 57301 or 58324 activity. In conjunction with such treatment, pharmacogenomics (i.e., the study of the relationship between an individual's genotype and that individual's response to a foreign compound or drug) can be considered. Differences in metabolism of therapeutics can lead to severe toxicity or therapeutic failure by altering the relation between dose and blood concentration of the pharmacologically active drug. Thus, a physician or clinician can consider applying knowledge obtained in relevant pharmacogenomics studies in determining whether to administer a 38554, 57301 or 58324 molecule or 38554, 57301 or 58324 modulator as well as tailoring the dosage and/or therapeutic regimen of treatment with a 38554, 57301 or 58324 molecule or 38554, 57301 or 58324 modulator.

Pharmacogenomics deals with clinically significant hereditary variations in the response to drugs due to altered drug disposition and abnormal action in affected persons. See, for example, Eichelbaum, M. *et al.* (1996) *Clin. Exp. Pharmacol. Physiol.* 23:983-985 and Linder, M.W. *et al.* (1997) *Clin. Chem.* 43:254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as "a genome-wide association", relies primarily on a high-resolution map of the human genome consisting of already known gene-related markers (*e.g.*, a "bi-allelic" gene marker map which consists of 60,000-100,000 polymorphic or variable sites on the human genome, each of which has two variants.) Such a high-resolution genetic map can be compared to a map of the genome of each of a statistically significant number of patients taking part in a Phase II/III drug trial to identify markers associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP can occur once per every 1000 bases of DNA. A SNP can be involved in a disease process, however, the vast majority can not be disease-associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that can be common among such genetically similar individuals.

Alternatively, a method termed the "candidate gene approach", can be utilized to identify genes that predict drug response. According to this method, if a gene that encodes a drug's target is known (*e.g.*, a 38554, 57301 or 58324 protein of the present invention), all common variants of that gene can be fairly easily identified in the population and it can be determined if having one version of the gene versus another is associated with a particular drug response.

Alternatively, a method termed the "gene expression profiling", can be utilized to identify genes that predict drug response. For example, the gene expression of an animal dosed with a drug (*e.g.*, a 38554, 57301 or 58324 molecule or 38554, 57301 or 58324 modulator of the present invention) can give an indication whether gene pathways related to toxicity have been turned on.

Information generated from more than one of the above pharmacogenomics approaches can be used to determine appropriate dosage and treatment regimens for prophylactic or therapeutic treatment of an individual. This knowledge, when applied to dosing or drug selection, can avoid adverse reactions or therapeutic failure and thus enhance therapeutic or prophylactic efficiency when treating a subject with a 38554, 57301 or 58324 molecule or 38554, 57301 or 58324 modulator, such as a modulator identified by one of the exemplary screening assays described herein.

The present invention further provides methods for identifying new agents, or combinations, that are based on identifying agents that modulate the activity of one or more of the gene products encoded by one or more of the 38554, 57301 or 58324 genes of the present invention, wherein these products can be associated with resistance of the cells to a therapeutic agent. Specifically, the activity of the proteins encoded by the 38554, 57301 or 58324 genes of the present invention can be used as a basis for identifying agents for overcoming agent resistance. By blocking the activity of one or more of the resistance proteins, target cells, *e.g.*, human cells, will become sensitive to treatment with an agent that the unmodified target cells were resistant to.

Monitoring the influence of agents (*e.g.*, drugs) on the expression or activity of a 38554, 57301 or 58324 protein can be applied in clinical trials. For example, the effectiveness of an agent determined by a screening assay as described herein to increase 38554, 57301 or 58324 gene expression, protein levels, or upregulate 38554, 57301 or 58324 activity, can be monitored in clinical trials of subjects exhibiting decreased 38554, 57301 or 58324 gene expression, protein levels, or downregulated 38554, 57301 or 58324 activity. Alternatively, the effectiveness of an agent determined by a screening assay to decrease 38554, 57301 or 58324 gene expression, protein levels, or downregulate 38554, 57301 or 58324 activity, can be monitored in clinical trials of subjects exhibiting increased 38554, 57301 or 58324 gene expression, protein levels, or upregulated 38554, 57301 or 58324 activity. In such clinical trials, the expression or activity of a 38554, 57301 or 58324 gene, and preferably, other genes that have been implicated in, for example, a 38554-, 57301- or 58324-associated disorder can be used as a "read out" or markers of the phenotype of a particular cell.

### Other Embodiments

In another aspect, the invention features a method of analyzing a plurality of capture probes. The method is useful, *e.g.*, to analyze gene expression. The method includes: providing a two dimensional array having a plurality of addresses, each address of the plurality being positionally distinguishable from each other address of the plurality, and each address of the plurality having a unique capture probe, *e.g.*, a nucleic acid or peptide sequence, wherein the capture probes are from a cell or subject which expresses 38554, 57301 or 58324 or from a cell or subject in which a 38554, 57301 or 58324 mediated response has been elicited; contacting the array with a 38554, 57301 or 58324 nucleic acid (preferably purified), a 38554, 57301 or 58324 polypeptide (preferably purified), or an anti-38554, 57301 or 58324 antibody, and thereby evaluating the plurality of capture probes. Binding, *e.g.*, in the case of a nucleic acid, hybridization with a capture probe at an address of the plurality, is detected, *e.g.*, by a signal generated from a label attached to the 38554, 57301 or 58324 nucleic acid, polypeptide, or antibody.

The capture probes can be a set of nucleic acids from a selected sample, *e.g.*, a sample of nucleic acids derived from a control or non-stimulated tissue or cell.

The method can include contacting the 38554, 57301 or 58324 nucleic acid, polypeptide, or antibody with a first array having a plurality of capture probes and a second array having a different plurality of capture probes. The results of each hybridization can be compared, *e.g.*, to analyze differences in expression between a first and second sample. The first plurality of capture probes can be from a control sample, *e.g.*, a wild type, normal, or non-diseased, non-stimulated, sample, *e.g.*, a biological fluid, tissue, or cell sample. The second plurality of capture probes can be from an experimental sample, *e.g.*, a mutant type, at risk, disease-state or disorder-state, or stimulated, sample, *e.g.*, a biological fluid, tissue, or cell sample.

The plurality of capture probes can be a plurality of nucleic acid probes each of which specifically hybridizes, with an allele of 38554, 57301 or 58324. Such methods can be used to diagnose a subject, *e.g.*, to evaluate risk for a disease or disorder, to evaluate suitability of a selected treatment for a subject, to evaluate whether a subject has a disease or disorder.

The method can be used to detect SNPs, as described above.

In another aspect, the invention features, a method of analyzing 38554, 57301 or 58324, *e.g.*, analyzing structure, function, or relatedness to other nucleic acid or amino acid sequences. The method includes: providing a 38554, 57301 or 58324 nucleic acid or amino acid sequence; comparing the 38554, 57301 or 58324 sequence with one or more preferably a plurality of sequences from a collection of sequences, *e.g.*, a nucleic acid or protein sequence database; to thereby analyze 38554, 57301 or 58324.

The method can include evaluating the sequence identity between a 38554, 57301 or 58324 sequence and a database sequence. The method can be performed by accessing the database at a second site, *e.g.*, over the internet. Preferred databases include GenBank™ and SwissProt.

In another aspect, the invention features, a set of oligonucleotides, useful, *e.g.*, for identifying SNP's, or identifying specific alleles of 38554, 57301 or 58324. The set includes a plurality of oligonucleotides, each of which has a different nucleotide at an interrogation position, *e.g.*, an SNP or the site of a mutation. In a preferred embodiment, the oligonucleotides of the plurality identical in sequence with one another (except for differences in length). The oligonucleotides can be provided with differential labels, such that an oligonucleotide which hybridizes to one allele provides a signal that is distinguishable from an oligonucleotides which hybridizes to a second allele.

The sequences of 38554, 57301 or 58324 molecules are provided in a variety of mediums to facilitate use thereof. A sequence can be provided as a manufacture, other than an isolated nucleic acid or amino acid molecule, which contains a 38554, 57301 or 58324 molecule. Such a manufacture can provide a nucleotide or amino acid sequence, *e.g.,* an open reading frame, in a form which allows examination of the manufacture using means not directly applicable to examining the nucleotide or amino acid sequences, or a subset thereof, as they exist in nature or in purified form.

A 38554, 57301 or 58324 nucleotide or amino acid sequence can be recorded on computer readable media. As used herein, "computer readable media" refers to any medium that can be read and accessed directly by a computer. Such media include, but are not limited to: magnetic storage media, such as floppy discs, hard disc storage medium, and magnetic tape; optical storage media such as compact disc and CD-ROM; electrical storage media such as RAM, ROM, EPROM, EEPROM, and the like; and general hard disks and hybrids of these categories such as magnetic/optical storage media. The medium is adapted or configured for having thereon 38554, 57301 or 58324 sequence information of the present invention.

As used herein, the term "electronic apparatus" is intended to include any suitable computing or processing apparatus of other device configured or adapted for storing data or information. Examples of electronic apparatus suitable for use with the present invention include stand-alone computing apparatus; networks, including a local area network (LAN), a wide area network (WAN) Internet, Intranet, and Extranet; electronic appliances such as personal digital assistants (PDAs), cellular phones, pagers, and the like; and local and distributed processing systems.

As used herein, "recorded" refers to a process for storing or encoding information on the electronic apparatus readable medium. Those skilled in the art can readily adopt any of the presently known methods for recording information on known media to generate manufactures comprising the 38554, 57301 or 58324 sequence information.

A variety of data storage structures are available to a skilled artisan for creating a computer readable medium having recorded thereon a 38554, 57301 or 58324 nucleotide or amino acid sequence of the present invention. The choice of the data storage structure will generally be based on the means chosen to access the stored information. In addition, a variety of data processor programs and formats can be used to store the nucleotide sequence information of the present invention on computer readable medium. The sequence information can be represented in a word processing text file, formatted in commercially-available software such as WordPerfect and Microsoft Word, or represented in the form of an ASCII file, stored in a database application, such as DB2, Sybase, Oracle, or the like. The skilled artisan can readily adapt any number of data processor structuring formats (*e.g.*, text file or database) in order to obtain computer readable medium having recorded thereon the nucleotide sequence information of the present invention.

By providing the 38554, 57301 or 58324 nucleotide or amino acid sequences of the invention in computer readable form, the skilled artisan can routinely access the sequence information for a variety of purposes. For example, one skilled in the art can use the nucleotide or amino acid sequences of the invention in computer readable form to compare a target sequence or target structural motif with the sequence information stored within the data storage means. A search is used to identify fragments or regions of the sequences of the invention which match a particular target sequence or target motif.

The present invention therefore provides a medium for holding instructions for performing a method for determining whether a subject has a transporter-associated or another38554-, 57301- or 58324-associated disease or disorder or a pre-disposition to a transporter-associated or another38554-, 57301- or 58324-associated disease or disorder, wherein the method comprises the steps of determining 38554, 57301 or 58324 sequence information associated with the subject and based on the 38554, 57301 or 58324 sequence information, determining whether the subject has a transporter-associated or another38554-, 57301- or 58324-associated disease or disorder and/or recommending a particular treatment for the disease, disorder, or pre-disease condition.

The present invention further provides in an electronic system and/or in a network, a method for determining whether a subject has a transporter-associated or another38554-, 57301- or 58324-associated disease or disorder or a pre-disposition to a disease associated with 38554, 57301 or 58324, wherein the method comprises the steps of determining 38554, 57301 or 58324 sequence information associated with the subject, and based on the 38554, 57301 or 58324 sequence information, determining whether the subject has a 38554-, 57301- or 58324-associated disease or disorder or a pre-disposition to a 38554-, 57301- or 58324-associated disease or disorder, and/or recommending a particular treatment for the disease, disorder, or pre-disease condition. The method may further comprise the step of receiving phenotypic information associated with the subject and/or acquiring from a network phenotypic information associated with the subject.

The present invention also provides in a network, a method for determining whether a subject has a 38554-, 57301- or 58324-associated disease or disorder or a pre-disposition to a 38554-, 57301- or 58324-associated disease or disorder, said method comprising the steps of receiving 38554, 57301 or 58324 sequence information from the subject and/or information related thereto, receiving phenotypic information associated with the subject, acquiring information from the network corresponding to 38554, 57301 or 58324 and/or corresponding to a transporter-associated or another38554-, 57301- or 58324-associated disease or disorder, and based on one or more of the phenotypic information, the 38554, 57301 or 58324 information (*e.g.*, sequence information and/or information related thereto), and the acquired information, determining whether the subject has a 38554-, 57301- or 58324-associated disease or disorder or a pre-disposition to a 38554-, 57301- or 58324-associated disease or disorder. The method may further comprise the step of recommending a particular treatment for the disease, disorder, or pre-disease condition.

The present invention also provides a business method for determining whether a subject has a 38554-, 57301- or 58324-associated disease or disorder or a pre-disposition to a 38554-, 57301- or 58324-associated disease or disorder, said method comprising the steps of receiving information related to 38554, 57301 or 58324 (*e.g.*, sequence information and/or information related thereto), receiving phenotypic information associated with the subject, acquiring information from the network related to 38554, 57301 or 58324 and/or related to a 38554-, 57301- or 58324-associated disease or disorder, and based on one or more of the phenotypic information, the 38554, 57301 or 58324 information, and the acquired information, determining whether the subject has a 38554-, 57301- or 58324-associated disease or disorder or a pre-disposition to a 38554-, 57301- or 58324-associated disease or disorder. The method may further comprise the step of recommending a particular treatment for the disease, disorder, or pre-disease condition.

The invention also includes an array comprising a 38554, 57301 or 58324 sequence of the present invention. The array can be used to assay expression of one or more genes in the array. In one embodiment, the array can be used to assay gene expression in a tissue to ascertain tissue specificity of genes in the array. In this manner, up to about 7600 genes can be simultaneously assayed for expression, one of which can be 38554, 57301 or 58324. This allows a profile to be developed showing a battery of genes specifically expressed in one or more tissues.

In addition to such qualitative information, the invention allows the quantitation of gene expression. Thus, not only tissue specificity, but also the level of expression of a battery of genes in the tissue if ascertainable. Thus, genes can be grouped on the basis of their tissue expression *per se* and level of expression in that tissue. This is useful, for example, in ascertaining the relationship of gene expression in that tissue. Thus, one tissue can be perturbed and the effect on gene expression in a second tissue can be determined. In this context, the effect of one cell type on another cell type in response to a biological stimulus can be determined. In this context, the effect of one cell type on another cell type in response to a biological stimulus can be determined. Such a determination is useful, for example, to know the effect of cell-cell interaction at the level of gene expression. If an agent is administered therapeutically to treat one cell type but has an undesirable effect on another cell type, the invention provides an assay to determine the molecular basis of the undesirable effect and thus provides the opportunity to co-administer a counteracting agent or otherwise treat the undesired effect. Similarly, even within a single cell type, undesirable biological effects can be determined at the molecular level. Thus, the effects of an agent on expression of other than the target gene can be ascertained and counteracted.

In another embodiment, the array can be used to monitor the time course of expression of one or more genes in the array. This can occur in various biological contexts, as disclosed herein, for example development of a transporter-associated or another38554-, 57301- or 58324-associated disease or disorder, progression of transporter-associated or another38554-, 57301- or 58324-associated disease or disorder, and processes, such a cellular transformation associated with the transporter-associated or another38554-, 57301-or 58324-associated disease or disorder.

The array is also useful for ascertaining the effect of the expression of a gene on the expression of other genes in the same cell or in different cells (*e.g.*, acertaining the effect of 38554, 57301 or 58324 expression on the expression of other genes). This provides, for example, for a selection of alternate molecular targets for therapeutic intervention if the ultimate or downstream target cannot be regulated.

The array is also useful for ascertaining differential expression patterns of one or more genes in normal and abnormal cells. This provides a battery of genes (*e.g.*, including 38554, 57301 or 58324) that could serve as a molecular target for diagnosis or therapeutic intervention.

As used herein, a "target sequence" can be any DNA or amino acid sequence of six or more nucleotides or two or more amino acids. A skilled artisan can readily recognize that the longer a target sequence is, the less likely a target sequence will be present as a random occurrence in the database. Typical sequence lengths of a target sequence are from about 10 to 100 amino acids or from about 30 to 300 nucleotide residues. However, it is well recognized that commercially important fragments, such as sequence fragments involved in gene expression and protein processing, may be of shorter length.

Computer software is publicly available which allows a skilled artisan to access sequence information provided in a computer readable medium for analysis and comparison to other sequences. A variety of known algorithms are disclosed publicly and a variety of commercially available software for conducting search means are and can be used in the computer-based systems of the present invention. Examples of such software include, but are not limited to, MacPattern (EMBL), BLASTN and BLASTX (NCBI).

Thus, the invention features a method of making a computer readable record of a sequence of a 38554, 57301 or 58324 sequence which includes recording the sequence on a computer readable matrix. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

In another aspect, the invention features a method of analyzing a sequence. The method includes: providing a 38554, 57301 or 58324 sequence, or record, in computer readable form; comparing a second sequence to the 38554, 57301 or 58324 sequence; thereby analyzing a sequence. Comparison can include comparing to sequences for sequence identity or determining if one sequence is included within the other, *e.g.*, determining if the 38554, 57301 or 58324 sequence includes a sequence being compared. In a preferred embodiment the 38554, 57301 or 58324 or second sequence is stored on a first computer, *e.g.*, at a first site and the comparison is performed, read, or recorded on a second computer, *e.g.,* at a second site. *E.g.,* the 38554, 57301 or 58324 or second sequence can be stored in a public or proprietary database in one computer, and the results of the comparison performed, read, or recorded on a second computer. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

This invention is further illustrated by the following exemplification, which should not be construed as limiting.

### EXEMPLIFICATION

### Gene Expression Analysis

The expression of 38554, 57301 and 58324 mRNA in various human tissues was analyzed. Total RNA was prepared from various human tissues by a single step extraction method using RNA STAT-60 according to the manufacturer's instructions (TelTest, Inc). Each RNA preparation was treated with DNase I (Ambion) at 37°C for 1 hour. DNAse I treatment was determined to be complete if the sample required at least 38 PCR amplification cycles to reach a threshold level of fluorescence using β-2 microglobulin as an internal amplicon reference. The integrity of the RNA samples following DNase I treatment was confirmed by agarose gel electrophoresis and ethidium bromide staining. After phenol extraction cDNA was prepared from the sample using the SUPERSCRIPT™ Choice System following the manufacturer's instructions (GibcoBRL). A negative control of RNA without reverse transcriptase was mock reverse transcribed for each RNA sample.

Human 38554, 57301 and 58324 expression was measured by TaqMan® quantitative PCR (Perkin Elmer Applied Biosystems) in cDNA prepared from a variety of normal and diseased (e.g., cancerous) human tissues or cell lines.

Probes were designed by PrimerExpress software (PE Biosystems) based on the sequence of the human 38554, 57301 and 58324 genes. Each human 38554, 57301 and 58324 gene probe was labeled using FAM (6-carboxyfluorescein), and the β2-microglobulin reference probe was labeled with a different fluorescent dye, VIC. The differential labeling of the target gene and internal reference gene thus enabled measurement in same well. Forward and reverse primers and the probes for both β2-microglobulin and target gene were added to the TaqMan® Universal PCR Master Mix (PE Applied Biosystems). Although the final concentration of primer and probe could vary, each was internally consistent within a given experiment. A typical experiment contained 200nM of forward and reverse primers plus 100nM probe for β-2 microglobulin and 600 nM forward and reverse primers plus 200 nM probe for the target gene. TaqMan matrix experiments were carried out on an ABI PRISM 7700 Sequence Detection System (PE Applied Biosystems). The thermal cycler conditions were as follows: hold for 2 min at 50°C and 10 min at 95°C, followed by two-step PCR for 40 cycles of 95°C for 15 sec followed by 60°C for 1 min.

The following method was used to quantitatively calculate human 38554, 57301 and 58324 gene expression in the various tissues relative to β-2 microglobulin expression in the same tissue. The threshold cycle (Ct) value is defined as the cycle at which a statistically significant increase in fluorescence is detected. A lower Ct value is indicative of a higher mRNA concentration. The Ct value of the human 38554, 57301 and 58324 gene is normalized by subtracting the Ct value of the β-2 microglobulin gene to obtain a _{Δ}Ct value using the following formula: _{Δ}Ct=Ct_{human 59914 and 59921} - Ct _{β-2 microglobulin}. Expression is then calibrated against a cDNA sample showing a comparatively low level of expression of the human 38554, 57301 and 58324 gene. The _{Δ}Ct value for the calibrator sample is then subtracted from _{Δ}Ct for each tissue sample according to the following formula: _{ΔΔ}Ct=_{Δ}Ct-ₛₐₘₚₗₑ - _{Δ}Ct-_{calibrator}. Relative expression is then calculated using the arithmetic formula given by 2^{-ΔΔCt}. Expression of the target human 38554, 57301 and 58324 gene in each of the tissues tested is then graphically represented as discussed in more detail below.

This analysis found 38554 mRNA expression at high levels in human brain cortex and hypothalamus tissue, at medium levels in dorsal root ganglion, spinal cord, choroid plexus, and testes, at small levels in normal artery, human umbilical vein endothelial cells, hemangioma tissue, tissue from heart undergoing congestive heart failure, and kidney; and at trace levels in salivary glands, normal colon, colon tumor, normal lung, normal tonsil, mammary gland and pancreas; 57301 mRNA expression at high levels in kidney; and 58324 mRNA expression at small levels in hemangioma tissue.

### Expression of 38554 in Rodent Tissue and Rodent Pain models

To study the expression of 38554 in animal models of pain, rats were subjected to the following procedures: ligation of the sciatic nerve to produce chronic constriction injury (Bennett GJ & Xie YK, 1988; Pain 33; 87-107), plantar injection of complete Freund's adjuvant (Stein C, Millan MJ and Herz A, 1988; Pharmacol Biochem Behav 31; 445-451) to produce inflammatory pain, or axotomy of the sciatic nerve (Curtis et al., 1994; Neuron 12; 191-204) to produce chronic pain. TaqMan® quantitative PCR (PE Applied Biosystems) to measure the expression of the rat ortholog of human 38554 in cDNA prepared from a variety of normal and diseased (e.g., pain models) tissues was performed by the same methods as for the human tissue, as described above, except 18S RNA was used as an internal amplicon reference and reference probe; the integrity of the RNA samples following DNase I treatment was confirmed by 1.2% agarose gel electrophoresis; probes were designed by PrimerExpress software (PE Biosystems) based on the sequence of the rat 38554 gene; 200nM of forward and reverse primers plus 100nM probe for 18S and 900 nM forward and reverse primers plus 250 nM probe; and the Ct value of the rat 38554 gene is normalized by subtracting the Ct value of the 18S to obtain a _{Δ}Ct value using the following formula: _{Δ}Ct=averageCt _{rat 38554} - averageCt _{18S}.

The results indicated high levels of rat 38554 expression in brain, spinal cord, small levels of rat 38554 expression in dorsal root ganglion, and trace levels of rat 38554 expression in superior cervical ganglion, ovary and uterus. In the analysis of tissues using pain models, up-regulation of rat 38554 expression is found in rodent dorsal root ganglion after axotomy.

### In Situ Hybridization

The human 38554 clone was used to make probes for *in situ* hybridization experiments. Expression of 38554 mRNA in monkey and rodent neurological tissues confirms the expression found by TaqMan® quantitative PCR in human neurological tissues. In the rat neurological tissues, *in situ* hybridization found 38554 expression in cortex, hippocampus, spinal cord, and cerebrum. In the monkey neurological tissues, *in situ* hybridization found 38554 expression in the cortex, choroid plexus and dorsal root ganglion. Glial cells were the specific cell type labeled in these tissues; in choroid plexus, the cells are similar to epithelial cells.

The contents of all references, patents and published patent applications cited throughout this application are incorporated herein by reference.

### Equivalents

Those skilled in the art will recognize, or be able to ascertain using no more than routine experimentation, many equivalents to the specific embodiments of the invention described herein.

## Claims

1. An isolated nucleic acid molecule selected from the group consisting of:
a) a nucleic acid molecule comprising a nucleotide sequence of SEQ ID NO:1 or SEQ ID NO:3; a nucleic acid molecule comprising a nucleotide sequence of SEQ ID NO:22 or SEQ ID NO:24; a nucleic acid molecule comprising a nucleotide sequence of SEQ ID NO:4 or SEQ ID NO:6; or a nucleic acid molecule comprising a nucleotide sequence which is at least 86% identical to the nucleotide sequence of SEQ ID NO:7 or SEQ ID NO:9;
b) a nucleic acid molecule comprising a fragment of at least 417 nucleotides of the nucleotide sequence of SEQ ID NO: 7 or SEQ ID NO:9;
c) a nucleic acid molecule which encodes a polypeptide comprising the amino acid sequence of SEQ ID NO:2; a polypeptide comprising the amino acid sequence of SEQ ID NO:23; a polypeptide comprising the amino acid sequence of SEQ ID NO:5; or a polypeptide comprising the amino acid sequence of SEQ ID NO:8;
d) a nucleic acid molecule which encodes a fragment of a polypeptide comprising the amino acid sequence of SEQ ID NO:2, wherein the fragment comprises at least 15 contiguous amino acids of SEQ ID NO: 2; a nucleic acid molecule which encodes a fragment of a polypeptide comprising the amino acid sequence of SEQ ID NO:23, wherein the fragment comprises at least 15 contiguous amino acids of SEQ ID NO: 23; a nucleic acid molecule which encodes a fragment of a polypeptide comprising the amino acid sequence of SEQ ID NO:5, wherein the fragment comprises at least 15 contiguous amino acids of SEQ ID NO: 5; a nucleic acid molecule which encodes a fragment of a polypeptide comprising the amino acid sequence of SEQ ID NO:8, wherein the fragment comprises at least 15 contiguous amino acids of SEQ ID NO: 8; and
e) a nucleic acid molecule which encodes a naturally occurring allelic variant of a polypeptide comprising the amino acid sequence of SEQ ID NO:2, SEQ ID NO:23, SEQ ID NO:5 or SEQ ID NO:8, wherein the nucleic acid molecule hybridizes to a nucleic acid molecule comprising SEQ ID NO: 1, 3, 22, 24, 4, 6, 7, 9, or a complement thereof, under stringent conditions.

2. The isolated nucleic acid molecule of claim 1, which is selected from the group consisting of:
a) a nucleic acid comprising the nucleotide sequence of SEQ ID NO:1, SEQ ID NO:3, SEQ ID NO:22, SEQ ID NO:24, SEQ ID NO:4, SEQ ID NO:6, SEQ ID NO:7, or SEQ ID NO:9; and
b) a nucleic acid molecule which encodes a polypeptide comprising the amino acid sequence of SEQ ID NO:2, SEQ ID NO:23, SEQ ID NO:5, or SEQ ID NO:8.

3. The nucleic acid molecule of claim 1 or 2, further comprising vector nucleic acid sequences.

4. The nucleic acid molecule of claim 1, 2 or 3, further comprising nucleic acid sequences encoding a heterologous polypeptide.

5. A host cell which contains the nucleic acid molecule of any of claims 1 to 4.

6. The host cell of claim 5 which is a mammalian host cell.

7. A non-human mammalian host cell containing the nucleic acid molecule of any of claims 1 to 4.

8. An isolated polypeptide selected from the group consisting of:
a) a polypeptide which is encoded by a nucleic acid molecule comprising a nucleotide sequence of SEQ ID NO: 1 or SEQ ID NO:3, or a complement thereof; a nucleotide sequence of SEQ ID NO: 22 or SEQ ID NO:24, or a complement thereof; a nucleotide sequence of SEQ ID NO: 4 or SEQ ID NO:6, or a complement thereof; or a nucleotide sequence which is at least 86% identical to a nucleic acid comprising the nucleotide sequence of SEQ ID NO: 7 or SEQ ID NO:9, or a complement thereof;
b) a naturally occurring allelic variant of a polypeptide comprising the amino acid sequence of SEQ ID NO:2, wherein the polypeptide is encoded by a nucleic acid molecule which hybridizes to a nucleic acid molecule comprising SEQ ID NO: 1, SEQ ID NO:3, or a complement thereof under stringent conditions; a polypeptide comprising the amino acid sequence of SEQ ID NO:23, wherein the polypeptide is encoded by a nucleic acid molecule which hybridizes to a nucleic acid molecule comprising SEQ ID NO: 22, SEQ ID NO:24, or a complement thereof under stringent conditions; a polypeptide comprising the amino acid sequence of SEQ ID NO:5, wherein the polypeptide is encoded by a nucleic acid molecule which hybridizes to a nucleic acid molecule comprising SEQ ID NO: 4, SEQ ID NO:6, or a complement thereof under stringent conditions; a polypeptide comprising the amino acid sequence of SEQ ID NO:8, wherein the polypeptide is encoded by a nucleic acid molecule which hybridizes to a nucleic acid molecule comprising SEQ ID NO: 7, SEQ ID NO:9, or a complement thereof under stringent conditions; and
c) a fragment of a polypeptide comprising the amino acid sequence of SEQ ID NO:2, SEQ ID NO:23, SEQ ID NO:5, or SEQ ID NO:8, wherein the fragment comprises at least 15 contiguous amino acids of SEQ ID NO:2, SEQ ID NO:23, SEQ ID NO:5, or SEQ ID NO:8.

9. The isolated polypeptide of claim 8 comprising the amino acid sequence of SEQ ID NO:2, SEQ ID NO:23, SEQ ID NO:5, or SEQ ID NO:8.

10. The polypeptide of claim 8 or 9, further comprising heterologous amino acid sequences.

11. An antibody which selectively binds to a polypeptide of claim 8, 9 or 10.

12. A method for producing a polypeptide selected from the group consisting of:
a) a polypeptide comprising the amino acid sequence of SEQ ID NO:2, SEQ ID NO:23, SEQ ID NO:5, or SEQ ID NO:8;
b) a polypeptide comprising a fragment of the amino acid sequence of SEQ ID NO:2, SEQ ID NO:23, SEQ ID NO:5, or SEQ ID NO:8, wherein the fragment comprises at least 15 contiguous amino acids of SEQ ID NO:2, SEQ ID NO:23, SEQ ID NO:5, or SEQ ID NO:8; and
c) a naturally occurring allelic variant of a polypeptide comprising the amino acid sequence of SEQ ID NO:2, SEQ ID NO:23, SEQ ID NO:5, or SEQ ID NO:8, wherein the polypeptide is encoded by a nucleic acid molecule which hybridizes to a nucleic acid molecule comprising SEQ ID NO:1, SEQ ID NO:3, SEQ ID NO:22, SEQ ID NO:24, SEQ ID NO:4, SEQ ID NO:6, SEQ ID NO:7, SEQ ID NO:9, or a complement thereof under stringent conditions;
comprising culturing the host cell of claim 5 under conditions in which the nucleic acid molecule is expressed.

13. A method for detecting the presence of a polypeptide of claim 8, 9 or 10 in a sample, comprising:
a) contacting the sample with a compound which selectively binds to a polypeptide of claim 8, 9 or 10, as the case may be; and
b) determining whether the compound binds to the polypeptide in the sample.

14. The method of claim 13, wherein the compound which binds to the polypeptide is an antibody.

15. A kit comprising a compound which selectively binds to a polypeptide of claim 8, 9 or 10 and instructions for use.

16. A method for detecting the presence of a nucleic acid molecule of any of claims 1 to 4 in a sample, comprising the steps of:
a) contacting the sample with a nucleic acid probe or primer which selectively hybridizes to the nucleic acid molecule; and
b) determining whether the nucleic acid probe or primer binds to a nucleic acid molecule in the sample.

17. The method of claim 16, wherein the sample comprises mRNA molecules and is contacted with a nucleic acid probe.

18. A kit comprising a compound which selectively hybridizes to a nucleic acid molecule of claim 1 and instructions for use.

19. A method for identifying a compound which binds to a polypeptide of claim 8, 9 or 10, comprising the steps of:
a) contacting a polypeptide, or a cell expressing a polypeptide of claim 8, 9 or 10, as the case may be, with a test compound; and
b) determining whether the polypeptide binds to the test compound.

20. The method of claim 19, wherein the binding of the test compound to the polypeptide is detected by a method selected from the group consisting of:
a) detection of binding by direct detecting of test compound/polypeptide binding;
b) detection of binding using a competition binding assay;
c) detection of binding using an assay for 38554-, 57301-, and 58324-mediated signal transduction.

21. A method for modulating the activity of a polypeptide of claim 8, 9 or 10, comprising contacting a polypeptide or a cell expressing a polypeptide of claim 8, 9 or 10, as the case may be, with a compound which binds to the polypeptide in a sufficient concentration to modulate the activity of the polypeptide.

22. A method for identifying a compound which modulates the activity of a polypeptide of claim 8, 9 or 10, comprising:
a) contacting a polypeptide of claim 8, 9 or 10, as the case may be, with a test compound; and
b) determining the effect of the test compound on the activity of the polypeptide to thereby identify a compound which modulates the activity of the polypeptide.

23. A pharmaceutical composition, comprising a compound which modulates the expression or activity of a 38554 nucleic acid molecule or polypeptide.

24. Use of a compound which modulates the expression or activity of a 38554 nucleic acid molecule or polypeptide in the preparation of a medicament for treating pain disorders in a subject.


SEQUENCE LISTING

<110> Curtis, Rory A.J.
      Silos-Santiago, Inmaculada
      Millennium Pharmaceuticals, Inc.

<120> 38554, 57301, and 58324, Human Organic
  Ion Transporters and Uses Therefor

<130> P71924EP

<150> 60/275,172
<151> 2001-03-12

<160> 24

<170> FastSEQ for Windows Version 4.0

<210> 1
<211> 3220
<212> DNA
<213> homo sapiens

<220>
<221> CDS
<222> (338)...(2476)

<400> 1
   tcgggaagcg cgccattgtg ttggtacccg ggaattcgcg gccgcgtcga cccccctgcg 60
   gagttgtgtt ttctgggaat cagcaacaaa ttgcaaagaa atggctcaaa agcttcagct 120
   ctttctgtgc cctgggagct gagatgcacg tcagtggcct tgccagcgtg gccaattctc 180
   tgctgactgc cagaaaaaag aggccaggaa gaaagaggaa agagaagaga tcgctcaggg 240
   tctgtggtgt gtggtccatc ctcttgctga gcacattgaa aggaactggc tatctttgat 300
   ctcttcctcc agatcagagt caaggaatgt gtttata atg gac act tca tcc aaa 355
                                           Met Asp Thr Ser Ser Lys
                                            1               5
   gaa aat atc cag ttg ttc tgc aaa act tca gtg caa cct gtt gga agg 403
   Glu Asn Ile Gln Leu Phe Cys Lys Thr Ser Val Gln Pro Val Gly Arg
               10                  15                  20
   cct tct ttt aaa aca gaa tat ccc tcc tca gaa gaa aag caa cca tgc 451
   Pro Ser Phe Lys Thr Glu Tyr Pro Ser Ser Glu Glu Lys Gln Pro Cys
           25                  30                  35
   tgt ggt gaa cta aag gtg ttc ttg tgt gcc ttg tct ttt gtt tac ttt 499
   Cys Gly Glu Leu Lys Val Phe Leu Cys Ala Leu Ser Phe Val Tyr Phe
       40                  45                  50
   gcc aaa gca ttg gca gaa ggc tat ctg aag agc acc atc act cag ata 547
   Ala Lys Ala Leu Ala Glu Gly Tyr Leu Lys Ser Thr Ile Thr Gln Ile
   55                  60                  65                  70
   gag aga agg ttt gat atc cct tct tca ctg gtg gga gtt att ggt ggt 595
   Glu Arg Arg Phe Asp Ile Pro Ser Ser Leu Val Gly Val Ile Gly Gly
                   75                  80                  85
   agt ttt gaa att ggg aat ctc tta gtt ata aca ttt gtt agc tac ttt 643
   Ser Phe Glu Ile Gly Asn Leu Leu Val Ile Thr Phe Val Ser Tyr Phe
   90                  95                  100
   gga gcc aaa ctt cac agg cca aaa ata att gga gca ggg tgt gta atc     691
   Gly Ala Lys Leu His Arg Pro Lys Ile Ile Gly Ala Gly Cys Val Ile
           105                 110                 115
   atg gga gtt gga aca ctg ctc att gca atg cct cag ttc ttc atg gag     739
   Met Gly Val Gly Thr Leu Leu Ile Ala Met Pro Gln Phe Phe Met Glu
       120                 125                 130
   cag tac aaa tat gag aga tat tct cct tcc tcc aat tct act ctc agc     787
   Gln Tyr Lys Tyr Glu Arg Tyr Ser Pro Ser Ser Asn Ser Thr Leu Ser
   135                 140                 145                 150
   atc tct ccg cgt ctc cta gag tca agc agt caa tta cca gtt tca gtt     835
   Ile Ser Pro Arg Leu Leu Glu Ser Ser Ser Gln Leu Pro Val Ser Val
                   155                 160                 165
   atg gaa aaa tca aaa tcc aaa ata agt aac gaa tgt gaa gtg gac act     883
   Met Glu Lys Ser Lys Ser Lys Ile Ser Asn Glu Cys Glu Val Asp Thr
               170                 175                 180
   agc tct tcc atg tgg att tat gtt ttc ctg ggt aat ctt ctt cgt gga     931
   Ser Ser Ser Met Trp Ile Tyr Val Phe Leu Gly Asn Leu Leu Arg Gly
           185                 190                 195
   ata gga gaa act ccc att cag cct ttg ggc att gcc tac ctg gat gat     979
   Ile Gly Glu Thr Pro Ile Gln Pro Leu Gly Ile Ala Tyr Leu Asp Asp
       200                 205                 210
   ttt gcc agt gaa gac aat gca gct ttc tat att ggg tgt gtg cag acg     1027
   Phe Ala Ser Glu Asp Asn Ala Ala Phe Tyr Ile Gly Cys Val Gln Thr
   215                 220                 225                 230
   gtt gca att ata gga cca atc ttt ggt ttc ctg tta ggc tca tta tgt     1075
   Val Ala Ile Ile Gly Pro Ile Phe Gly Phe Leu Leu Gly Ser Leu Cys
                   235                 240                 245
   gcc aaa cta tat gtt gac att ggc ttt gta aac cta gat cac ata acc     1123
   Ala Lys Leu Tyr Val Asp Ile Gly Phe Val Asn Leu Asp His Ile Thr
               250                 255                 260
   att aca cca aaa gat ccc cag tgg gta gga gcc tgg tgg ctt ggc tat     1171
   Ile Thr Pro Lys Asp Pro Gln Trp Val Gly Ala Trp Trp Leu Gly Tyr
           265                 270                 275
   cta ata gca gga atc ata agt ctt ctt gca gct gtg cct ttc tgg tat     1219
   Leu Ile Ala Gly Ile Ile Ser Leu Leu Ala Ala Val Pro Phe Trp Tyr
       280                 285                 290
   tta cca aag agt tta cca aga tcc caa agt aga gag gat tct aat tct     1267
   Leu Pro Lys Ser Leu Pro Arg Ser Gln Ser Arg Glu Asp Ser Asn Ser
   295                 300                 305                 310
   tcc tct gag aaa tcc aag ttt att ata gat gat cac aca gac tac caa     1315
   Ser Ser Glu Lys Ser Lys Phe Ile Ile Asp Asp His Thr Asp Tyr Gln
                   315                 320                 325
   aca ccc cag gga gaa aat gca aaa ata atg gaa atg gca aga gat ttt     1363
   Thr Pro Gln Gly Glu Asn Ala Lys Ile Met Glu Met Ala Arg Asp Phe
               330                 335                 340
   ctt cca tca ctg aag aat ctt ttt gga aac cca gta tac ttc cta tat 1411
   Leu Pro Ser Leu Lys Asn Leu Phe Gly Asn Pro Val Tyr Phe Leu Tyr
   345 350 355
   tta tgt aca agc act gtt cag ttc aat tct ctg ttc ggc atg gtg acg 1459
   Leu Cys Thr Ser Thr Val Gln Phe Asn Ser Leu Phe Gly Met Val Thr
   360 365 370
   tac aaa cca aag tac att gag cag cag tat gga cag tca tcc tcc agg 1507
   Tyr Lys Pro Lys Tyr Ile Glu Gln Gln Tyr Gly Gln Ser Ser Ser Arg
   375 380 385 390
   gcc aac ttt gtg atc ggg ctc atc aac att cca gca gtg gcc ctt gga 1555
   Ala Asn Phe Val Ile Gly Leu Ile Asn Ile Pro Ala Val Ala Leu Gly
   395 400 405
   ata ttc tct ggg ggg ata gtt atg aaa aaa ttc aga atc agt gtg tgt 1603
   Ile Phe Ser Gly Gly Ile Val Met Lys Lys Phe Arg Ile Ser Val Cys
   410 415 420
   gga gct gca aaa ctc tac ttg gga tca tct gtc ttt ggt tac ctc cta 1651
   Gly Ala Ala Lys Leu Tyr Leu Gly Ser Ser Val Phe Gly Tyr Leu Leu
   425 430 435
   ttt ctt tcc ctg ttt gca ctg ggc tgt gaa aat tct gat gtg gca gga 1699
   Phe Leu Ser Leu Phe Ala Leu Gly Cys Glu Asn Ser Asp Val Ala Gly
   440 445 450
   cta act gtc tcc tac caa gga acc aaa cct gtc tct tat cat gaa cga 1747
   Leu Thr Val Ser Tyr Gln Gly Thr Lys Pro Val Ser Tyr His Glu Arg
   455 460 465 470
   gct ctc ttt tca gat tgc aac tca aga tgc aaa tgt tca gag aca aaa 1795
   Ala Leu Phe Ser Asp Cys Asn Ser Arg Cys Lys Cys Ser Glu Thr Lys
   475 480 485
   tgg gaa ccc atg tgc ggt gaa aat gga atc aca tat gta tca gct tgt 1843
   Trp Glu Pro Met Cys Gly Glu Asn Gly Ile Thr Tyr Val Ser Ala Cys
   490 495 500
   ctt gct ggt tgt caa acc tcc aac agg agt gga aaa aat att ata ttt 1891
   Leu Ala Gly Cys Gln Thr Ser Asn Arg Ser Gly Lys Asn Ile Ile Phe
   505 510 515
   tac aac tgc act tgt gtg gga att gca gct tct aaa tcc gga aat tcc 1939
   Tyr Asn Cys Thr Cys Val Gly Ile Ala Ala Ser Lys Ser Gly Asn Ser
   520 525 530
   tca ggc ata gtg gga aga tgt cag aaa gac aat gga tgt ccc caa atg 1987
   Ser Gly Ile Val Gly Arg Cys Gln Lys Asp Asn Gly Cys Pro Gln Met
   535 540 545 550
   ttt ctg tat ttc ctt gta att tca gtc atc aca tcc tat act tta tcc 2035
   Phe Leu Tyr Phe Leu Val Ile Ser Val Ile Thr Ser Tyr Thr Leu Ser
   555 560 565
   cta ggt ggc ata cct gga tac ata tta ctt ctg agg tgc att aag cca 2083
   Leu Gly Gly Ile Pro Gly Tyr Ile Leu Leu Leu Arg Cys Ile Lys Pro
   570 575 580
   cag ctt aag tct ttt gcc ttg ggt atc tac aca tta gca ata aga gtt 2131
   Gln Leu Lys Ser Phe Ala Leu Gly Ile Tyr Thr Leu Ala Ile Arg Val
   585 590 595
   ctt gca gga atc cca gct cca gtg tat ttt gga gtt ttg att gat act 2179
   Leu Ala Gly Ile Pro Ala Pro Val Tyr Phe Gly Val Leu Ile Asp Thr
   600 605 610
   tca tgc ctc aaa tgg gga ttt aaa aga tgt gga agt aga gga tca tgc 2227
   Ser Cys Leu Lys Trp Gly Phe Lys Arg Cys Gly Ser Arg Gly Ser Cys
   615 620 625 630
   aga tta tat gat tca aat gtc ttc aga cat ata tat ctg gga cta act 2275
   Arg Leu Tyr Asp Ser Asn Val Phe Arg His Ile Tyr Leu Gly Leu Thr
   635 640 645
   gtg ata ctg ggc aca gtg tca att ctc cta agc att gca gta ctt ttc 2323
   Val Ile Leu Gly Thr Val Ser Ile Leu Leu Ser Ile Ala Val Leu Phe
   650 655 660
   att tta aag aaa aat tat gtt tca aaa cac aga agt ttt ata acc aag 2371
   Ile Leu Lys Lys Asn Tyr Val Ser Lys His Arg Ser Phe Ile Thr Lys
   665 670 675
   aga gaa aga aca atg gtg tct aca aga ttc caa aag gaa aat tac act 2419
   Arg Glu Arg Thr Met Val Ser Thr Arg Phe Gln Lys Glu Asn Tyr Thr
   680 685 690
   aca agt gat cat ctg cta caa ccc aac tac tgg cca ggc aag gaa act 2467
   Thr Ser Asp His Leu Leu Gln Pro Asn Tyr Trp Pro Gly Lys Glu Thr
   695 700 705 710
   caa ctt tag aaacatgatg actggaagtc atgtcttcta attggttgac 2516
   Gln Leu *
   attttgcaaa caaataaatt gtaatcaaaa gagctctaaa tttgtaattt ctttctcctt 2576
   tcaaaaaatg tctactttgt tttggtccta ggcattaggt aatataactg ataatatact 2636
   gaaacatata atggaagatg cagatgataa aactaatttt gaacttttta atttatataa 2696
   attattttat atcacttact tatttcactt tattttgctt tgtgctcatt gatatatatt 2756
   agctgtactc ctagaagaac aattgtctct attgtcacac atggttatat ttaaagtaat 2816
   ttctgaactg tgtaatgtgt ctagagtaag caaatactgc taacaattaa ctcatacctt 2876
   gggttccttc aagtattact cctatagtat tttctcccat agctgtcttc atctgtgtat 2936
   tttaataatg atcttaggat ggagcagaac atggagagga agatttcatt ttaagctcct 2996
   ccttttcttt gaaatacaat aatttatata gaaatgtgta gcagcaaatt atattgggga 3056
   ttagaatttt gaattaatag ctctcctact attaatttac atgtgctttt tgtgtggcgc 3116
   tataagtgac tatggttgta aagtaataaa attgatgtta acatgcccaa aaaaaaaaaa 3176
   aaaaaaaacc aaaaaaaaaa aaaaaaaagg gcgggccgct agac 3220

<210> 2
<211> 712
<212> PRT
<213> homo sapiens

<400> 2
Met Asp Thr Ser Ser Lys Glu Asn Ile Gln Leu Phe Cys Lys Thr Ser
1               5                   10                  15
Val Gln Pro Val Gly Arg Pro Ser Phe Lys Thr Glu Tyr Pro Ser Ser
            20                  25                  30
Glu Glu Lys Gln Pro Cys Cys Gly Glu Leu Lys Val Phe Leu Cys Ala
        35                  40                  45

Leu Ser Phe Val Tyr Phe Ala Lys Ala Leu Ala Glu Gly Tyr Leu Lys
    50                  55                  60
Ser Thr Ile Thr Gln Ile Glu Arg Arg Phe Asp Ile Pro Ser Ser Leu
65                  70                  75                  80
Val Gly Val Ile Gly Gly Ser Phe Glu Ile Gly Asn Leu Leu Val Ile
                85                  90                  95
Thr Phe Val Ser Tyr Phe Gly Ala Lys Leu His Arg Pro Lys Ile Ile
            100                 105                 110
Gly Ala Gly Cys Val Ile Met Gly Val Gly Thr Leu Leu Ile Ala Met
        115                 120                 125
Pro Gln Phe Phe Met Glu Gln Tyr Lys Tyr Glu Arg Tyr Ser Pro Ser
    130                 135                 140
Ser Asn Ser Thr Leu Ser Ile Ser Pro Arg Leu Leu Glu Ser Ser Ser
145                 150                 155                 160
Gln Leu Pro Val Ser Val Met Glu Lys Ser Lys Ser Lys Ile Ser Asn
                165                 170                 175
Glu Cys Glu Val Asp Thr Ser Ser Ser Met Trp Ile Tyr Val Phe Leu
            180                 185                 190
Gly Asn Leu Leu Arg Gly Ile Gly Glu Thr Pro Ile Gln Pro Leu Gly
        195                 200                 205
Ile Ala Tyr Leu Asp Asp Phe Ala Ser Glu Asp Asn Ala Ala Phe Tyr
    210                 215                 220
Ile Gly Cys Val Gln Thr Val Ala Ile Ile Gly Pro Ile Phe Gly Phe
225                 230                 235                 240
Leu Leu Gly Ser Leu Cys Ala Lys Leu Tyr Val Asp Ile Gly Phe Val
                245                 250                 255
Asn Leu Asp His Ile Thr Ile Thr Pro Lys Asp Pro Gln Trp Val Gly
            260                 265                 270
Ala Trp Trp Leu Gly Tyr Leu Ile Ala Gly Ile Ile Ser Leu Leu Ala
        275                 280                 285
Ala Val Pro Phe Trp Tyr Leu Pro Lys Ser Leu Pro Arg Ser Gln Ser
    290                 295                 300
Arg Glu Asp Ser Asn Ser Ser Ser Glu Lys Ser Lys Phe Ile Ile Asp
305                 310                 315                 320
Asp His Thr Asp Tyr Gln Thr Pro Gln Gly Glu Asn Ala Lys Ile Met
                325                 330                 335
Glu Met Ala Arg Asp Phe Leu Pro Ser Leu Lys Asn Leu Phe Gly Asn
            340                 345                 350
Pro Val Tyr Phe Leu Tyr Leu Cys Thr Ser Thr Val Gln Phe Asn Ser
        355                 360                 365
Leu Phe Gly Met Val Thr Tyr Lys Pro Lys Tyr Ile Glu Gln Gln Tyr
    370                 375                 380
Gly Gln Ser Ser Ser Arg Ala Asn Phe Val Ile Gly Leu Ile Asn Ile
385                 390                 395                 400
Pro Ala Val Ala Leu Gly Ile Phe Ser Gly Gly Ile Val Met Lys Lys
                405                 410                 415
Phe Arg Ile Ser Val Cys Gly Ala Ala Lys Leu Tyr Leu Gly Ser Ser
            420                 425                 430
Val Phe Gly Tyr Leu Leu Phe Leu Ser Leu Phe Ala Leu Gly Cys Glu
        435                 440                 445
Asn Ser Asp Val Ala Gly Leu Thr Val Ser Tyr Gln Gly Thr Lys Pro
    450                 455                 460
Val Ser Tyr His Glu Arg Ala Leu Phe Ser Asp Cys Asn Ser Arg Cys
465                 470                 475                 480
Lys Cys Ser Glu Thr Lys Trp Glu Pro Met Cys Gly Glu Asn Gly Ile
                485                 490                 495
Thr Tyr Val Ser Ala Cys Leu Ala Gly Cys Gln Thr Ser Asn Arg Ser
            500                 505                 510
Gly Lys Asn Ile Ile Phe Tyr Asn Cys Thr Cys Val Gly Ile Ala Ala
        515                 520                 525
Ser Lys Ser Gly Asn Ser Ser Gly Ile Val Gly Arg Cys Gln Lys Asp

530                 535                 540
Asn Gly Cys Pro Gln Met Phe Leu Tyr Phe Leu Val Ile Ser Val Ile
545                 550                 555                 560
Thr Ser Tyr Thr Leu Ser Leu Gly Gly Ile Pro Gly Tyr Ile Leu Leu
                565                 570                 575
Leu Arg Cys Ile Lys Pro Gln Leu Lys Ser Phe Ala Leu Gly Ile Tyr
            580                 585                 590
Thr Leu Ala Ile Arg Val Leu Ala Gly Ile Pro Ala Pro Val Tyr Phe
        595                 600                 605
Gly Val Leu Ile Asp Thr Ser Cys Leu Lys Trp Gly Phe Lys Arg Cys
    610                 615                 620
Gly Ser Arg Gly Ser Cys Arg Leu Tyr Asp Ser Asn Val Phe Arg His
625                 630                 635                 640
Ile Tyr Leu Gly Leu Thr Val Ile Leu Gly Thr Val Ser Ile Leu Leu
                645                 650                 655
Ser Ile Ala Val Leu Phe Ile Leu Lys Lys Asn Tyr Val Ser Lys His
            660                 665                 670
Arg Ser Phe Ile Thr Lys Arg Glu Arg Thr Met Val Ser Thr Arg Phe
        675                 680                 685
Gln Lys Glu Asn Tyr Thr Thr Ser Asp His Leu Leu Gln Pro Asn Tyr
    690                 695                 700
Trp Pro Gly Lys Glu Thr Gln Leu
705                 710

<210> 3
<211> 2139
<212> DNA
<213> homo sapiens

<220>
<221> CDS
<222> (1)...(2139)

<400> 3
atg gac act tca tcc aaa gaa aat atc cag ttg ttc tgc aaa act tca     48
Met Asp Thr Ser Ser Lys Glu Asn Ile Gln Leu Phe Cys Lys Thr Ser
 1               5                  10                  15

gtg caa cct gtt gga agg cct tct ttt aaa aca gaa tat ccc tcc tca     96
Val Gln Pro Val Gly Arg Pro Ser Phe Lys Thr Glu Tyr Pro Ser Ser
            20                  25                  30

gaa gaa aag caa cca tgc tgt ggt gaa cta aag gtg ttc ttg tgt gcc     144
Glu Glu Lys Gln Pro Cys Cys Gly Glu Leu Lys Val Phe Leu Cys Ala
        35                  40                  45

ttg tct ttt gtt tac ttt gcc aaa gca ttg gca gaa ggc tat ctg aag     192
Leu Ser Phe Val Tyr Phe Ala Lys Ala Leu Ala Glu Gly Tyr Leu Lys
    50                  55                  60

agc acc atc act cag ata gag aga agg ttt gat atc cct tct tca ctg     240
Ser Thr Ile Thr Gln Ile Glu Arg Arg Phe Asp Ile Pro Ser Ser Leu
65                  70                  75                  80

gtg gga gtt att ggt ggt agt ttt gaa att ggg aat ctc tta gtt ata     288
Val Gly Val Ile Gly Gly Ser Phe Glu Ile Gly Asn Leu Leu Val Ile
                85                  90                  95

aca ttt gtt agc tac ttt gga gcc aaa ctt cac agg cca aaa ata att     336
Thr Phe Val Ser Tyr Phe Gly Ala Lys Leu His Arg Pro Lys Ile Ile

100                 105                 110

gga gca ggg tgt gta atc atg gga gtt gga aca ctg ctc att gca atg     384
Gly Ala Gly Cys Val Ile Met Gly Val Gly Thr Leu Leu Ile Ala Met
        115                 120                 125

cct cag ttc ttc atg gag cag tac aaa tat gag aga tat tct cct tcc     432
Pro Gln Phe Phe Met Glu Gln Tyr Lys Tyr Glu Arg Tyr Ser Pro Ser
    130                 135                 140

tcc aat tct act ctc agc atc tct ccg cgt ctc cta gag tca agc agt     480
Ser Asn Ser Thr Leu Ser Ile Ser Pro Arg Leu Leu Glu Ser Ser Ser
145                 150                 155                 160

caa tta cca gtt tca gtt atg gaa aaa tca aaa tcc aaa ata agt aac     528
Gln Leu Pro Val Ser Val Met Glu Lys Ser Lys Ser Lys Ile Ser Asn
                165                 170                 175

gaa tgt gaa gtg gac act agc tct tcc atg tgg att tat gtt ttc ctg     576
Glu Cys Glu Val Asp Thr Ser Ser Ser Met Trp Ile Tyr Val Phe Leu
            180                 185                 190

ggt aat ctt ctt cgt gga ata gga gaa act ccc att cag cct ttg ggc     624
Gly Asn Leu Leu Arg Gly Ile Gly Glu Thr Pro Ile Gln Pro Leu Gly
        195                 200                 205

att gcc tac ctg gat gat ttt gcc agt gaa gac aat gca gct ttc tat     672
Ile Ala Tyr Leu Asp Asp Phe Ala Ser Glu Asp Asn Ala Ala Phe Tyr
    210                 215                 220

att ggg tgt gtg cag acg gtt gca att ata gga cca atc ttt ggt ttc     720
Ile Gly Cys Val Gln Thr Val Ala Ile Ile Gly Pro Ile Phe Gly Phe
225                 230                 235                 240

ctg tta ggc tca tta tgt gcc aaa cta tat gtt gac att ggc ttt gta     768
Leu Leu Gly Ser Leu Cys Ala Lys Leu Tyr Val Asp Ile Gly Phe Val
                245                 250                 255

aac cta gat cac ata acc att aca cca aaa gat ccc cag tgg gta gga     816
Asn Leu Asp His Ile Thr Ile Thr Pro Lys Asp Pro Gln Trp Val Gly
            260                 265                 270

gcc tgg tgg ctt ggc tat cta ata gca gga atc ata agt ctt ctt gca     864
Ala Trp Trp Leu Gly Tyr Leu Ile Ala Gly Ile Ile Ser Leu Leu Ala
        275                 280                 285

gct gtg cct ttc tgg tat tta cca aag agt tta cca aga tcc caa agt     912
Ala Val Pro Phe Trp Tyr Leu Pro Lys Ser Leu Pro Arg Ser Gln Ser
    290                 295                 300

aga gag gat tct aat tct tcc tct gag aaa tcc aag ttt att ata gat     960
Arg Glu Asp Ser Asn Ser Ser Ser Glu Lys Ser Lys Phe Ile Ile Asp
305                 310                 315                 320

gat cac aca gac tac caa aca ccc cag gga gaa aat gca aaa ata atg    1008
Asp His Thr Asp Tyr Gln Thr Pro Gln Gly Glu Asn Ala Lys Ile Met
                325                 330                 335

gaa atg gca aga gat ttt ctt cca tca ctg aag aat ctt ttt gga aac    1056
Glu Met Ala Arg Asp Phe Leu Pro Ser Leu Lys Asn Leu Phe Gly Asn
            340                 345                 350

cca gta tac ttc cta tat tta tgt aca agc act gtt cag ttc aat tct     1104
Pro Val Tyr Phe Leu Tyr Leu Cys Thr Ser Thr Val Gln Phe Asn Ser
        355                 360                 365

ctg ttc ggc atg gtg acg tac aaa cca aag tac att gag cag cag tat     1152
Leu Phe Gly Met Val Thr Tyr Lys Pro Lys Tyr Ile Glu Gln Gln Tyr
    370                 375                 380

gga cag tca tcc tcc agg gcc aac ttt gtg atc ggg ctc atc aac att     1200
Gly Gln Ser Ser Ser Arg Ala Asn Phe Val Ile Gly Leu Ile Asn Ile
385                 390                 395                 400

cca gca gtg gcc ctt gga ata ttc tct ggg ggg ata gtt atg aaa aaa     1248
Pro Ala Val Ala Leu Gly Ile Phe Ser Gly Gly Ile Val Met Lys Lys
                405                 410                 415

ttc aga atc agt gtg tgt gga gct gca aaa ctc tac ttg gga tca tct     1296
Phe Arg Ile Ser Val Cys Gly Ala Ala Lys Leu Tyr Leu Gly Ser Ser
            420                 425                 430

gtc ttt ggt tac ctc cta ttt ctt tcc ctg ttt gca ctg ggc tgt gaa     1344
Val Phe Gly Tyr Leu Leu Phe Leu Ser Leu Phe Ala Leu Gly Cys Glu
        435                 440                 445

aat tct gat gtg gca gga cta act gtc tcc tac caa gga acc aaa cct     1392
Asn Ser Asp Val Ala Gly Leu Thr Val Ser Tyr Gln Gly Thr Lys Pro
    450                 455                 460

gtc tct tat cat gaa cga gct ctc ttt tca gat tgc aac tca aga tgc     1440
Val Ser Tyr His Glu Arg Ala Leu Phe Ser Asp Cys Asn Ser Arg Cys
465                 470                 475                 480

aaa tgt tca gag aca aaa tgg gaa ccc atg tgc ggt gaa aat gga atc     1488
Lys Cys Ser Glu Thr Lys Trp Glu Pro Met Cys Gly Glu Asn Gly Ile
                485                 490                 495

aca tat gta tca gct tgt ctt gct ggt tgt caa acc tcc aac agg agt     1536
Thr Tyr Val Ser Ala Cys Leu Ala Gly Cys Gln Thr Ser Asn Arg Ser
            500                 505                 510

gga aaa aat att ata ttt tac aac tgc act tgt gtg gga att gca gct     1584
Gly Lys Asn Ile Ile Phe Tyr Asn Cys Thr Cys Val Gly Ile Ala Ala
        515                 520                 525

tct aaa tcc gga aat tcc tca ggc ata gtg gga aga tgt cag aaa gac     1632
Ser Lys Ser Gly Asn Ser Ser Gly Ile Val Gly Arg Cys Gln Lys Asp
    530                 535                 540

aat gga tgt ccc caa atg ttt ctg tat ttc ctt gta att tca gtc atc     1680
Asn Gly Cys Pro Gln Met Phe Leu Tyr Phe Leu Val Ile Ser Val Ile
545                 550                 555                 560

aca tcc tat act tta tcc cta ggt ggc ata cct gga tac ata tta ctt     1728
Thr Ser Tyr Thr Leu Ser Leu Gly Gly Ile Pro Gly Tyr Ile Leu Leu
                565                 570                 575

ctg agg tgc att aag cca cag ctt aag tct ttt gcc ttg ggt atc tac     1776
Leu Arg Cys Ile Lys Pro Gln Leu Lys Ser Phe Ala Leu Gly Ile Tyr
            580                 585                 590

aca tta gca ata aga gtt ctt gca gga atc cca gct cca gtg tat ttt    1824
Thr Leu Ala Ile Arg Val Leu Ala Gly Ile Pro Ala Pro Val Tyr Phe
        595                 600                 605

gga gtt ttg att gat act tca tgc ctc aaa tgg gga ttt aaa aga tgt    1872
Gly Val Leu Ile Asp Thr Ser Cys Leu Lys Trp Gly Phe Lys Arg Cys
    610                 615                 620

gga agt aga gga tca tgc aga tta tat gat tca aat gtc ttc aga cat    1920
Gly Ser Arg Gly Ser Cys Arg Leu Tyr Asp Ser Asn Val Phe Arg His
625                 630                 635                 640

ata tat ctg gga cta act gtg ata ctg ggc aca gtg tca att ctc cta    1968
Ile Tyr Leu Gly Leu Thr Val Ile Leu Gly Thr Val Ser Ile Leu Leu
                645                 650                 655

agc att gca gta ctt ttc att tta aag aaa aat tat gtt tca aaa cac    2016
Ser Ile Ala Val Leu Phe Ile Leu Lys Lys Asn Tyr Val Ser Lys His
            660                 665                 670

aga agt ttt ata acc aag aga gaa aga aca atg gtg tct aca aga ttc    2064
Arg Ser Phe Ile Thr Lys Arg Glu Arg Thr Met Val Ser Thr Arg Phe
        675                 680                 685

caa aag gaa aat tac act aca agt gat cat ctg cta caa ccc aac tac    2112
Gln Lys Glu Asn Tyr Thr Thr Ser Asp His Leu Leu Gln Pro Asn Tyr
    690                 695                 700

tgg cca ggc aag gaa act caa ctt tag                                2139
Trp Pro Gly Lys Glu Thr Gln Leu  *
705                 710


<210> 4
<211> 2866
<212> DNA
<213> homo sapiens

<220>
<221> CDS
<222> (365)...(2026)

<223> "n" represents ambiguous nucleotides

<221> misc_feature
<222> (1)...(2866)
<223> n = A,T,C or G

<400> 4
aagtgtcacc ggtcacactt taattccagt ctaaaattaa agtcttcagt ctccacattc 60
cctactttcc aaattcagct ttcccgggag gtctggagca gctgcctctc tggggagatg 120
ctggaggtct cggaatcacc tcacacggcc tcagggccca gttggagcca ccccaagtga 180
caccagcagg cagatgacca gagagcctga gcctccggcc ccgagtctgt gaagcctagc 240
cgctgggctg gagaagccac tgtgggcacc accgtggggg aaacaggccc gttgccctgg 300
cctctttgcc ctgggccagc ctttgtgaag tgggcccctc ttctgggccc cttgagtagg 360
ttcc atg gca ttt tct gaa ctc ctg gac ctc gtg ggt ggc ctg ggc agg    409
     Met Ala Phe Ser Glu Leu Leu Asp Leu Val Gly Gly Leu Gly Arg
      1               5                  10                  15

ttc cag gtt ctc cag acg atg gct ctg atg gtc tcc atc atg tgg ctg    457
Phe Gln Val Leu Gln Thr Met Ala Leu Met Val Ser Ile Met Trp Leu

20                  25                  30

tgt acc cag agc atg ctg gag aac ttc tcg gcc gcc gtg ccc agc cac     505
Cys Thr Gln Ser Met Leu Glu Asn Phe Ser Ala Ala Val Pro Ser His
            35                  40                  45

cgc tgc tgg gca ccc ctc ctg gac aac agc acg gct cag gcc agc atc     553
Arg Cys Trp Ala Pro Leu Leu Asp Asn Ser Thr Ala Gln Ala Ser Ile
        50                  55                  60

cta ggg agc ttg agt cct gag gcc ctc ctg gct att tcc atc ccg ccg     601
Leu Gly Ser Leu Ser Pro Glu Ala Leu Leu Ala Ile Ser Ile Pro Pro
    65                  70                  75

ggc ccc aac cag agg ccc cac cag tgc cgc cgc ttc cgc cag cca cag     649
Gly Pro Asn Gln Arg Pro His Gln Cys Arg Arg Phe Arg Gln Pro Gln
80                  85                  90                  95

tgg cag ctc ttg gac ccc aat gcc acg gcc acc agc tgg agc gag gcc     697
Trp Gln Leu Leu Asp Pro Asn Ala Thr Ala Thr Ser Trp Ser Glu Ala
                100                 105                 110

gac acg gag ccg tgt gtg gat ggc tgg gtc tat gac cgc agc atc ttc     745
Asp Thr Glu Pro Cys Val Asp Gly Trp Val Tyr Asp Arg Ser Ile Phe
            115                 120                 125

acc tcc aca atc gtg gcc aag tgg aac ctc gtg tgt gac tct cat gct     793
Thr Ser Thr Ile Val Ala Lys Trp Asn Leu Val Cys Asp Ser His Ala
        130                 135                 140

ctg aag ccc atg gcc cag tcc atc tac ctg gct ggg att ctg gtg gga     841
Leu Lys Pro Met Ala Gln Ser Ile Tyr Leu Ala Gly Ile Leu Val Gly
    145                 150                 155

gct gct gcg tgc ggc cct gcc tca gac agg ttt ggg cgc agg ctg gtg     889
Ala Ala Ala Cys Gly Pro Ala Ser Asp Arg Phe Gly Arg Arg Leu Val
160                 165                 170                 175

cta acc tgg agc tac ctt cag atg gct gtg atg ggt acg gca gct gcc     937
Leu Thr Trp Ser Tyr Leu Gln Met Ala Val Met Gly Thr Ala Ala Ala
                180                 185                 190

ttc gcc cct gcc ttc ccc gtg tac tgn ntg ntc cgc ttc ctg ttg gcc     985
Phe Ala Pro Ala Phe Pro Val Tyr Xaa Xaa Xaa Arg Phe Leu Leu Ala
            195                 200                 205

ttc cac gcg tcc ggg gct gaa cca ctc ggt ctc ctt gca gtg atg gag     1033
Phe His Ala Ser Gly Ala Glu Pro Leu Gly Leu Leu Ala Val Met Glu
        210                 215                 220

tgg acg gcg gca cgg gcc cga ccc ttg gtg atg acc ttg aac tct ctg     1081
Trp Thr Ala Ala Arg Ala Arg Pro Leu Val Met Thr Leu Asn Ser Leu
    225                 230                 235

ggc ttc agc ttc ggc cat ggc ctg aca gct gca gtg gcc tac ggt gtg     1129
Gly Phe Ser Phe Gly His Gly Leu Thr Ala Ala Val Ala Tyr Gly Val
240                 245                 250                 255

cgg gac tgg aca ctg ctg cag ctg gtg gtc tcg gtc ccc ttc ttc ctc     1177
Arg Asp Trp Thr Leu Leu Gln Leu Val Val Ser Val Pro Phe Phe Leu
                260                 265                 270

tgc ttt ttg tac tcc tgg tgg ctg gca gag tcg gca cga tgg ctc ctc    1225
Cys Phe Leu Tyr Ser Trp Trp Leu Ala Glu Ser Ala Arg Trp Leu Leu
            275                 280                 285

acc aca ggc agg ctg gat tgg ggc ctg cag gag ctg tgg agg gtg gct    1273
Thr Thr Gly Arg Leu Asp Trp Gly Leu Gln Glu Leu Trp Arg Val Ala
        290                 295                 300

ccc atc aac gga aag ggg gca gtg cag gac acc ctg acc cct gag gtc    1321
Pro Ile Asn Gly Lys Gly Ala Val Gln Asp Thr Leu Thr Pro Glu Val
    305                 310                 315

ttg ctt tca gcc atg cgg gag gag ctg agc atg ggc cag cct cct gcc    1369
Leu Leu Ser Ala Met Arg Glu Glu Leu Ser Met Gly Gln Pro Pro Ala
320                 325                 330                 335

agc ctg ggc acc ctg ctc cgc atg ccc gga ctg cgc ttc cgg acc tgt    1417
Ser Leu Gly Thr Leu Leu Arg Met Pro Gly Leu Arg Phe Arg Thr Cys
                340                 345                 350

atc tcc acg ttg tgc tgg ttc gcc ttt ggc ttc acc ttc ttc ggc ctg    1465
Ile Ser Thr Leu Cys Trp Phe Ala Phe Gly Phe Thr Phe Phe Gly Leu
            355                 360                 365

gcc ctg gac ctg cag gcc ctg ggc agc aac atc ttc ctg ctc caa atg    1513
Ala Leu Asp Leu Gln Ala Leu Gly Ser Asn Ile Phe Leu Leu Gln Met
        370                 375                 380

ttc att ggt gtc gtg gac atc cca gcc aag atg ggc gcc ctg ctg ctg    1561
Phe Ile Gly Val Val Asp Ile Pro Ala Lys Met Gly Ala Leu Leu Leu
    385                 390                 395

ctg agc cac ctg ggc cgc cgc ccc acg ctg gcc gca tcc ctg ttg ctg    1609
Leu Ser His Leu Gly Arg Arg Pro Thr Leu Ala Ala Ser Leu Leu Leu
400                 405                 410                 415

gcg ggg ctc tgc att ctg gcc aac acg ctg gtg ccc cac gaa atg ggg    1657
Ala Gly Leu Cys Ile Leu Ala Asn Thr Leu Val Pro His Glu Met Gly
                420                 425                 430

gct ctg cgc tca gcc ttg gcc gtg ctg ggg ctg ggc ggg gtg ggg gct    1705
Ala Leu Arg Ser Ala Leu Ala Val Leu Gly Leu Gly Gly Val Gly Ala
            435                 440                 445

gcc ttc acc tgc atc acc atc tac agc agc gag ctc ttc ccc act gtg    1753
Ala Phe Thr Cys Ile Thr Ile Tyr Ser Ser Glu Leu Phe Pro Thr Val
        450                 455                 460

ctc agg atg acg gca gtg ggc ttg ggc cag atg gca gcc cgt gga gga    1801
Leu Arg Met Thr Ala Val Gly Leu Gly Gln Met Ala Ala Arg Gly Gly
    465                 470                 475

gcc atc ctg ggg cct ctg gtc cgg ctg ctg ggt gtc cat ggc ccc tgg    1849
Ala Ile Leu Gly Pro Leu Val Arg Leu Leu Gly Val His Gly Pro Trp
480                 485                 490                 495

ctg ccc ttg ctg gtg tat ggg acg gtg cca gtg ctg agt ggc ctg gcc    1897
Leu Pro Leu Leu Val Tyr Gly Thr Val Pro Val Leu Ser Gly Leu Ala
                500                 505                 510

gca ctg ctt ctg ccc gag acc cag agc ttg ccg ctg ccc gac acc atc 1945
Ala Leu Leu Leu Pro Glu Thr Gln Ser Leu Pro Leu Pro Asp Thr Ile
            515                 520                 525

caa gat gtg cag aac cag gca gta aag aag gca aca cat ggc acg ctg 1993
Gln Asp Val Gln Asn Gln Ala Val Lys Lys Ala Thr His Gly Thr Leu
        530                 535                 540

ggg aac tct gtc cta aaa tcc aca cag ttt tag cctcctgggg aacctgcgat 2046
Gly Asn Ser Val Leu Lys Ser Thr Gln Phe *
    545                 550

gggacggtca gaggaagaga cttcttctgt tctctggaga aggcaggagg aaagcaaaga 2106
cctccatttc cagaggccca gaggctgccc tctgaggtcc ccactctccc ccagggctgc 2166
ccctccaggt gagccctgcc cctctcacag tccaaggggc ccccttcaat actgaagggg 2226
aaaaggacag tttgattggc aggaggtgac ccagtgcacc atcaccctgc cctgccctcg 2286
tggcttcgga gagcagaggg gtcaggccca ggggaacgag ctggccttgc caaccctctg 2346
cttgactccg cactgccact tgtcccccca cacccgtcca cctgcccaga gctcagagct 2406
aaccaccatc catggtcaag acctctccta gctccacaca agcagtagag tctcagctcc 2466
acagctttac ccagaagccc tgtaagcctg gcccctggcc cctccccatg tccctccagg 2526
cctcagccac ctgcccacca catcctctgc ctgctgtccc cttcccaccc tcatccctga 2586
ccgactccac ttaaccccca aacccagccc cccttccagg ggtccagggc cagcctgaga 2646
tgcccgtgaa actcctaccc acagttacag ccacaagcct gcctcctccc accctgccag 2706
cctatgagtt cccagagggt tggggcagtc ccatgacccc atgtcccagc tccccacaca 2766
gcgctgggcc agagaggcat tggtgcgagg gattgaataa agaaacaaat gaaaaaaaaa 2826
aaaaaaaaaa agcattgcgg ccgcaagctt tttcccttta 2866

<210> 5
<211> 553
<212> PRT
<213> homo sapiens

<220>
<223> unknown amino acids at 200-202

<221> VARIANT
<222> (1)...(553)
<223> Xaa = Any Amino Acid

<400> 5
Met Ala Phe Ser Glu Leu Leu Asp Leu Val Gly Gly Leu Gly Arg Phe
1               5                   10                  15
Gln Val Leu Gln Thr Met Ala Leu Met Val Ser Ile Met Trp Leu Cys
            20                  25                  30
Thr Gln Ser Met Leu Glu Asn Phe Ser Ala Ala Val Pro Ser His Arg
        35                  40                  45
Cys Trp Ala Pro Leu Leu Asp Asn Ser Thr Ala Gln Ala Ser Ile Leu
    50                  55                  60
Gly Ser Leu Ser Pro Glu Ala Leu Leu Ala Ile Ser Ile Pro Pro Gly
65                  70                  75                  80
Pro Asn Gln Arg Pro His Gln Cys Arg Arg Phe Arg Gln Pro Gln Trp
                85                  90                  95
Gln Leu Leu Asp Pro Asn Ala Thr Ala Thr Ser Trp Ser Glu Ala Asp
            100                 105                 110
Thr Glu Pro Cys Val Asp Gly Trp Val Tyr Asp Arg Ser Ile Phe Thr
        115                 120                 125
Ser Thr Ile Val Ala Lys Trp Asn Leu Val Cys Asp Ser His Ala Leu
    130                 135                 140
Lys Pro Met Ala Gln Ser Ile Tyr Leu Ala Gly Ile Leu Val Gly Ala
145                 150                 155                 160
Ala Ala Cys Gly Pro Ala Ser Asp Arg Phe Gly Arg Arg Leu Val Leu

                165                 170                 175
Thr Trp Ser Tyr Leu Gln Met Ala Val Met Gly Thr Ala Ala Ala Phe
            180                 185                 190
Ala Pro Ala Phe Pro Val Tyr Xaa Xaa Xaa Arg Phe Leu Leu Ala Phe
        195                 200                 205
His Ala Ser Gly Ala Glu Pro Leu Gly Leu Leu Ala Val Met Glu Trp
    210                 215                 220
Thr Ala Ala Arg Ala Arg Pro Leu Val Met Thr Leu Asn Ser Leu Gly
225                 230                 235                 240
Phe Ser Phe Gly His Gly Leu Thr Ala Ala Val Ala Tyr Gly Val Arg
                245                 250                 255
Asp Trp Thr Leu Leu Gln Leu Val Val Ser Val Pro Phe Phe Leu Cys
            260                 265                 270
Phe Leu Tyr Ser Trp Trp Leu Ala Glu Ser Ala Arg Trp Leu Leu Thr
        275                 280                 285
Thr Gly Arg Leu Asp Trp Gly Leu Gln Glu Leu Trp Arg Val Ala Pro
    290                 295                 300
Ile Asn Gly Lys Gly Ala Val Gln Asp Thr Leu Thr Pro Glu Val Leu
305                 310                 315                 320
Leu Ser Ala Met Arg Glu Glu Leu Ser Met Gly Gln Pro Pro Ala Ser
                325                 330                 335
Leu Gly Thr Leu Leu Arg Met Pro Gly Leu Arg Phe Arg Thr Cys Ile
            340                 345                 350
Ser Thr Leu Cys Trp Phe Ala Phe Gly Phe Thr Phe Phe Gly Leu Ala
        355                 360                 365
Leu Asp Leu Gln Ala Leu Gly Ser Asn Ile Phe Leu Leu Gln Met Phe
    370                 375                 380
Ile Gly Val Val Asp Ile Pro Ala Lys Met Gly Ala Leu Leu Leu Leu
385                 390                 395                 400
Ser His Leu Gly Arg Arg Pro Thr Leu Ala Ala Ser Leu Leu Leu Ala
                405                 410                 415
Gly Leu Cys Ile Leu Ala Asn Thr Leu Val Pro His Glu Met Gly Ala
            420                 425                 430
Leu Arg Ser Ala Leu Ala Val Leu Gly Leu Gly Gly Val Gly Ala Ala
        435                 440                 445
Phe Thr Cys Ile Thr Ile Tyr Ser Ser Glu Leu Phe Pro Thr Val Leu
    450                 455                 460
Arg Met Thr Ala Val Gly Leu Gly Gln Met Ala Ala Arg Gly Gly Ala
465                 470                 475                 480
Ile Leu Gly Pro Leu Val Arg Leu Leu Gly Val His Gly Pro Trp Leu
                485                 490                 495
Pro Leu Leu Val Tyr Gly Thr Val Pro Val Leu Ser Gly Leu Ala Ala
            500                 505                 510
Leu Leu Leu Pro Glu Thr Gln Ser Leu Pro Leu Pro Asp Thr Ile Gln
        515                 520                 525
Asp Val Gln Asn Gln Ala Val Lys Lys Ala Thr His Gly Thr Leu Gly
    530                 535                 540
Asn Ser Val Leu Lys Ser Thr Gln Phe
545                 550

<210> 6
<211> 1662
<212> DNA
<213> homo sapiens

<220>
<221> CDS
<222> (1)...(1662)

<223> n represents ambiguous nucleotides

<221> misc_feature
<222> (1)...(1662)
<223> n = A,T,C or G

<400> 6
atg gca ttt tct gaa ctc ctg gac ctc gtg ggt ggc ctg ggc agg ttc 48
Met Ala Phe Ser Glu Leu Leu Asp Leu Val Gly Gly Leu Gly Arg Phe
1               5                   10                  15

cag gtt ctc cag acg atg gct ctg atg gtc tcc atc atg tgg ctg tgt 96
Gln Val Leu Gln Thr Met Ala Leu Met Val Ser Ile Met Trp Leu Cys
            20                  25                  30

acc cag agc atg ctg gag aac ttc tcg gcc gcc gtg ccc agc cac cgc 144
Thr Gln Ser Met Leu Glu Asn Phe Ser Ala Ala Val Pro Ser His Arg
        35                  40                  45

tgc tgg gca ccc ctc ctg gac aac agc acg gct cag gcc agc atc cta 192
Cys Trp Ala Pro Leu Leu Asp Asn Ser Thr Ala Gln Ala Ser Ile Leu
    50                  55                  60

ggg agc ttg agt cct gag gcc ctc ctg gct att tcc atc ccg ccg ggc 240
Gly Ser Leu Ser Pro Glu Ala Leu Leu Ala Ile Ser Ile Pro Pro Gly
65                  70                  75                  80

ccc aac cag agg ccc cac cag tgc cgc cgc ttc cgc cag cca cag tgg 288
Pro Asn Gln Arg Pro His Gln Cys Arg Arg Phe Arg Gln Pro Gln Trp
                85                  90                  95

cag ctc ttg gac ccc aat gcc acg gcc acc agc tgg agc gag gcc gac 336
Gln Leu Leu Asp Pro Asn Ala Thr Ala Thr Ser Trp Ser Glu Ala Asp
            100                 105                 110

acg gag ccg tgt gtg gat ggc tgg gtc tat gac cgc agc atc ttc acc 384
Thr Glu Pro Cys Val Asp Gly Trp Val Tyr Asp Arg Ser Ile Phe Thr
        115                 120                 125

tcc aca atc gtg gcc aag tgg aac ctc gtg tgt gac tct cat gct ctg 432
Ser Thr Ile Val Ala Lys Trp Asn Leu Val Cys Asp Ser His Ala Leu
    130                 135                 140

aag ccc atg gcc cag tcc atc tac ctg gct ggg att ctg gtg gga gct 480
Lys Pro Met Ala Gln Ser Ile Tyr Leu Ala Gly Ile Leu Val Gly Ala
145                 150                 155                 160

gct gcg tgc ggc cct gcc tca gac agg ttt ggg cgc agg ctg gtg cta 528
Ala Ala Cys Gly Pro Ala Ser Asp Arg Phe Gly Arg Arg Leu Val Leu
                165                 170                 175

acc tgg agc tac ctt cag atg gct gtg atg ggt acg gca gct gcc ttc 576
Thr Trp Ser Tyr Leu Gln Met Ala Val Met Gly Thr Ala Ala Ala Phe
            180                 185                 190

gcc cct gcc ttc ccc gtg tac tgn ntg ntc cgc ttc ctg ttg gcc ttc 624
Ala Pro Ala Phe Pro Val Tyr Xaa Xaa Xaa Arg Phe Leu Leu Ala Phe
        195                 200                 205

cac gcg tcc ggg gct gaa cca ctc ggt ctc ctt gca gtg atg gag tgg 672
His Ala Ser Gly Ala Glu Pro Leu Gly Leu Leu Ala Val Met Glu Trp
    210                 215                 220

acg gcg gca cgg gcc cga ccc ttg gtg atg acc ttg aac tct ctg ggc 720
Thr Ala Ala Arg Ala Arg Pro Leu Val Met Thr Leu Asn Ser Leu Gly
225                 230                 235                 240

ttc agc ttc ggc cat ggc ctg aca gct gca gtg gcc tac ggt gtg cgg 768
Phe Ser Phe Gly His Gly Leu Thr Ala Ala Val Ala Tyr Gly Val Arg
                245                 250                 255

gac tgg aca ctg ctg cag ctg gtg gtc tcg gtc ccc ttc ttc ctc tgc 816
Asp Trp Thr Leu Leu Gln Leu Val Val Ser Val Pro Phe Phe Leu Cys
            260                 265                 270

ttt ttg tac tcc tgg tgg ctg gca gag tcg gca cga tgg ctc ctc acc 864
Phe Leu Tyr Ser Trp Trp Leu Ala Glu Ser Ala Arg Trp Leu Leu Thr
        275                 280                 285

aca ggc agg ctg gat tgg ggc ctg cag gag ctg tgg agg gtg gct ccc 912
Thr Gly Arg Leu Asp Trp Gly Leu Gln Glu Leu Trp Arg Val Ala Pro
    290                 295                 300

atc aac gga aag ggg gca gtg cag gac acc ctg acc cct gag gtc ttg 960
Ile Asn Gly Lys Gly Ala Val Gln Asp Thr Leu Thr Pro Glu Val Leu
305                 310                 315                 320

ctt tca gcc atg cgg gag gag ctg agc atg ggc cag cct cct gcc agc 1008
Leu Ser Ala Met Arg Glu Glu Leu Ser Met Gly Gln Pro Pro Ala Ser
                325                 330                 335

ctg ggc acc ctg ctc cgc atg ccc gga ctg cgc ttc cgg acc tgt atc 1056
Leu Gly Thr Leu Leu Arg Met Pro Gly Leu Arg Phe Arg Thr Cys Ile
            340                 345                 350

tcc acg ttg tgc tgg ttc gcc ttt ggc ttc acc ttc ttc ggc ctg gcc 1104
Ser Thr Leu Cys Trp Phe Ala Phe Gly Phe Thr Phe Phe Gly Leu Ala
        355                 360                 365

ctg gac ctg cag gcc ctg ggc agc aac atc ttc ctg ctc caa atg ttc 1152
Leu Asp Leu Gln Ala Leu Gly Ser Asn Ile Phe Leu Leu Gln Met Phe
    370                 375                 380

att ggt gtc gtg gac atc cca gcc aag atg ggc gcc ctg ctg ctg ctg 1200
Ile Gly Val Val Asp Ile Pro Ala Lys Met Gly Ala Leu Leu Leu Leu
385                 390                 395                 400

agc cac ctg ggc cgc cgc ccc acg ctg gcc gca tcc ctg ttg ctg gcg 1248
Ser His Leu Gly Arg Arg Pro Thr Leu Ala Ala Ser Leu Leu Leu Ala
                405                 410                 415

ggg ctc tgc att ctg gcc aac acg ctg gtg ccc cac gaa atg ggg gct 1296
Gly Leu Cys Ile Leu Ala Asn Thr Leu Val Pro His Glu Met Gly Ala
            420                 425                 430

ctg cgc tca gcc ttg gcc gtg ctg ggg ctg ggc ggg gtg ggg gct gcc 1344
Leu Arg Ser Ala Leu Ala Val Leu Gly Leu Gly Gly Val Gly Ala Ala
        435                 440                 445

ttc acc tgc atc acc atc tac agc agc gag ctc ttc ccc act gtg ctc 1392
Phe Thr Cys Ile Thr Ile Tyr Ser Ser Glu Leu Phe Pro Thr Val Leu
    450                 455                 460

agg atg acg gca gtg ggc ttg ggc cag atg gca gcc cgt gga gga gcc 1440
Arg Met Thr Ala Val Gly Leu Gly Gln Met Ala Ala Arg Gly Gly Ala
465                 470                 475                 480

atc ctg ggg cct ctg gtc cgg ctg ctg ggt gtc cat ggc ccc tgg ctg 1488
Ile Leu Gly Pro Leu Val Arg Leu Leu Gly Val His Gly Pro Trp Leu
                485                 490                 495

ccc ttg ctg gtg tat ggg acg gtg cca gtg ctg agt ggc ctg gcc gca 1536
Pro Leu Leu Val Tyr Gly Thr Val Pro Val Leu Ser Gly Leu Ala Ala
            500                 505                 510

ctg ctt ctg ccc gag acc cag agc ttg ccg ctg ccc gac acc atc caa 1584
Leu Leu Leu Pro Glu Thr Gln Ser Leu Pro Leu Pro Asp Thr Ile Gln
        515                 520                 525

gat gtg cag aac cag gca gta aag aag gca aca cat ggc acg ctg ggg 1632
Asp Val Gln Asn Gln Ala Val Lys Lys Ala Thr His Gly Thr Leu Gly
    530                 535                 540

aac tct gtc cta aaa tcc aca cag ttt tag 1662
Asn Ser Val Leu Lys Ser Thr Gln Phe *
545                 550

<210> 7
<211> 2480
<212> DNA
<213> homo sapiens

<220>
<221> CDS
<222> (148)...(2307)

<400> 7
gtcgacccac gcgtccgggc ggcaagagcc ccttgtggcc accgagtcct ccgacgccct 60
cgccaggctg gcctttgggt tggcccaggc aggacgggca gccgagagca ctcgggccgc 120
gtcgccagga gccgcccagg gtgagcc atg ttc gta ggc gtc gcc cgg cac tct 174
Met Phe Val Gly Val Ala Arg His Ser
1               5

ggg agc cag gat gaa gtc tca agg gga gta gag ccg ctg gag gcc gcg 222
Gly Ser Gln Asp Glu Val Ser Arg Gly Val Glu Pro Leu Glu Ala Ala
10                  15                  20                  25

cgg gcc cag cct gct aag gac agg agg gcc aag gga acc ccg aag tcc 270
Arg Ala Gln Pro Ala Lys Asp Arg Arg Ala Lys Gly Thr Pro Lys Ser
                30                  35                  40

tcg aag ccc ggg aaa aaa cac cgg tat ctg aga cta ctt cca gag gcc 318
Ser Lys Pro Gly Lys Lys His Arg Tyr Leu Arg Leu Leu Pro Glu Ala
            45                  50                  55

ttg ata agg ttc ggc ggt ttc cga aaa agg aaa aaa gcc aag tcc tca 366
Leu Ile Arg Phe Gly Gly Phe Arg Lys Arg Lys Lys Ala Lys Ser Ser
        60                  65                  70

gtt tcc aag aag ccg gga gaa gtg gat gac agt ttg gag cag ccc tgt 414
Val Ser Lys Lys Pro Gly Glu Val Asp Asp Ser Leu Glu Gln Pro Cys
    75                  80                  85

ggt ttg ggc tgc tta gtc agc acc tgc tgt ggg tgt tgc aat aac att 462
Gly Leu Gly Cys Leu Val Ser Thr Cys Cys Gly Cys Cys Asn Asn Ile
90                  95                  100                 105

cgc tgc ttc atg att ttc tac tgc atc ctg ctc ata tgt caa ggt gtg 510
Arg Cys Phe Met Ile Phe Tyr Cys Ile Leu Leu Ile Cys Gln Gly Val
                110                 115                 120

gtg ttt ggt ctt ata gat gtc agc att ggc gat ttt cag aag gaa tat 558
Val Phe Gly Leu Ile Asp Val Ser Ile Gly Asp Phe Gln Lys Glu Tyr
            125                 130                 135

caa ctg aaa acc att gag aag ttg gca ttg gaa aag agt tac gat att 606
Gln Leu Lys Thr Ile Glu Lys Leu Ala Leu Glu Lys Ser Tyr Asp Ile
        140                 145                 150

tca tct ggc ctg gta gca ata ttt ata gca ttc tat gga gac aga aaa 654
Ser Ser Gly Leu Val Ala Ile Phe Ile Ala Phe Tyr Gly Asp Arg Lys
    155                 160                 165

aaa gta ata tgg ttt gta gct tcc tcc ttt tta ata gga ctt gga tca 702
Lys Val Ile Trp Phe Val Ala Ser Ser Phe Leu Ile Gly Leu Gly Ser
170                 175                 180                 185

ctt tta tgt gct ttt cca tcc att aat gaa gaa aat aaa caa agt aag 750
Leu Leu Cys Ala Phe Pro Ser Ile Asn Glu Glu Asn Lys Gln Ser Lys
                190                 195                 200

gta gga att gaa gat att tgc gaa gaa ata aag gtt gtc agt ggt tgc 798
Val Gly Ile Glu Asp Ile Cys Glu Glu Ile Lys Val Val Ser Gly Cys
            205                 210                 215

cag agc agt ggt ata tca ttc caa tca aaa tac ctg tct ttc ttc atc 846
Gln Ser Ser Gly Ile Ser Phe Gln Ser Lys Tyr Leu Ser Phe Phe Ile
        220                 225                 230

ctt ggg cag act gtg cag gga ata gca gga atg cct ctt tat atc ctt 894
Leu Gly Gln Thr Val Gln Gly Ile Ala Gly Met Pro Leu Tyr Ile Leu
    235                 240                 245

gga ata acc ttt att gat gag aat gtt gct aca cac tca gct ggt atc 942
Gly Ile Thr Phe Ile Asp Glu Asn Val Ala Thr His Ser Ala Gly Ile
250                 255                 260                 265

tat tta ggt att gca gaa tgt aca tca atg att gga tat gct ctg ggt 990
Tyr Leu Gly Ile Ala Glu Cys Thr Ser Met Ile Gly Tyr Ala Leu Gly
                270                 275                 280

tat gtg cta gga gca cca cta gtt aaa gtc cct gag aat act act tct 1038
Tyr Val Leu Gly Ala Pro Leu Val Lys Val Pro Glu Asn Thr Thr Ser
            285                 290                 295

gca aca aac act aca gtc aat aat ggt agt cca gaa tgg cta tgg act 1086
Ala Thr Asn Thr Thr Val Asn Asn Gly Ser Pro Glu Trp Leu Trp Thr
        300                 305                 310

tgg tgg att aat ttt ctt ttt gcc gct gtc gtt gca tgg tgt aca tta 1134
Trp Trp Ile Asn Phe Leu Phe Ala Ala Val Val Ala Trp Cys Thr Leu
    315                 320                 325

ata cca ttg tca tgc ttt cca aac aat atg cca ggt tca aca cgg ata 1182

Ile Pro Leu Ser Cys Phe Pro Asn Asn Met Pro Gly Ser Thr Arg Ile
330                 335                 340                 345

aaa gct agg aaa cgt aaa cag ctt cat ttt ttt gac agc aga ctt aaa     1230
Lys Ala Arg Lys Arg Lys Gln Leu His Phe Phe Asp Ser Arg Leu Lys
                350                 355                 360

gat ctg aaa ctt gga act aat atc aag gat tta tgt gct gct ctt tgg     1278
Asp Leu Lys Leu Gly Thr Asn Ile Lys Asp Leu Cys Ala Ala Leu Trp
            365                 370                 375

att ctg atg aag aat cca gtg ctc ata tgc cta gct ctg tca aaa gct     1326
Ile Leu Met Lys Asn Pro Val Leu Ile Cys Leu Ala Leu Ser Lys Ala
        380                 385                 390

aca gaa tat tta gtt att att gga gct tct gaa ttt ttg cct ata tat     1374
Thr Glu Tyr Leu Val Ile Ile Gly Ala Ser Glu Phe Leu Pro Ile Tyr
    395                 400                 405

tta gaa aat cag ttt ata tta aca ccc act gtg gca act aca ctt gca     1422
Leu Glu Asn Gln Phe Ile Leu Thr Pro Thr Val Ala Thr Thr Leu Ala
410                 415                 420                 425

gga ctt gtt tta att cca gga ggt gca ctt ggc cag ctt ctg gga ggt     1470
Gly Leu Val Leu Ile Pro Gly Gly Ala Leu Gly Gln Leu Leu Gly Gly
                430                 435                 440

gtc att gtt tcc aca tta gaa atg tct tgt aaa gcc ctt atg aga ttt     1518
Val Ile Val Ser Thr Leu Glu Met Ser Cys Lys Ala Leu Met Arg Phe
            445                 450                 455

ata atg gtt aca tct gtg ata tca ctt ata ctg ctt gtg ttt att att     1566
Ile Met Val Thr Ser Val Ile Ser Leu Ile Leu Leu Val Phe Ile Ile
        460                 465                 470

ttt gta cgc tgt aat cca gtg caa ttt gct ggg atc aat gaa gat tat     1614
Phe Val Arg Cys Asn Pro Val Gln Phe Ala Gly Ile Asn Glu Asp Tyr
    475                 480                 485

gat gga aca ggg aag ttg gga aac ctc acg gct cct tgc aat gaa aaa     1662
Asp Gly Thr Gly Lys Leu Gly Asn Leu Thr Ala Pro Cys Asn Glu Lys
490                 495                 500                 505

tgt aga tgc tca tct tca att tat tct tct ata tgt gga aga gat gat     1710
Cys Arg Cys Ser Ser Ser Ile Tyr Ser Ser Ile Cys Gly Arg Asp Asp
                510                 515                 520

att gaa tat ttt tct ccc tgc ttt gca ggg tgt aca tat tct aaa gca     1758
Ile Glu Tyr Phe Ser Pro Cys Phe Ala Gly Cys Thr Tyr Ser Lys Ala
            525                 530                 535

caa aac caa aaa aag atg tac tac aat tgt tct tgc att aaa gaa gga     1806
Gln Asn Gln Lys Lys Met Tyr Tyr Asn Cys Ser Cys Ile Lys Glu Gly
        540                 545                 550

tta ata act gca gat gca gaa ggt gat ttt att gat gcc aga ccc ggg     1854
Leu Ile Thr Ala Asp Ala Glu Gly Asp Phe Ile Asp Ala Arg Pro Gly
    555                 560                 565

aaa tgt gat gca aag tgc tat aag tta cct ttg ttc att gct ttt atc     1902
Lys Cys Asp Ala Lys Cys Tyr Lys Leu Pro Leu Phe Ile Ala Phe Ile

570                 575                 580                 585

ttt tct aca ctt ata ttt tct ggt ttt tct ggt gta cca atc gtc ttg     1950
Phe Ser Thr Leu Ile Phe Ser Gly Phe Ser Gly Val Pro Ile Val Leu
                590                 595                 600

gcc atg acg cgg gtt gta cct gac aaa ctg cgt tct ctg gcc ttg ggt     1998
Ala Met Thr Arg Val Val Pro Asp Lys Leu Arg Ser Leu Ala Leu Gly
            605                 610                 615

gta agc tat gtg att ttg aga ata ttt ggg act att cct gga cca tca     2046
Val Ser Tyr Val Ile Leu Arg Ile Phe Gly Thr Ile Pro Gly Pro Ser
        620                 625                 630

atc ttt aaa atg tca gga gaa act tct tgt att tta cgg gat gtt aat     2094
Ile Phe Lys Met Ser Gly Glu Thr Ser Cys Ile Leu Arg Asp Val Asn
    635                 640                 645

aaa tgt gga cac aga gga cgt tgt tgg ata tat aac aag aca aaa atg     2142
Lys Cys Gly His Arg Gly Arg Cys Trp Ile Tyr Asn Lys Thr Lys Met
650                 655                 660                 665

gct ttc tta ttg gta gga ata tgt ttt ctt tgc aaa cta tgc act atc     2190
Ala Phe Leu Leu Val Gly Ile Cys Phe Leu Cys Lys Leu Cys Thr Ile
                670                 675                 680

atc ttc act act att gca ttt ttc ata tac aaa cgt cgt cta aat gag     2238
Ile Phe Thr Thr Ile Ala Phe Phe Ile Tyr Lys Arg Arg Leu Asn Glu
            685                 690                 695

aac act gac ttc cca gat gta act gtg aag aat cca aaa gtt aag aaa     2286
Asn Thr Asp Phe Pro Asp Val Thr Val Lys Asn Pro Lys Val Lys Lys
        700                 705                 710

aaa gaa gaa act gac ttg taa ctggatcatc attgtattct ccaagatttg     2337
Lys Glu Glu Thr Asp Leu  *
    715

tttctgtgcc caactttcag aagaggaaaa tcacacatta tgtttacata agtagcaaaa 2397
atatatttat ggtgatctgc attttcataa taaagtgtcc tattgtgaaa caaaaaaaaa 2457
aaaaaaaaaa aaagggcggc cgc                                         2480

<210> 8
<211> 719
<212> PRT
<213> homo sapiens

<400> 8
Met Phe Val Gly Val Ala Arg His Ser Gly Ser Gln Asp Glu Val Ser
1               5                   10                  15
Arg Gly Val Glu Pro Leu Glu Ala Ala Arg Ala Gln Pro Ala Lys Asp
            20                  25                  30
Arg Arg Ala Lys Gly Thr Pro Lys Ser Ser Lys Pro Gly Lys Lys His
        35                  40                  45
Arg Tyr Leu Arg Leu Leu Pro Glu Ala Leu Ile Arg Phe Gly Gly Phe
    50                  55                  60
Arg Lys Arg Lys Lys Ala Lys Ser Ser Val Ser Lys Lys Pro Gly Glu
65                  70                  75                  80
Val Asp Asp Ser Leu Glu Gln Pro Cys Gly Leu Gly Cys Leu Val Ser
                85                  90                  95
Thr Cys Cys Gly Cys Cys Asn Asn Ile Arg Cys Phe Met Ile Phe Tyr

            100                 105                 110
Cys Ile Leu Leu Ile Cys Gln Gly Val Val Phe Gly Leu Ile Asp Val
        115                 120                 125
Ser Ile Gly Asp Phe Gln Lys Glu Tyr Gln Leu Lys Thr Ile Glu Lys
    130                 135                 140
Leu Ala Leu Glu Lys Ser Tyr Asp Ile Ser Ser Gly Leu Val Ala Ile
145                 150                 155                 160
Phe Ile Ala Phe Tyr Gly Asp Arg Lys Lys Val Ile Trp Phe Val Ala
                165                 170                 175
Ser Ser Phe Leu Ile Gly Leu Gly Ser Leu Leu Cys Ala Phe Pro Ser
            180                 185                 190
Ile Asn Glu Glu Asn Lys Gln Ser Lys Val Gly Ile Glu Asp Ile Cys
        195                 200                 205
Glu Glu Ile Lys Val Val Ser Gly Cys Gln Ser Ser Gly Ile Ser Phe
    210                 215                 220
Gln Ser Lys Tyr Leu Ser Phe Phe Ile Leu Gly Gln Thr Val Gln Gly
225                 230                 235                 240
Ile Ala Gly Met Pro Leu Tyr Ile Leu Gly Ile Thr Phe Ile Asp Glu
                245                 250                 255
Asn Val Ala Thr His Ser Ala Gly Ile Tyr Leu Gly Ile Ala Glu Cys
            260                 265                 270
Thr Ser Met Ile Gly Tyr Ala Leu Gly Tyr Val Leu Gly Ala Pro Leu
        275                 280                 285
Val Lys Val Pro Glu Asn Thr Thr Ser Ala Thr Asn Thr Thr Val Asn
    290                 295                 300
Asn Gly Ser Pro Glu Trp Leu Trp Thr Trp Trp Ile Asn Phe Leu Phe
305                 310                 315                 320
Ala Ala Val Val Ala Trp Cys Thr Leu Ile Pro Leu Ser Cys Phe Pro
                325                 330                 335
Asn Asn Met Pro Gly Ser Thr Arg Ile Lys Ala Arg Lys Arg Lys Gln
            340                 345                 350
Leu His Phe Phe Asp Ser Arg Leu Lys Asp Leu Lys Leu Gly Thr Asn
        355                 360                 365
Ile Lys Asp Leu Cys Ala Ala Leu Trp Ile Leu Met Lys Asn Pro Val
    370                 375                 380
Leu Ile Cys Leu Ala Leu Ser Lys Ala Thr Glu Tyr Leu Val Ile Ile
385                 390                 395                 400
Gly Ala Ser Glu Phe Leu Pro Ile Tyr Leu Glu Asn Gln Phe Ile Leu
                405                 410                 415
Thr Pro Thr Val Ala Thr Thr Leu Ala Gly Leu Val Leu Ile Pro Gly
            420                 425                 430
Gly Ala Leu Gly Gln Leu Leu Gly Gly Val Ile Val Ser Thr Leu Glu
        435                 440                 445
Met Ser Cys Lys Ala Leu Met Arg Phe Ile Met Val Thr Ser Val Ile
    450                 455                 460
Ser Leu Ile Leu Leu Val Phe Ile Ile Phe Val Arg Cys Asn Pro Val
465                 470                 475                 480
Gln Phe Ala Gly Ile Asn Glu Asp Tyr Asp Gly Thr Gly Lys Leu Gly
                485                 490                 495
Asn Leu Thr Ala Pro Cys Asn Glu Lys Cys Arg Cys Ser Ser Ser Ile
            500                 505                 510
Tyr Ser Ser Ile Cys Gly Arg Asp Asp Ile Glu Tyr Phe Ser Pro Cys
        515                 520                 525
Phe Ala Gly Cys Thr Tyr Ser Lys Ala Gln Asn Gln Lys Lys Met Tyr
    530                 535                 540
Tyr Asn Cys Ser Cys Ile Lys Glu Gly Leu Ile Thr Ala Asp Ala Glu
545                 550                 555                 560
Gly Asp Phe Ile Asp Ala Arg Pro Gly Lys Cys Asp Ala Lys Cys Tyr
                565                 570                 575
Lys Leu Pro Leu Phe Ile Ala Phe Ile Phe Ser Thr Leu Ile Phe Ser
            580                 585                 590

Gly Phe Ser Gly Val Pro Ile Val Leu Ala Met Thr Arg Val Val Pro
        595                 600                 605
Asp Lys Leu Arg Ser Leu Ala Leu Gly Val Ser Tyr Val Ile Leu Arg
    610                 615                 620
Ile Phe Gly Thr Ile Pro Gly Pro Ser Ile Phe Lys Met Ser Gly Glu
625                 630                 635                 640
Thr Ser Cys Ile Leu Arg Asp Val Asn Lys Cys Gly His Arg Gly Arg
                645                 650                 655
Cys Trp Ile Tyr Asn Lys Thr Lys Met Ala Phe Leu Leu Val Gly Ile
            660                 665                 670
Cys Phe Leu Cys Lys Leu Cys Thr Ile Ile Phe Thr Thr Ile Ala Phe
        675                 680                 685
Phe Ile Tyr Lys Arg Arg Leu Asn Glu Asn Thr Asp Phe Pro Asp Val
    690                 695                 700
Thr Val Lys Asn Pro Lys Val Lys Lys Lys Glu Glu Thr Asp Leu
705                 710                 715


<210> 9
<211> 2160
<212> DNA
<213> homo sapiens

<220>
<221> CDS
<222> (1)...(2160)

<400> 9
atg ttc gta ggc gtc gcc cgg cac tct ggg agc cag gat gaa gtc tca      48
Met Phe Val Gly Val Ala Arg His Ser Gly Ser Gln Asp Glu Val Ser
 1               5                  10                  15

agg gga gta gag ccg ctg gag gcc gcg cgg gcc cag cct gct aag gac      96
Arg Gly Val Glu Pro Leu Glu Ala Ala Arg Ala Gln Pro Ala Lys Asp
            20                  25                  30

agg agg gcc aag gga acc ccg aag tcc tcg aag ccc ggg aaa aaa cac     144
Arg Arg Ala Lys Gly Thr Pro Lys Ser Ser Lys Pro Gly Lys Lys His
        35                  40                  45

cgg tat ctg aga cta ctt cca gag gcc ttg ata agg ttc ggc ggt ttc     192
Arg Tyr Leu Arg Leu Leu Pro Glu Ala Leu Ile Arg Phe Gly Gly Phe
    50                  55                  60

cga aaa agg aaa aaa gcc aag tcc tca gtt tcc aag aag ccg gga gaa     240
Arg Lys Arg Lys Lys Ala Lys Ser Ser Val Ser Lys Lys Pro Gly Glu
 65                  70                  75                  80

gtg gat gac agt ttg gag cag ccc tgt ggt ttg ggc tgc tta gtc agc     288
Val Asp Asp Ser Leu Glu Gln Pro Cys Gly Leu Gly Cys Leu Val Ser
                85                  90                  95

acc tgc tgt ggg tgt tgc aat aac att cgc tgc ttc atg att ttc tac     336
Thr Cys Cys Gly Cys Cys Asn Asn Ile Arg Cys Phe Met Ile Phe Tyr
            100                 105                 110

tgc atc ctg ctc ata tgt caa ggt gtg gtg ttt ggt ctt ata gat gtc     384
Cys Ile Leu Leu Ile Cys Gln Gly Val Val Phe Gly Leu Ile Asp Val
        115                 120                 125

agc att ggc gat ttt cag aag gaa tat caa ctg aaa acc att gag aag     432

Ser Ile Gly Asp Phe Gln Lys Glu Tyr Gln Leu Lys Thr Ile Glu Lys
    130                 135                 140

ttg gca ttg gaa aag agt tac gat att tca tct ggc ctg gta gca ata      480
Leu Ala Leu Glu Lys Ser Tyr Asp Ile Ser Ser Gly Leu Val Ala Ile
145                 150                 155                 160

ttt ata gca ttc tat gga gac aga aaa aaa gta ata tgg ttt gta gct      528
Phe Ile Ala Phe Tyr Gly Asp Arg Lys Lys Val Ile Trp Phe Val Ala
                165                 170                 175

tcc tcc ttt tta ata gga ctt gga tca ctt tta tgt gct ttt cca tcc      576
Ser Ser Phe Leu Ile Gly Leu Gly Ser Leu Leu Cys Ala Phe Pro Ser
            180                 185                 190

att aat gaa gaa aat aaa caa agt aag gta gga att gaa gat att tgc      624
Ile Asn Glu Glu Asn Lys Gln Ser Lys Val Gly Ile Glu Asp Ile Cys
        195                 200                 205

gaa gaa ata aag gtt gtc agt ggt tgc cag agc agt ggt ata tca ttc      672
Glu Glu Ile Lys Val Val Ser Gly Cys Gln Ser Ser Gly Ile Ser Phe
    210                 215                 220

caa tca aaa tac ctg tct ttc ttc atc ctt ggg cag act gtg cag gga      720
Gln Ser Lys Tyr Leu Ser Phe Phe Ile Leu Gly Gln Thr Val Gln Gly
225                 230                 235                 240

ata gca gga atg cct ctt tat atc ctt gga ata acc ttt att gat gag      768
Ile Ala Gly Met Pro Leu Tyr Ile Leu Gly Ile Thr Phe Ile Asp Glu
                245                 250                 255

aat gtt gct aca cac tca gct ggt atc tat tta ggt att gca gaa tgt      816
Asn Val Ala Thr His Ser Ala Gly Ile Tyr Leu Gly Ile Ala Glu Cys
            260                 265                 270

aca tca atg att gga tat gct ctg ggt tat gtg cta gga gca cca cta      864
Thr Ser Met Ile Gly Tyr Ala Leu Gly Tyr Val Leu Gly Ala Pro Leu
        275                 280                 285

gtt aaa gtc cct gag aat act act tct gca aca aac act aca gtc aat      912
Val Lys Val Pro Glu Asn Thr Thr Ser Ala Thr Asn Thr Thr Val Asn
    290                 295                 300

aat ggt agt cca gaa tgg cta tgg act tgg tgg att aat ttt ctt ttt      960
Asn Gly Ser Pro Glu Trp Leu Trp Thr Trp Trp Ile Asn Phe Leu Phe
305                 310                 315                 320

gcc gct gtc gtt gca tgg tgt aca tta ata cca ttg tca tgc ttt cca     1008
Ala Ala Val Val Ala Trp Cys Thr Leu Ile Pro Leu Ser Cys Phe Pro
                325                 330                 335

aac aat atg cca ggt tca aca cgg ata aaa gct agg aaa cgt aaa cag     1056
Asn Asn Met Pro Gly Ser Thr Arg Ile Lys Ala Arg Lys Arg Lys Gln
            340                 345                 350

ctt cat ttt ttt gac agc aga ctt aaa gat ctg aaa ctt gga act aat     1104
Leu His Phe Phe Asp Ser Arg Leu Lys Asp Leu Lys Leu Gly Thr Asn
        355                 360                 365

atc aag gat tta tgt gct gct ctt tgg att ctg atg aag aat cca gtg     1152
Ile Lys Asp Leu Cys Ala Ala Leu Trp Ile Leu Met Lys Asn Pro Val

370                 375                 380

ctc ata tgc cta gct ctg tca aaa gct aca gaa tat tta gtt att att     1200
Leu Ile Cys Leu Ala Leu Ser Lys Ala Thr Glu Tyr Leu Val Ile Ile
385                 390                 395                 400

gga gct tct gaa ttt ttg cct ata tat tta gaa aat cag ttt ata tta     1248
Gly Ala Ser Glu Phe Leu Pro Ile Tyr Leu Glu Asn Gln Phe Ile Leu
                405                 410                 415

aca ccc act gtg gca act aca ctt gca gga ctt gtt tta att cca gga     1296
Thr Pro Thr Val Ala Thr Thr Leu Ala Gly Leu Val Leu Ile Pro Gly
            420                 425                 430

ggt gca ctt ggc cag ctt ctg gga ggt gtc att gtt tcc aca tta gaa     1344
Gly Ala Leu Gly Gln Leu Leu Gly Gly Val Ile Val Ser Thr Leu Glu
        435                 440                 445

atg tct tgt aaa gcc ctt atg aga ttt ata atg gtt aca tct gtg ata     1392
Met Ser Cys Lys Ala Leu Met Arg Phe Ile Met Val Thr Ser Val Ile
    450                 455                 460

tca ctt ata ctg ctt gtg ttt att att ttt gta cgc tgt aat cca gtg     1440
Ser Leu Ile Leu Leu Val Phe Ile Ile Phe Val Arg Cys Asn Pro Val
465                 470                 475                 480

caa ttt gct ggg atc aat gaa gat tat gat gga aca ggg aag ttg gga     1488
Gln Phe Ala Gly Ile Asn Glu Asp Tyr Asp Gly Thr Gly Lys Leu Gly
                485                 490                 495

aac ctc acg gct cct tgc aat gaa aaa tgt aga tgc tca tct tca att     1536
Asn Leu Thr Ala Pro Cys Asn Glu Lys Cys Arg Cys Ser Ser Ser Ile
            500                 505                 510

tat tct tct ata tgt gga aga gat gat att gaa tat ttt tct ccc tgc     1584
Tyr Ser Ser Ile Cys Gly Arg Asp Asp Ile Glu Tyr Phe Ser Pro Cys
        515                 520                 525

ttt gca ggg tgt aca tat tct aaa gca caa aac caa aaa aag atg tac     1632
Phe Ala Gly Cys Thr Tyr Ser Lys Ala Gln Asn Gln Lys Lys Met Tyr
    530                 535                 540

tac aat tgt tct tgc att aaa gaa gga tta ata act gca gat gca gaa     1680
Tyr Asn Cys Ser Cys Ile Lys Glu Gly Leu Ile Thr Ala Asp Ala Glu
545                 550                 555                 560

ggt gat ttt att gat gcc aga ccc ggg aaa tgt gat gca aag tgc tat     1728
Gly Asp Phe Ile Asp Ala Arg Pro Gly Lys Cys Asp Ala Lys Cys Tyr
                565                 570                 575

aag tta cct ttg ttc att gct ttt atc ttt tct aca ctt ata ttt tct     1776
Lys Leu Pro Leu Phe Ile Ala Phe Ile Phe Ser Thr Leu Ile Phe Ser
            580                 585                 590

ggt ttt tct ggt gta cca atc gtc ttg gcc atg acg cgg gtt gta cct     1824
Gly Phe Ser Gly Val Pro Ile Val Leu Ala Met Thr Arg Val Val Pro
        595                 600                 605

gac aaa ctg cgt tct ctg gcc ttg ggt gta agc tat gtg att ttg aga     1872
Asp Lys Leu Arg Ser Leu Ala Leu Gly Val Ser Tyr Val Ile Leu Arg
    610                 615                 620

ata ttt ggg act att cct gga cca tca atc ttt aaa atg tca gga gaa     1920
Ile Phe Gly Thr Ile Pro Gly Pro Ser Ile Phe Lys Met Ser Gly Glu
625                 630                 635                 640

act tct tgt att tta cgg gat gtt aat aaa tgt gga cac aga gga cgt     1968
Thr Ser Cys Ile Leu Arg Asp Val Asn Lys Cys Gly His Arg Gly Arg
                645                 650                 655

tgt tgg ata tat aac aag aca aaa atg gct ttc tta ttg gta gga ata     2016
Cys Trp Ile Tyr Asn Lys Thr Lys Met Ala Phe Leu Leu Val Gly Ile
            660                 665                 670

tgt ttt ctt tgc aaa cta tgc act atc atc ttc act act att gca ttt     2064
Cys Phe Leu Cys Lys Leu Cys Thr Ile Ile Phe Thr Thr Ile Ala Phe
        675                 680                 685

ttc ata tac aaa cgt cgt cta aat gag aac act gac ttc cca gat gta     2112
Phe Ile Tyr Lys Arg Arg Leu Asn Glu Asn Thr Asp Phe Pro Asp Val
    690                 695                 700

act gtg aag aat cca aaa gtt aag aaa aaa gaa gaa act gac ttg taa     2160
Thr Val Lys Asn Pro Lys Val Lys Lys Lys Glu Glu Thr Asp Leu  *
705                 710                 715


<210> 10
<211> 61
<212> PRT
<213> Artificial Sequence

<220>
<223> consensus

<400> 10
Cys Ser Arg Tyr Pro Asn Pro Thr Ser Lys Asp Gly Lys Leu Val Ala
1               5                   10                  15
Cys Pro Arg Glu Tyr Asp Pro Val Cys Gly Ser Asp Gly Val Thr Tyr
            20                  25                  30
Ser Asn Glu Cys Glu Leu Lys Lys Ala Ala Cys Ala Glu Asn Val Glu
        35                  40                  45
Gln Gly Thr Asn Ile Glu Lys Lys His Asp Gly Pro Cys
    50                  55                  60


<210> 11
<211> 58
<212> PRT
<213> Artificial Sequence

<220>
<223> consensus

<400> 11
Asp Cys Ser Glu Tyr Arg Ser Pro Thr Ser Gly Gly Leu Leu Ala Cys
1               5                   10                  15
Pro Arg Glu Tyr Asp Pro Val Cys Gly Ser Asp Gly Val Thr Tyr Ser
            20                  25                  30
Asn Glu Cys Glu Leu Cys Ala Ala Ala Cys Glu Ala Glu Gln Gly Lys
        35                  40                  45

Ser Ile Glu Val Lys His Asp Gly Pro Cys
    50                  55

<210> 12
<211> 192
<212> PRT
<213> Artificial Sequence

<220>
<223> consensus

<400> 12
Cys Asn Glu Gln Cys Ser Cys Glu Thr Ser Thr Trp His Pro Val Cys
1               5                   10                  15
Gly Asp Asp Asn Gly Leu Ala Tyr Tyr Ser Pro Cys His Ala Gly Cys
            20                  25                  30
Ser Glu Thr Asn Gln Ser Ser Gly Thr Gly Thr Asn Met Val Phe Thr
        35                  40                  45
Asn Cys Ser Cys Val Gln Thr Ser Gly Asn Ser Ser Ala Val Val Lys
    50                  55                  60
Lys Cys Cys Lys Asn Pro Glu Cys Gln Asn Lys Leu Gln Tyr Phe Leu
65                  70                  75                  80
Ile Leu Met Ile Phe Gly Ser Phe Ile Tyr Ser Leu Ala Ala Val Pro
                85                  90                  95
Gly Tyr Met Val Ile Leu Arg Cys Val Asn Pro Glu Glu Lys Ser Leu
            100                 105                 110
Ala Leu Gly Leu His Trp Phe Cys Val Arg Ile Phe Gly Thr Ile Pro
        115                 120                 125
Ala Pro Ile Ile Phe Gly Leu Leu Ile Asp Met Ser Cys Leu His Trp
    130                 135                 140
Asn Lys Gln Cys Cys Gly Glu Arg Gly Ser Cys Arg Met Tyr Asp Asn
145                 150                 155                 160
Asp Ser Leu Arg Asn Met Tyr His Gly Leu Thr Val Ala Leu Arg Val
                165                 170                 175
Ile Ser Leu Ile Pro Ala Phe Phe Val Trp Trp Leu Met Lys Lys Asn
            180                 185                 190

<210> 13
<211> 712
<212> PRT
<213> homo sapiens

<400> 13
Met Asp Thr Ser Ser Lys Glu Asn Ile Gln Leu Phe Cys Lys Thr Ser
1               5                   10                  15
Val Gln Pro Val Gly Arg Pro Ser Phe Lys Thr Glu Tyr Pro Ser Ser
            20                  25                  30
Glu Glu Lys Gln Pro Cys Cys Gly Glu Leu Lys Val Phe Leu Cys Ala
        35                  40                  45
Leu Ser Phe Val Tyr Phe Ala Lys Ala Leu Ala Glu Gly Tyr Leu Lys
    50                  55                  60
Ser Thr Ile Thr Gln Ile Glu Arg Arg Phe Asp Ile Pro Ser Ser Leu
65                  70                  75                  80
Val Gly Val Ile Asp Gly Ser Phe Glu Ile Gly Asn Leu Leu Val Ile
                85                  90                  95
Thr Phe Val Ser Tyr Phe Gly Ala Lys Leu His Arg Pro Lys Ile Ile
            100                 105                 110
Gly Ala Gly Cys Val Ile Met Gly Val Gly Thr Leu Leu Ile Ala Met
        115                 120                 125

Pro Gln Phe Phe Met Glu Gln Tyr Lys Tyr Glu Arg Tyr Ser Pro Ser
    130                 135                 140
Ser Asn Ser Thr Leu Ser Ile Ser Pro Cys Leu Leu Glu Ser Ser Ser
145                 150                 155                 160
Gln Leu Pro Val Ser Val Met Glu Lys Ser Lys Ser Lys Ile Ser Asn
                165                 170                 175
Glu Cys Glu Val Asp Thr Ser Ser Ser Met Trp Ile Tyr Val Phe Leu
            180                 185                 190
Gly Asn Leu Leu Arg Gly Ile Gly Glu Thr Pro Ile Gln Pro Leu Gly
        195                 200                 205
Ile Ala Tyr Leu Asp Asp Phe Ala Ser Glu Asp Asn Ala Ala Phe Tyr
    210                 215                 220
Ile Gly Cys Val Gln Thr Val Ala Ile Ile Gly Pro Ile Phe Gly Phe
225                 230                 235                 240
Leu Leu Gly Ser Leu Cys Ala Lys Leu Tyr Val Asp Ile Gly Phe Val
                245                 250                 255
Asn Leu Asp His Ile Thr Ile Thr Pro Lys Asp Pro Gln Trp Val Gly
            260                 265                 270
Ala Trp Trp Leu Gly Tyr Leu Ile Ala Gly Ile Ile Ser Leu Leu Ala
        275                 280                 285
Ala Val Pro Phe Trp Tyr Leu Pro Lys Ser Leu Pro Arg Ser Gln Ser
    290                 295                 300
Arg Glu Asp Ser Asn Ser Ser Ser Glu Lys Ser Lys Phe Ile Ile Asp
305                 310                 315                 320
Asp His Thr Asp Tyr Gln Thr Pro Gln Gly Glu Asn Ala Lys Ile Met
                325                 330                 335
Glu Met Ala Arg Asp Phe Leu Pro Ser Leu Lys Asn Leu Phe Gly Asn
            340                 345                 350
Pro Val Tyr Phe Leu Tyr Leu Cys Thr Ser Thr Val Gln Phe Asn Ser
        355                 360                 365
Leu Phe Gly Met Val Thr Tyr Lys Pro Lys Tyr Ile Glu Gln Gln Tyr
    370                 375                 380
Gly Gln Ser Ser Ser Arg Ala Asn Phe Val Ile Gly Leu Ile Asn Ile
385                 390                 395                 400
Pro Ala Val Ala Leu Gly Ile Phe Ser Gly Gly Ile Val Met Lys Lys
                405                 410                 415
Phe Arg Ile Ser Val Cys Gly Ala Ala Lys Leu Tyr Leu Gly Ser Ser
            420                 425                 430
Val Phe Gly Tyr Leu Leu Phe Leu Ser Leu Phe Ala Leu Gly Cys Glu
        435                 440                 445
Asn Ser Asp Val Ala Gly Leu Thr Val Ser Tyr Gln Gly Thr Lys Pro
    450                 455                 460
Val Ser Tyr His Glu Arg Ala Leu Phe Ser Asp Cys Asn Ser Arg Cys
465                 470                 475                 480
Lys Cys Ser Glu Thr Lys Trp Glu Pro Met Cys Gly Glu Asn Gly Ile
                485                 490                 495
Thr Tyr Val Ser Ala Cys Leu Ala Gly Cys Gln Thr Ser Asn Arg Ser
            500                 505                 510
Gly Lys Asn Ile Ile Phe Tyr Asn Cys Thr Cys Val Gly Ile Ala Ala
        515                 520                 525
Ser Lys Ser Gly Asn Ser Ser Gly Ile Val Gly Arg Cys Gln Lys Asp
    530                 535                 540
Asn Gly Cys Pro Gln Met Phe Leu Tyr Phe Leu Val Ile Ser Val Ile
545                 550                 555                 560
Thr Ser Tyr Thr Leu Ser Leu Gly Gly Ile Pro Gly Tyr Ile Leu Leu
                565                 570                 575
Leu Arg Cys Ile Lys Pro Gln Leu Lys Ser Phe Ala Leu Gly Ile Tyr
            580                 585                 590
Thr Leu Ala Ile Arg Val Leu Ala Gly Ile Pro Ala Pro Val Tyr Phe
        595                 600                 605
Gly Val Leu Ile Asp Thr Ser Cys Leu Lys Trp Gly Phe Lys Arg Cys

610                 615                 620
Gly Ser Arg Gly Ser Cys Arg Leu Tyr Asp Ser Asn Val Phe Arg His
625                 630                 635                 640
Ile Tyr Leu Gly Leu Thr Val Ile Leu Gly Thr Val Ser Ile Leu Leu
                645                 650                 655
Ser Ile Ala Val Leu Phe Ile Leu Lys Lys Asn Tyr Val Ser Lys His
            660                 665                 670
Arg Ser Phe Ile Thr Lys Arg Glu Arg Thr Met Val Ser Thr Arg Phe
        675                 680                 685
Gln Lys Glu Asn Tyr Thr Thr Ser Asp His Leu Leu Gln Pro Asn Tyr
    690                 695                 700
Trp Pro Gly Lys Glu Thr Gln Leu
705                 710

<210> 14
<211> 487
<212> PRT
<213> Artificial Sequence

<220>
<223> consensus

<400> 14
Val Ala Leu Val Ala Ala Leu Gly Gly Gly Phe Leu Phe Gly Tyr Asp
1               5                   10                  15
Thr Gly Val Ile Gly Gly Phe Leu Ala Leu Ile Asp Phe Leu Phe Arg
            20                  25                  30
Phe Gly Leu Leu Thr Ser Ser Gly Ala Leu Ala Glu Leu Gly Tyr Ser
        35                  40                  45
Thr Val Leu Thr Gly Leu Val Val Ser Ile Phe Phe Leu Gly Arg Leu
    50                  55                  60
Ile Gly Ser Leu Phe Ala Gly Lys Leu Gly Asp Arg Phe Gly Arg Lys
65                  70                  75                  80
Lys Ser Leu Leu Ile Ala Leu Val Leu Phe Val Ile Gly Ala Leu Leu
                85                  90                  95
Ser Gly Ala Ala Pro Gly Tyr Thr Thr Ile Gly Leu Trp Ala Phe Tyr
            100                 105                 110
Leu Leu Ile Val Gly Arg Val Leu Val Gly Leu Gly Val Gly Gly Ala
        115                 120                 125
Ser Val Leu Val Pro Met Tyr Ile Ser Glu Ile Ala Pro Lys Ala Leu
    130                 135                 140
Arg Gly Ala Leu Gly Ser Leu Tyr Gln Leu Ala Ile Thr Ile Gly Ile
145                 150                 155                 160
Leu Val Ala Ala Ile Ile Gly Leu Gly Leu Asn Lys Thr Asn Asn Asp
                165                 170                 175
Ser Ala Leu Asn Ser Trp Gly Trp Arg Ile Pro Leu Gly Leu Gln Leu
            180                 185                 190
Val Pro Ala Leu Leu Leu Leu Ile Gly Leu Leu Phe Leu Pro Glu Ser
        195                 200                 205
Pro Arg Trp Leu Val Glu Lys Gly Lys Leu Glu Glu Ala Arg Glu Val
    210                 215                 220
Leu Ala Lys Leu Arg Gly Val Glu Asp Val Asp Gln Glu Ile Gln Glu
225                 230                 235                 240
Ile Lys Ala Glu Leu Glu Ala Gly Val Glu Glu Glu Lys Ala Gly Lys
                245                 250                 255
Ala Ser Trp Gly Glu Leu Phe Arg Gly Arg Thr Arg Pro Lys Val Arg
            260                 265                 270
Gln Arg Leu Leu Met Gly Val Met Leu Gln Ala Phe Gln Gln Leu Thr
        275                 280                 285
Gly Ile Asn Ala Ile Phe Tyr Tyr Ser Pro Thr Ile Phe Lys Ser Val

290                 295                 300
Gly Val Ser Asp Ser Arg Ala Ser Leu Leu Val Thr Ile Ile Val Gly
305                 310                 315                 320
Val Val Asn Phe Val Phe Thr Leu Val Ala Leu Ile Phe Leu Val Asp
                325                 330                 335
Arg Phe Gly Arg Arg Pro Leu Leu Leu Leu Gly Ala Ala Gly Met Ala
            340                 345                 350
Ile Cys Phe Leu Ile Leu Gly Ala Ser Ile Gly Val Ala Leu Leu Leu
        355                 360                 365
Leu Asn Lys Pro Lys Asp Pro Leu Ser Lys Ala Ala Gly Ile Val Ala
    370                 375                 380
Ile Val Phe Ile Leu Leu Phe Ile Ala Phe Phe Ala Leu Gly Trp Gly
385                 390                 395                 400
Pro Ile Pro Trp Val Ile Leu Ser Glu Leu Phe Pro Thr Lys Val Arg
                405                 410                 415
Ser Lys Ala Leu Ala Leu Ala Thr Ala Ala Asn Trp Leu Ala Asn Phe
            420                 425                 430
Ile Ile Gly Phe Leu Phe Pro Tyr Ile Thr Gly Ala Ile Gly Leu Ala
        435                 440                 445
Leu Gly Gly Tyr Val Phe Leu Val Phe Ala Gly Leu Leu Val Leu Phe
    450                 455                 460
Ile Leu Phe Val Phe Phe Phe Val Pro Glu Thr Lys Gly Arg Thr Leu
465                 470                 475                 480
Glu Glu Ile Glu Glu Leu Phe
                485

<210> 15
<211> 44
<212> PRT
<213> Artificial Sequence

<220>
<223> consensus

<400> 15
Asn Gly Thr Glu Pro Asn Gly Ser Asp Pro Val Thr Glu Pro Cys Ser
1               5                   10                  15
Asp Gly Trp Val Tyr Asp His Ser Thr Phe Pro Ser Thr Ile Val Thr
            20                  25                  30
Glu Trp Asp Leu Val Cys Asn His Arg Ala Leu Arg
        35                  40

<210> 16
<211> 550
<212> PRT
<213> homo sapiens

<400> 16
Met Ala Phe Ser Lys Leu Leu Glu Gln Ala Gly Gly Val Gly Leu Phe
1               5                   10                  15
Gln Thr Leu Gln Val Leu Thr Phe Ile Leu Pro Cys Leu Met Ile Pro
            20                  25                  30
Ser Gln Met Leu Leu Glu Asn Phe Ser Ala Ala Ile Pro Gly His Arg
        35                  40                  45
Cys Trp Thr His Met Leu Asp Asn Gly Ser Ala Val Ser Thr Asn Met
    50                  55                  60
Thr Pro Lys Ala Leu Leu Thr Ile Ser Ile Pro Pro Gly Pro Asn Gln
65                  70                  75                  80
Gly Pro His Gln Cys Arg Arg Phe Arg Gln Pro Gln Trp Gln Leu Leu

                85                  90                  95
Asp Pro Asn Ala Thr Ala Thr Ser Trp Ser Glu Ala Asp Thr Glu Pro
            100                 105                 110
Cys Val Asp Gly Trp Val Tyr Asp Arg Ser Val Phe Thr Ser Thr Ile
        115                 120                 125
Val Ala Lys Trp Asp Leu Val Cys Ser Ser Gln Gly Leu Lys Pro Leu
    130                 135                 140
Ser Gln Ser Ile Phe Met Ser Gly Ile Leu Val Gly Ser Phe Ile Trp
145                 150                 155                 160
Gly Leu Leu Ser Tyr Arg Phe Gly Arg Lys Pro Met Leu Ser Trp Cys
                165                 170                 175
Cys Leu Gln Leu Ala Val Ala Gly Thr Ser Thr Ile Phe Ala Pro Thr
            180                 185                 190
Phe Val Ile Tyr Cys Gly Leu Arg Phe Val Ala Ala Phe Gly Met Ala
        195                 200                 205
Gly Ile Phe Leu Ser Ser Leu Thr Leu Met Val Glu Trp Thr Thr Thr
    210                 215                 220
Ser Arg Arg Ala Val Thr Met Thr Val Val Gly Cys Ala Phe Ser Ala
225                 230                 235                 240
Gly Gln Ala Ala Leu Gly Gly Leu Ala Phe Ala Leu Arg Asp Trp Arg
                245                 250                 255
Thr Leu Gln Leu Ala Ala Ser Val Pro Phe Phe Ala Ile Ser Leu Ile
            260                 265                 270
Ser Trp Trp Leu Pro Glu Ser Ala Arg Trp Leu Ile Ile Lys Gly Lys
        275                 280                 285
Pro Asp Gln Ala Leu Gln Glu Leu Arg Lys Val Ala Arg Ile Asn Gly
    290                 295                 300
His Lys Glu Ala Lys Asn Leu Thr Ile Glu Val Leu Met Ser Ser Val
305                 310                 315                 320
Lys Glu Glu Val Ala Ser Ala Lys Glu Pro Arg Ser Val Leu Asp Leu
                325                 330                 335
Phe Cys Val Pro Val Leu Arg Trp Arg Ser Cys Ala Met Leu Val Val
            340                 345                 350
Asn Phe Ser Leu Leu Ile Ser Tyr Tyr Gly Leu Val Phe Asp Leu Gln
        355                 360                 365
Ser Leu Gly Arg Asp Ile Phe Leu Leu Gln Ala Leu Phe Gly Ala Val
    370                 375                 380
Asp Phe Leu Gly Arg Ala Thr Thr Ala Leu Leu Leu Ser Phe Leu Gly
385                 390                 395                 400
Arg Arg Thr Ile Gln Ala Gly Ser Gln Ala Met Ala Gly Leu Ala Ile
                405                 410                 415
Leu Ala Asn Met Leu Val Pro Gln Asp Leu Gln Thr Leu Arg Val Val
            420                 425                 430
Phe Ala Val Leu Gly Lys Gly Cys Phe Gly Ile Ser Leu Thr Cys Leu
        435                 440                 445
Thr Ile Tyr Lys Ala Glu Leu Phe Pro Thr Pro Val Arg Met Thr Ala
    450                 455                 460
Asp Gly Ile Leu His Thr Val Gly Arg Leu Gly Ala Met Met Gly Pro
465                 470                 475                 480
Leu Ile Leu Met Ser Arg Gln Ala Leu Pro Leu Leu Pro Pro Leu Leu
                485                 490                 495
Tyr Gly Val Ile Ser Ile Ala Ser Ser Leu Val Val Leu Phe Phe Leu
            500                 505                 510
Pro Glu Thr Gln Gly Leu Pro Leu Pro Asp Thr Ile Gln Asp Leu Glu
        515                 520                 525
Ser Gln Lys Ser Thr Ala Ala Gln Gly Asn Arg Gln Glu Ala Val Thr
    530                 535                 540
Val Glu Ser Thr Ser Leu
545                 550

<210> 17
<211> 553
<212> PRT
<213> Mus musculus

<400> 17
Met Ala Phe Pro Glu Leu Leu Asp Arg Val Gly Gly Leu Gly Arg Phe
1               5                   10                  15
Gln Leu Phe Gln Thr Val Ala Leu Val Thr Pro Ile Leu Trp Val Thr
            20                  25                  30
Thr Gln Asn Met Leu Glu Asn Phe Ser Ala Ala Val Pro His His Arg
        35                  40                  45
Cys Trp Val Pro Leu Leu Asp Asn Ser Thr Ser Gln Ala Ser Ile Pro
    50                  55                  60
Gly Asp Leu Gly Pro Asp Val Leu Leu Ala Val Ser Ile Pro Pro Gly
65                  70                  75                  80
Pro Asp Gln Gln Pro His Gln Cys Leu Arg Phe Arg Gln Pro Gln Trp
                85                  90                  95
Gln Leu Thr Glu Ser Asn Ala Thr Ala Thr Asn Trp Ser Asp Ala Ala
            100                 105                 110
Thr Glu Pro Cys Glu Asp Gly Trp Val Tyr Asp His Ser Thr Phe Arg
        115                 120                 125
Ser Thr Ile Val Thr Thr Trp Asp Leu Val Cys Asn Ser Gln Ala Leu
    130                 135                 140
Arg Pro Met Ala Gln Ser Ile Phe Leu Ala Gly Ile Leu Val Gly Ala
145                 150                 155                 160
Ala Val Cys Gly His Ala Ser Asp Arg Phe Gly Arg Arg Arg Val Leu
                165                 170                 175
Thr Trp Ser Tyr Leu Leu Val Ser Val Ser Gly Thr Ala Ala Ala Phe
            180                 185                 190
Met Pro Thr Phe Pro Leu Tyr Cys Leu Phe Arg Phe Leu Leu Ala Ser
        195                 200                 205
Ala Val Ala Gly Val Met Met Asn Thr Ala Ser Leu Leu Met Glu Trp
    210                 215                 220
Thr Ser Ala Gln Gly Ser Pro Leu Val Met Thr Leu Asn Ala Leu Gly
225                 230                 235                 240
Phe Ser Phe Gly Gln Val Leu Thr Gly Ser Val Ala Tyr Gly Val Arg
                245                 250                 255
Ser Trp Arg Met Leu Gln Leu Ala Val Ser Ala Pro Phe Phe Leu Phe
            260                 265                 270
Phe Val Tyr Ser Trp Trp Leu Pro Glu Ser Ala Arg Trp Leu Ile Thr
        275                 280                 285
Val Gly Lys Leu Asp Gln Gly Leu Gln Glu Leu Gln Arg Val Ala Ala
    290                 295                 300
Val Asn Arg Arg Lys Ala Glu Gly Asp Thr Leu Thr Met Glu Val Leu
305                 310                 315                 320
Arg Ser Ala Met Glu Glu Glu Pro Ser Arg Asp Lys Ala Gly Ala Ser
                325                 330                 335
Leu Gly Thr Leu Leu His Thr Pro Gly Leu Arg His Arg Thr Ile Ile
            340                 345                 350
Ser Met Leu Cys Trp Phe Ala Phe Gly Phe Thr Phe Tyr Gly Leu Ala
        355                 360                 365
Leu Asp Leu Gln Ala Leu Gly Ser Asn Ile Phe Leu Leu Gln Ala Leu
    370                 375                 380
Ile Gly Ile Val Asp Phe Pro Val Lys Thr Gly Ser Leu Leu Leu Ile
385                 390                 395                 400
Ser Arg Leu Gly Arg Arg Leu Cys Gln Val Ser Phe Leu Val Leu Pro
                405                 410                 415
Gly Leu Cys Ile Leu Ser Asn Ile Leu Val Pro His Gly Met Gly Val
            420                 425                 430
Leu Arg Ser Ala Leu Ala Val Leu Gly Leu Gly Cys Leu Gly Gly Ala

435                 440                 445
Phe Thr Cys Ile Thr Ile Phe Ser Ser Glu Leu Phe Pro Thr Val Ile
    450                 455                 460
Arg Met Thr Ala Val Gly Leu Cys Gln Val Ala Ala Arg Gly Gly Ala
465                 470                 475                 480
Met Leu Gly Pro Leu Val Arg Leu Leu Gly Val Tyr Gly Ser Trp Met
                485                 490                 495
Pro Leu Leu Val Tyr Gly Val Val Pro Val Leu Ser Gly Leu Ala Ala
            500                 505                 510
Leu Leu Leu Pro Glu Thr Lys Asn Leu Pro Leu Pro Asp Thr Ile Gln
        515                 520                 525
Asp Ile Gln Lys Gln Ser Val Lys Lys Val Thr His Asp Thr Pro Asp
    530                 535                 540
Gly Ser Ile Leu Met Ser Thr Arg Leu
545                 550

<210> 18
<211> 722
<212> PRT
<213> homo sapiens

<400> 18
Met Pro Leu His Gln Leu Gly Asp Lys Pro Leu Thr Phe Pro Ser Pro
 1               5                  10                  15
Asn Ser Ala Met Glu Asn Gly Leu Asp His Thr Pro Pro Ser Arg Arg
            20                  25                  30
Ala Ser Pro Gly Thr Pro Leu Ser Pro Gly Ser Leu Arg Ser Ala Ala
        35                  40                  45
His Ser Pro Leu Asp Thr Ser Lys Gln Pro Leu Cys Gln Leu Trp Ala
    50                  55                  60
Glu Lys His Gly Ala Arg Gly Thr His Glu Val Arg Tyr Val Ser Ala
65                  70                  75                  80
Gly Gln Ser Val Ala Cys Gly Trp Trp Ala Phe Ala Pro Pro Cys Leu
                85                  90                  95
Gln Val Leu Asn Thr Pro Lys Gly Ile Leu Phe Phe Leu Cys Ala Ala
            100                 105                 110
Ala Phe Leu Gln Gly Met Thr Val Asn Gly Phe Ile Asn Thr Val Ile
        115                 120                 125
Thr Ser Leu Glu Arg Arg Tyr Asp Leu His Ser Tyr Gln Ser Gly Leu
    130                 135                 140
Ile Ala Ser Ser Tyr Asp Ile Ala Ala Cys Leu Cys Leu Thr Phe Val
145                 150                 155                 160
Ser Tyr Phe Gly Gly Ser Gly His Lys Pro Arg Trp Leu Gly Trp Gly
                165                 170                 175
Val Leu Leu Met Gly Thr Gly Ser Leu Val Phe Ala Leu Pro His Phe
            180                 185                 190
Thr Ala Gly Arg Tyr Glu Val Glu Leu Asp Ala Gly Val Arg Thr Cys
        195                 200                 205
Pro Ala Asn Pro Gly Ala Val Cys Ala Asp Ser Thr Ser Gly Leu Ser
    210                 215                 220
Arg Tyr Gln Leu Val Phe Met Leu Gly Gln Phe Leu His Gly Val Gly
225                 230                 235                 240
Ala Thr Pro Leu Tyr Thr Leu Gly Val Thr Tyr Leu Asp Glu Asn Val
                245                 250                 255
Lys Ser Ser Cys Ser Pro Val Tyr Ile Ala Ile Phe Tyr Thr Ala Ala
            260                 265                 270
Ile Leu Gly Pro Ala Ala Gly Tyr Leu Ile Gly Gly Ala Leu Leu Asn
        275                 280                 285
Ile Tyr Thr Glu Met Gly Arg Arg Thr Glu Leu Thr Thr Glu Ser Pro
    290                 295                 300

Leu Trp Val Gly Ala Trp Trp Val Gly Phe Leu Gly Ser Gly Ala Ala
305                 310                 315                 320
Ala Phe Phe Thr Ala Val Pro Ile Leu Gly Tyr Pro Arg Gln Leu Pro
                325                 330                 335
Gly Ser Gln Arg Tyr Ala Val Met Arg Ala Ala Glu Met His Gln Leu
            340                 345                 350
Lys Asp Ser Ser Arg Gly Glu Ala Ser Asn Pro Asp Phe Gly Lys Thr
        355                 360                 365
Ile Arg Asp Leu Pro Leu Ser Ile Trp Leu Leu Leu Lys Asn Pro Thr
    370                 375                 380
Phe Ile Leu Leu Cys Leu Ala Gly Ala Thr Glu Ala Thr Leu Ile Thr
385                 390                 395                 400
Gly Met Ser Thr Phe Ser Pro Lys Phe Leu Glu Ser Gln Phe Ser Leu
                405                 410                 415
Ser Ala Ser Glu Ala Ala Thr Leu Phe Gly Tyr Leu Val Val Pro Ala
            420                 425                 430
Gly Gly Gly Gly Thr Phe Leu Gly Gly Phe Phe Val Asn Lys Leu Arg
        435                 440                 445
Leu Arg Gly Ser Ala Val Ile Lys Phe Cys Leu Phe Cys Thr Val Val
    450                 455                 460
Ser Leu Leu Gly Ile Leu Val Phe Ser Leu His Cys Pro Ser Val Pro
465                 470                 475                 480
Met Ala Gly Val Thr Ala Ser Tyr Gly Gly Ser Leu Leu Pro Glu Gly
                485                 490                 495
His Leu Asn Leu Thr Ala Pro Cys Asn Ala Ala Cys Ser Cys Gln Pro
            500                 505                 510
Glu His Tyr Ser Pro Val Cys Gly Ser Asp Gly Leu Met Tyr Phe Ser
        515                 520                 525
Leu Cys His Ala Gly Cys Pro Ala Ala Thr Glu Thr Asn Val Asp Gly
    530                 535                 540
Gln Lys Val Tyr Arg Asp Cys Ser Cys Ile Pro Gln Asn Leu Ser Ser
545                 550                 555                 560
Gly Phe Gly His Ala Thr Ala Gly Lys Cys Thr Ser Thr Cys Gln Arg
                565                 570                 575
Lys Pro Leu Leu Leu Val Phe Ile Phe Val Val Ile Phe Phe Thr Phe
            580                 585                 590
Leu Ser Ser Ile Pro Ala Leu Thr Ala Thr Leu Arg Cys Val Arg Asp
        595                 600                 605
Pro Gln Arg Ser Phe Ala Leu Gly Ile Gln Trp Ile Val Val Arg Ile
    610                 615                 620
Leu Gly Gly Ile Pro Gly Pro Ile Ala Phe Gly Trp Val Ile Asp Lys
625                 630                 635                 640
Ala Cys Leu Leu Trp Gln Asp Gln Cys Gly Gln Gln Gly Ser Cys Leu
                645                 650                 655
Val Tyr Gln Asn Ser Ala Met Ser Arg Tyr Ile Leu Ile Met Gly Leu
            660                 665                 670
Leu Tyr Lys Val Leu Gly Val Leu Phe Phe Ala Ile Ala Cys Phe Leu
        675                 680                 685
Tyr Lys Pro Leu Ser Glu Ser Ser Asp Gly Leu Glu Thr Cys Leu Pro
    690                 695                 700
Ser Gln Ser Ser Ala Pro Asp Ser Ala Thr Asp Ser Gln Leu Gln Ser
705                 710                 715                 720
Ser Val

<210> 19
<211> 9
<212> PRT
<213> Artificial Sequence

<220>
<223> consensus

<221> VARIANT
<222> 1
<223> The amino acid residue at position 1 can be Arg or
      Lys.

<221> VARIANT
<222> 2
<223> The amino acid residue at position 2 can be Leu or
      Ile.

<221> VARIANT
<222> (3)...(7)
<223> The amino acid residue at positions 3 through 7
      can be any amino acid.

<221> VARIANT
<222> 8
<223> The amino acid residue at position 8 can be His or
      Gln.

<400> 19
Xaa Xaa Xaa Xaa Xaa Xaa Xaa Xaa Leu
 1               5


<210> 20
<211> 18
<212> PRT
<213> Artificial Sequence

<220>
<223> consensus

<221> VARIANT
<222> 1
<223> The amino acid residue at position 1 can be Leu,
      Ile, Val, Met, Ser, Thr, Ala, or Gly.

<221> VARIANT
<222> 2
<223> The amino acid residue at position 2 can be Leu,
      Ile, Val, Met, Phe, Ser, Ala, or Gly.

<221> VARIANT
<222> 3,4,7,12-17
<223> The amino acid residue at these positions can be
      any amino acid.  The length of the segment with
      any amino acid after position 11 can be 4, 5, or 6
      residues.

<221> VARIANT
<222> 5
<223> The amino acid residue at position 5 can be Leu,
      Ile, Val, Met, Ser, or Ala.

<221> VARIANT
<222> 6
<223> The amino acid residue at position 6 can be Asp or

Glu.

<221> VARIANT
<222> 8
<223> The amino acid residue at position 8 can be Leu,
      Ile, Val, Met, Phe, Tyr, Trp, or Ala.

<221> VARIANT
<222> 11
<223> The amino acid residue at position 11 can be Arg
      or Lys.

<221> VARIANT
<222> 18
<223> The amino acid residue at position 18 can be Gly,
      Ser, Thr, or Ala.

<400> 20
Xaa Xaa Xaa Xaa Xaa Xaa Xaa Xaa Gly Arg Xaa Xaa Xaa Xaa Xaa Xaa
 1               5                   10                  15
Xaa Xaa

<210> 21
<211> 26
<212> PRT
<213> Artificial Sequence

<220>
<223> consensus

<221> VARIANT
<222> 1
<223> The amino acid residue at position 1 can be Leu,
      Ile, Val, Met, or Phe.

<221> VARIANT
<222> 2,5,6,8-15,17,18,20-25
<223> The amino acid residues at these positions can be
      any amino acid.

<221> VARIANT
<222> 4
<223> The amino acid residue at position 4 can be Leu,
      Ile, Val, Met, Phe, or Ala.

<221> VARIANT
<222> 16
<223> The amino acid residue at position 16 can be Leu,
      Ile, Phe, or Tyr.

<221> VARIANT
<222> 19
<223> The amino acid residue at position 19 can be Glu
      or Gln.

<221> VARIANT
<222> 26
<223> The amino acid residue at position 26 can be Arg
      or Lys.

<400> 21
Xaa Xaa Gly Xaa Xaa Xaa Gly Xaa Xaa Xaa Xaa Xaa Xaa Xaa Xaa Xaa
1               5                   10                  15
Xaa Xaa Xaa Xaa Xaa Xaa Xaa Xaa Xaa Xaa
            20                  25

<210> 22
<211> 3227
<212> DNA
<213> homo sapiens

<220>
<221> CDS
<222> (345)...(2483)

<400> 22
ccctttctcg ggaagcgcgc cattgtgttg gtacccggga attcgcggcc gcgtcgaccc 60
ccctgcggag ttgtgttttc tgggaatcag caacaaaytg caaagaaatg gctcaaaagc 120
ttcagctctt tctgtgccct gggagctgag atgcacgtca gtggccttgc cagcgtggcc 180
aattctctgc tgactgccag aaaaaagagg ccaggaagaa agaggaaaga gaagagatcg 240
ctcagggtct gtggtgtgtg gtccatcctc ttgctgagca cattgaaagg aactggctat 300
ctttgatctc ttcctccaga tcagagtcaa ggaatgtgtt tata atg gac act tca 356
                                                 Met Asp Thr Ser
                                                  1

tcc aaa gaa aat atc cag ttg ttc tgc aaa act tca gtg caa cct gtt 404
Ser Lys Glu Asn Ile Gln Leu Phe Cys Lys Thr Ser Val Gln Pro Val
5                   10                  15                  20

gga agg cct tct ttt aaa aca gaa tat ccc tcc tca gaa gaa aag caa 452
Gly Arg Pro Ser Phe Lys Thr Glu Tyr Pro Ser Ser Glu Glu Lys Gln
                25                  30                  35

cca tgc tgt ggt gaa cta aag gtg ttc ttg tgt gcc ttg tct ttt gtt 500
Pro Cys Cys Gly Glu Leu Lys Val Phe Leu Cys Ala Leu Ser Phe Val
            40                  45                  50

tac ttt gcc aaa gca ttg gca gaa ggc tat ctg aag agc acc atc act 548
Tyr Phe Ala Lys Ala Leu Ala Glu Gly Tyr Leu Lys Ser Thr Ile Thr
        55                  60                  65

cag ata gag aga agg ttt gat atc cct tct tca ctg gtg gga gtt att 596
Gln Ile Glu Arg Arg Phe Asp Ile Pro Ser Ser Leu Val Gly Val Ile
    70                  75                  80

gat ggt agt ttt gaa att ggg aat ctc tta gtt ata aca ttt gtt agc 644
Asp Gly Ser Phe Glu Ile Gly Asn Leu Leu Val Ile Thr Phe Val Ser
85                  90                  95                  100

tac ttt gga gcc aaa ctt cac agg cca aaa ata att gga gca ggg tgt 692
Tyr Phe Gly Ala Lys Leu His Arg Pro Lys Ile Ile Gly Ala Gly Cys
                105                 110                 115

gta atc atg gga gtt gga aca ctg ctc att gca atg cct cag ttc ttc 740
Val Ile Met Gly Val Gly Thr Leu Leu Ile Ala Met Pro Gln Phe Phe
            120                 125                 130

atg gag cag tac aaa tat gag aga tat tct cct tcc tcc aat tct act 788
Met Glu Gln Tyr Lys Tyr Glu Arg Tyr Ser Pro Ser Ser Asn Ser Thr

        135                 140                 145

ctc agc atc tct ccg tgt ctc cta gag tca agc agt caa tta cca gtt 836
Leu Ser Ile Ser Pro Cys Leu Leu Glu Ser Ser Ser Gln Leu Pro Val
    150                 155                 160

tca gtt atg gaa aaa tca aaa tcc aaa ata agt aac gaa tgt gaa gtg 884
Ser Val Met Glu Lys Ser Lys Ser Lys Ile Ser Asn Glu Cys Glu Val
165                 170                 175                 180

gac act agc tct tcc atg tgg att tat gtt ttc ctg ggc aat ctt ctt 932
Asp Thr Ser Ser Ser Met Trp Ile Tyr Val Phe Leu Gly Asn Leu Leu
                185                 190                 195

cgt gga ata gga gaa act ccc att cag cct ttg ggc att gcc tac ctg 980
Arg Gly Ile Gly Glu Thr Pro Ile Gln Pro Leu Gly Ile Ala Tyr Leu
            200                 205                 210

gat gat ttt gcc agt gaa gac aat gca gct ttc tat att ggg tgt gtg 1028
Asp Asp Phe Ala Ser Glu Asp Asn Ala Ala Phe Tyr Ile Gly Cys Val
        215                 220                 225

cag acg gtt gca att ata gga cca atc ttt ggt ttc ctg tta ggc tca 1076
Gln Thr Val Ala Ile Ile Gly Pro Ile Phe Gly Phe Leu Leu Gly Ser
    230                 235                 240

tta tgt gcc aaa cta tat gtt gac att ggc ttt gta aac cta gat cac 1124
Leu Cys Ala Lys Leu Tyr Val Asp Ile Gly Phe Val Asn Leu Asp His
245                 250                 255                 260

ata acc att acc cca aaa gat ccc cag tgg gta gga gcc tgg tgg ctt 1172
Ile Thr Ile Thr Pro Lys Asp Pro Gln Trp Val Gly Ala Trp Trp Leu
                265                 270                 275

ggc tat cta ata gca gga atc ata agt ctt ctt gca gct gtg cct ttc 1220
Gly Tyr Leu Ile Ala Gly Ile Ile Ser Leu Leu Ala Ala Val Pro Phe
            280                 285                 290

tgg tat tta cca aag agt tta cca aga tcc caa agt aga gag gat tct 1268
Trp Tyr Leu Pro Lys Ser Leu Pro Arg Ser Gln Ser Arg Glu Asp Ser
        295                 300                 305

aat tct tcc tct gag aaa tcc aag ttt att ata gat gat cac aca gac 1316
Asn Ser Ser Ser Glu Lys Ser Lys Phe Ile Ile Asp Asp His Thr Asp
    310                 315                 320

tac caa aca ccc cag gga gaa aat gca aaa ata atg gaa atg gca aga 1364
Tyr Gln Thr Pro Gln Gly Glu Asn Ala Lys Ile Met Glu Met Ala Arg
325                 330                 335                 340

gat ttt ctt cca tca ctg aag aat ctt ttt gga aac cca gta tac ttc 1412
Asp Phe Leu Pro Ser Leu Lys Asn Leu Phe Gly Asn Pro Val Tyr Phe
                345                 350                 355

cta tat tta tgt aca agc act gtt cag ttc aat tct ctg ttc ggc atg 1460
Leu Tyr Leu Cys Thr Ser Thr Val Gln Phe Asn Ser Leu Phe Gly Met
            360                 365                 370

gtg acg tac aaa cca aag tac att gag cag cag tat gga cag tca tcc 1508
Val Thr Tyr Lys Pro Lys Tyr Ile Glu Gln Gln Tyr Gly Gln Ser Ser
        375                 380                 385

tcc agg gcc aac ttt gtg atc ggg ctc atc aac att cca gca gtg gcc     1556
Ser Arg Ala Asn Phe Val Ile Gly Leu Ile Asn Ile Pro Ala Val Ala
    390                 395                 400

ctt gga ata ttc tct ggg ggg ata gtt atg aaa aaa ttc aga atc agt     1604
Leu Gly Ile Phe Ser Gly Gly Ile Val Met Lys Lys Phe Arg Ile Ser
405                 410                 415                 420

gtg tgt gga gct gca aaa ctc tac ttg gga tca tct gtc ttt ggt tac     1652
Val Cys Gly Ala Ala Lys Leu Tyr Leu Gly Ser Ser Val Phe Gly Tyr
                425                 430                 435

ctc cta ttt ctt tcc ctg ttt gca ctg ggc tgt gaa aat tct gat gtg     1700
Leu Leu Phe Leu Ser Leu Phe Ala Leu Gly Cys Glu Asn Ser Asp Val
            440                 445                 450

gca gga cta act gtc tcc tac caa gga acc aaa cct gtc tct tat cat     1748
Ala Gly Leu Thr Val Ser Tyr Gln Gly Thr Lys Pro Val Ser Tyr His
        455                 460                 465

gaa cga gct ctc ttt tca gat tgc aac tca aga tgc aaa tgt tca gag     1796
Glu Arg Ala Leu Phe Ser Asp Cys Asn Ser Arg Cys Lys Cys Ser Glu
    470                 475                 480

aca aaa tgg gaa ccc atg tgc ggt gaa aat gga atc aca tat gta tca     1844
Thr Lys Trp Glu Pro Met Cys Gly Glu Asn Gly Ile Thr Tyr Val Ser
485                 490                 495                 500

gct tgt ctt gct ggt tgt caa acc tcc aac agg agt gga aaa aat att     1892
Ala Cys Leu Ala Gly Cys Gln Thr Ser Asn Arg Ser Gly Lys Asn Ile
                505                 510                 515

ata ttt tac aac tgc act tgt gtg gga att gca gct tct aaa tcc gga     1940
Ile Phe Tyr Asn Cys Thr Cys Val Gly Ile Ala Ala Ser Lys Ser Gly
            520                 525                 530

aat tcc tca ggc ata gtg gga aga tgt cag aaa gac aat gga tgt ccc     1988
Asn Ser Ser Gly Ile Val Gly Arg Cys Gln Lys Asp Asn Gly Cys Pro
        535                 540                 545

caa atg ttt ctg tat ttc ctt gta att tca gtc atc aca tcc tat act     2036
Gln Met Phe Leu Tyr Phe Leu Val Ile Ser Val Ile Thr Ser Tyr Thr
    550                 555                 560

tta tcc cta ggt ggc ata cct gga tac ata tta ctt ctg agg tgc att     2084
Leu Ser Leu Gly Gly Ile Pro Gly Tyr Ile Leu Leu Leu Arg Cys Ile
565                 570                 575                 580

aag cca cag ctt aag tct ttt gcc ttg ggt atc tac aca tta gca ata     2132
Lys Pro Gln Leu Lys Ser Phe Ala Leu Gly Ile Tyr Thr Leu Ala Ile
                585                 590                 595

aga gtt ctt gca gga atc cca gct cca gtg tat ttt gga gtt ttg att     2180
Arg Val Leu Ala Gly Ile Pro Ala Pro Val Tyr Phe Gly Val Leu Ile
            600                 605                 610

gat act tca tgc ctc aaa tgg gga ttt aaa aga tgt gga agt aga gga     2228
Asp Thr Ser Cys Leu Lys Trp Gly Phe Lys Arg Cys Gly Ser Arg Gly
        615                 620                 625

tca tgc aga tta tat gat tca aat gtc ttc aga cat ata tat ctg gga 2276
Ser Cys Arg Leu Tyr Asp Ser Asn Val Phe Arg His Ile Tyr Leu Gly
    630                 635                 640

cta act gtg ata ctg ggc aca gtg tca att ctc cta agc att gca gta 2324
Leu Thr Val Ile Leu Gly Thr Val Ser Ile Leu Leu Ser Ile Ala Val
645                 650                 655                 660

ctt ttc att tta aag aaa aat tat gtt tca aaa cac aga agt ttt ata 2372
Leu Phe Ile Leu Lys Lys Asn Tyr Val Ser Lys His Arg Ser Phe Ile
                665                 670                 675

acc aag aga gaa aga aca atg gtg tct aca aga ttc caa aag gaa aat 2420
Thr Lys Arg Glu Arg Thr Met Val Ser Thr Arg Phe Gln Lys Glu Asn
            680                 685                 690

tac act aca agt gat cat ctg cta caa ccc aac tac tgg cca ggc aag 2468
Tyr Thr Thr Ser Asp His Leu Leu Gln Pro Asn Tyr Trp Pro Gly Lys
        695                 700                 705

gaa act caa ctt tag aaacatgatg actggaagtc atgtcttcta attggttgac 2523
Glu Thr Gln Leu *
    710

attttgcaaa caaataaatt gtaatcaaaa gagctctaaa tttgtaattt ctttctcctt 2583
tcaaaaaatg tctactttgt tttggtccta ggcattaggt aatataactg ataatatact 2643
gaaacatata atggaagatg cagatgataa aactaatttt gaacttttta atttatataa 2703
attattttat atcacttact tatttcactt tattttgctt tgtgctcatt gatatatatt 2763
agctgtactc ctagaagaac aattgtctct attgtcacac atggttatat ttaaagtaat 2823
ttctgaactg tgtaatgtgt ctagagtaag caaatactgc taacaattaa ctcatacctt 2883
gggttccttc aagtattact cctatagtat tttctcccat agctgtcttc atctgtgtat 2943
tttaataatg atcttaggat ggagcagaac atggagagga agatttcatt ttaagctcct 3003
ccttttcttt gaaatacaat aatttatata gaaatgtgta gcagcaaatt atattgggga 3063
ttagaatttt gaattaatag ctctcctact attaatttac atgtgctttt tgtgtggcgc 3123
tataagtgac tatggttgta aagtaataaa attgatgtta acatgcccaa aaaaaaaaaa 3183
aaaaaaaacc aaaaaaaaaa aaaaaaaagg gcgggccgct agac 3227

<210> 23
<211> 712
<212> PRT
<213> Homo sapiens

<400> 23
Met Asp Thr Ser Ser Lys Glu Asn Ile Gln Leu Phe Cys Lys Thr Ser
1               5                   10                  15
Val Gln Pro Val Gly Arg Pro Ser Phe Lys Thr Glu Tyr Pro Ser Ser
            20                  25                  30
Glu Glu Lys Gln Pro Cys Cys Gly Glu Leu Lys Val Phe Leu Cys Ala
        35                  40                  45
Leu Ser Phe Val Tyr Phe Ala Lys Ala Leu Ala Glu Gly Tyr Leu Lys
    50                  55                  60
Ser Thr Ile Thr Gln Ile Glu Arg Arg Phe Asp Ile Pro Ser Ser Leu
65                  70                  75                  80
Val Gly Val Ile Asp Gly Ser Phe Glu Ile Gly Asn Leu Leu Val Ile
                85                  90                  95
Thr Phe Val Ser Tyr Phe Gly Ala Lys Leu His Arg Pro Lys Ile Ile
            100                 105                 110
Gly Ala Gly Cys Val Ile Met Gly Val Gly Thr Leu Leu Ile Ala Met
        115                 120                 125
Pro Gln Phe Phe Met Glu Gln Tyr Lys Tyr Glu Arg Tyr Ser Pro Ser
    130                 135                 140

Ser Asn Ser Thr Leu Ser Ile Ser Pro Cys Leu Leu Glu Ser Ser Ser
145                 150                 155                 160
Gln Leu Pro Val Ser Val Met Glu Lys Ser Lys Ser Lys Ile Ser Asn
                165                 170                 175
Glu Cys Glu Val Asp Thr Ser Ser Ser Met Trp Ile Tyr Val Phe Leu
            180                 185                 190
Gly Asn Leu Leu Arg Gly Ile Gly Glu Thr Pro Ile Gln Pro Leu Gly
        195                 200                 205
Ile Ala Tyr Leu Asp Asp Phe Ala Ser Glu Asp Asn Ala Ala Phe Tyr
    210                 215                 220
Ile Gly Cys Val Gln Thr Val Ala Ile Ile Gly Pro Ile Phe Gly Phe
225                 230                 235                 240
Leu Leu Gly Ser Leu Cys Ala Lys Leu Tyr Val Asp Ile Gly Phe Val
                245                 250                 255
Asn Leu Asp His Ile Thr Ile Thr Pro Lys Asp Pro Gln Trp Val Gly
            260                 265                 270
Ala Trp Trp Leu Gly Tyr Leu Ile Ala Gly Ile Ile Ser Leu Leu Ala
        275                 280                 285
Ala Val Pro Phe Trp Tyr Leu Pro Lys Ser Leu Pro Arg Ser Gln Ser
    290                 295                 300
Arg Glu Asp Ser Asn Ser Ser Ser Glu Lys Ser Lys Phe Ile Ile Asp
305                 310                 315                 320
Asp His Thr Asp Tyr Gln Thr Pro Gln Gly Glu Asn Ala Lys Ile Met
                325                 330                 335
Glu Met Ala Arg Asp Phe Leu Pro Ser Leu Lys Asn Leu Phe Gly Asn
            340                 345                 350
Pro Val Tyr Phe Leu Tyr Leu Cys Thr Ser Thr Val Gln Phe Asn Ser
        355                 360                 365
Leu Phe Gly Met Val Thr Tyr Lys Pro Lys Tyr Ile Glu Gln Gln Tyr
    370                 375                 380
Gly Gln Ser Ser Ser Arg Ala Asn Phe Val Ile Gly Leu Ile Asn Ile
385                 390                 395                 400
Pro Ala Val Ala Leu Gly Ile Phe Ser Gly Gly Ile Val Met Lys Lys
                405                 410                 415
Phe Arg Ile Ser Val Cys Gly Ala Ala Lys Leu Tyr Leu Gly Ser Ser
            420                 425                 430
Val Phe Gly Tyr Leu Leu Phe Leu Ser Leu Phe Ala Leu Gly Cys Glu
        435                 440                 445
Asn Ser Asp Val Ala Gly Leu Thr Val Ser Tyr Gln Gly Thr Lys Pro
    450                 455                 460
Val Ser Tyr His Glu Arg Ala Leu Phe Ser Asp Cys Asn Ser Arg Cys
465                 470                 475                 480
Lys Cys Ser Glu Thr Lys Trp Glu Pro Met Cys Gly Glu Asn Gly Ile
                485                 490                 495
Thr Tyr Val Ser Ala Cys Leu Ala Gly Cys Gln Thr Ser Asn Arg Ser
            500                 505                 510
Gly Lys Asn Ile Ile Phe Tyr Asn Cys Thr Cys Val Gly Ile Ala Ala
        515                 520                 525
Ser Lys Ser Gly Asn Ser Ser Gly Ile Val Gly Arg Cys Gln Lys Asp
    530                 535                 540
Asn Gly Cys Pro Gln Met Phe Leu Tyr Phe Leu Val Ile Ser Val Ile
545                 550                 555                 560
Thr Ser Tyr Thr Leu Ser Leu Gly Gly Ile Pro Gly Tyr Ile Leu Leu
                565                 570                 575
Leu Arg Cys Ile Lys Pro Gln Leu Lys Ser Phe Ala Leu Gly Ile Tyr
            580                 585                 590
Thr Leu Ala Ile Arg Val Leu Ala Gly Ile Pro Ala Pro Val Tyr Phe
        595                 600                 605
Gly Val Leu Ile Asp Thr Ser Cys Leu Lys Trp Gly Phe Lys Arg Cys
    610                 615                 620
Gly Ser Arg Gly Ser Cys Arg Leu Tyr Asp Ser Asn Val Phe Arg His

625                 630                 635                 640
Ile Tyr Leu Gly Leu Thr Val Ile Leu Gly Thr Val Ser Ile Leu Leu
                645                 650                 655
Ser Ile Ala Val Leu Phe Ile Leu Lys Lys Asn Tyr Val Ser Lys His
            660                 665                 670
Arg Ser Phe Ile Thr Lys Arg Glu Arg Thr Met Val Ser Thr Arg Phe
        675                 680                 685
Gln Lys Glu Asn Tyr Thr Thr Ser Asp His Leu Leu Gln Pro Asn Tyr
    690                 695                 700
Trp Pro Gly Lys Glu Thr Gln Leu
705                 710

<210> 24
<211> 2139
<212> DNA
<213> Homo sapiens

<220>
<221> CDS
<222> (1)...(2139)

<400> 24
atg gac act tca tcc aaa gaa aat atc cag ttg ttc tgc aaa act tca      48
Met Asp Thr Ser Ser Lys Glu Asn Ile Gln Leu Phe Cys Lys Thr Ser
 1               5                  10                  15

gtg caa cct gtt gga agg cct tct ttt aaa aca gaa tat ccc tcc tca      96
Val Gln Pro Val Gly Arg Pro Ser Phe Lys Thr Glu Tyr Pro Ser Ser
            20                  25                  30

gaa gaa aag caa cca tgc tgt ggt gaa cta aag gtg ttc ttg tgt gcc     144
Glu Glu Lys Gln Pro Cys Cys Gly Glu Leu Lys Val Phe Leu Cys Ala
        35                  40                  45

ttg tct ttt gtt tac ttt gcc aaa gca ttg gca gaa ggc tat ctg aag     192
Leu Ser Phe Val Tyr Phe Ala Lys Ala Leu Ala Glu Gly Tyr Leu Lys
    50                  55                  60

agc acc atc act cag ata gag aga agg ttt gat atc cct tct tca ctg     240
Ser Thr Ile Thr Gln Ile Glu Arg Arg Phe Asp Ile Pro Ser Ser Leu
65                  70                  75                  80

gtg gga gtt att gat ggt agt ttt gaa att ggg aat ctc tta gtt ata     288
Val Gly Val Ile Asp Gly Ser Phe Glu Ile Gly Asn Leu Leu Val Ile
                85                  90                  95

aca ttt gtt agc tac ttt gga gcc aaa ctt cac agg cca aaa ata att     336
Thr Phe Val Ser Tyr Phe Gly Ala Lys Leu His Arg Pro Lys Ile Ile
            100                 105                 110

gga gca ggg tgt gta atc atg gga gtt gga aca ctg ctc att gca atg     384
Gly Ala Gly Cys Val Ile Met Gly Val Gly Thr Leu Leu Ile Ala Met
        115                 120                 125

cct cag ttc ttc atg gag cag tac aaa tat gag aga tat tct cct tcc     432
Pro Gln Phe Phe Met Glu Gln Tyr Lys Tyr Glu Arg Tyr Ser Pro Ser
    130                 135                 140

tcc aat tct act ctc agc atc tct ccg tgt ctc cta gag tca agc agt     480
Ser Asn Ser Thr Leu Ser Ile Ser Pro Cys Leu Leu Glu Ser Ser Ser

145                 150                 155                 160

caa tta cca gtt tca gtt atg gaa aaa tca aaa tcc aaa ata agt aac      528
Gln Leu Pro Val Ser Val Met Glu Lys Ser Lys Ser Lys Ile Ser Asn
                165                 170                 175

gaa tgt gaa gtg gac act agc tct tcc atg tgg att tat gtt ttc ctg      576
Glu Cys Glu Val Asp Thr Ser Ser Ser Met Trp Ile Tyr Val Phe Leu
            180                 185                 190

ggc aat ctt ctt cgt gga ata gga gaa act ccc att cag cct ttg ggc      624
Gly Asn Leu Leu Arg Gly Ile Gly Glu Thr Pro Ile Gln Pro Leu Gly
        195                 200                 205

att gcc tac ctg gat gat ttt gcc agt gaa gac aat gca gct ttc tat      672
Ile Ala Tyr Leu Asp Asp Phe Ala Ser Glu Asp Asn Ala Ala Phe Tyr
    210                 215                 220

att ggg tgt gtg cag acg gtt gca att ata gga cca atc ttt ggt ttc      720
Ile Gly Cys Val Gln Thr Val Ala Ile Ile Gly Pro Ile Phe Gly Phe
225                 230                 235                 240

ctg tta ggc tca tta tgt gcc aaa cta tat gtt gac att ggc ttt gta      768
Leu Leu Gly Ser Leu Cys Ala Lys Leu Tyr Val Asp Ile Gly Phe Val
                245                 250                 255

aac cta gat cac ata acc att acc cca aaa gat ccc cag tgg gta gga      816
Asn Leu Asp His Ile Thr Ile Thr Pro Lys Asp Pro Gln Trp Val Gly
            260                 265                 270

gcc tgg tgg ctt ggc tat cta ata gca gga atc ata agt ctt ctt gca      864
Ala Trp Trp Leu Gly Tyr Leu Ile Ala Gly Ile Ile Ser Leu Leu Ala
        275                 280                 285

gct gtg cct ttc tgg tat tta cca aag agt tta cca aga tcc caa agt      912
Ala Val Pro Phe Trp Tyr Leu Pro Lys Ser Leu Pro Arg Ser Gln Ser
    290                 295                 300

aga gag gat tct aat tct tcc tct gag aaa tcc aag ttt att ata gat      960
Arg Glu Asp Ser Asn Ser Ser Ser Glu Lys Ser Lys Phe Ile Ile Asp
305                 310                 315                 320

gat cac aca gac tac caa aca ccc cag gga gaa aat gca aaa ata atg     1008
Asp His Thr Asp Tyr Gln Thr Pro Gln Gly Glu Asn Ala Lys Ile Met
                325                 330                 335

gaa atg gca aga gat ttt ctt cca tca ctg aag aat ctt ttt gga aac     1056
Glu Met Ala Arg Asp Phe Leu Pro Ser Leu Lys Asn Leu Phe Gly Asn
            340                 345                 350

cca gta tac ttc cta tat tta tgt aca agc act gtt cag ttc aat tct     1104
Pro Val Tyr Phe Leu Tyr Leu Cys Thr Ser Thr Val Gln Phe Asn Ser
        355                 360                 365

ctg ttc ggc atg gtg acg tac aaa cca aag tac att gag cag cag tat     1152
Leu Phe Gly Met Val Thr Tyr Lys Pro Lys Tyr Ile Glu Gln Gln Tyr
    370                 375                 380

gga cag tca tcc tcc agg gcc aac ttt gtg atc ggg ctc atc aac att     1200
Gly Gln Ser Ser Ser Arg Ala Asn Phe Val Ile Gly Leu Ile Asn Ile
385                 390                 395                 400

cca gca gtg gcc ctt gga ata ttc tct ggg ggg ata gtt atg aaa aaa 1248
Pro Ala Val Ala Leu Gly Ile Phe Ser Gly Gly Ile Val Met Lys Lys
                405                 410                 415

ttc aga atc agt gtg tgt gga gct gca aaa ctc tac ttg gga tca tct 1296
Phe Arg Ile Ser Val Cys Gly Ala Ala Lys Leu Tyr Leu Gly Ser Ser
            420                 425                 430

gtc ttt ggt tac ctc cta ttt ctt tcc ctg ttt gca ctg ggc tgt gaa 1344
Val Phe Gly Tyr Leu Leu Phe Leu Ser Leu Phe Ala Leu Gly Cys Glu
        435                 440                 445

aat tct gat gtg gca gga cta act gtc tcc tac caa gga acc aaa cct 1392
Asn Ser Asp Val Ala Gly Leu Thr Val Ser Tyr Gln Gly Thr Lys Pro
    450                 455                 460

gtc tct tat cat gaa cga gct ctc ttt tca gat tgc aac tca aga tgc 1440
Val Ser Tyr His Glu Arg Ala Leu Phe Ser Asp Cys Asn Ser Arg Cys
465                 470                 475                 480

aaa tgt tca gag aca aaa tgg gaa ccc atg tgc ggt gaa aat gga atc 1488
Lys Cys Ser Glu Thr Lys Trp Glu Pro Met Cys Gly Glu Asn Gly Ile
                485                 490                 495

aca tat gta tca gct tgt ctt gct ggt tgt caa acc tcc aac agg agt 1536
Thr Tyr Val Ser Ala Cys Leu Ala Gly Cys Gln Thr Ser Asn Arg Ser
            500                 505                 510

gga aaa aat att ata ttt tac aac tgc act tgt gtg gga att gca gct 1584
Gly Lys Asn Ile Ile Phe Tyr Asn Cys Thr Cys Val Gly Ile Ala Ala
        515                 520                 525

tct aaa tcc gga aat tcc tca ggc ata gtg gga aga tgt cag aaa gac 1632
Ser Lys Ser Gly Asn Ser Ser Gly Ile Val Gly Arg Cys Gln Lys Asp
    530                 535                 540

aat gga tgt ccc caa atg ttt ctg tat ttc ctt gta att tca gtc atc 1680
Asn Gly Cys Pro Gln Met Phe Leu Tyr Phe Leu Val Ile Ser Val Ile
545                 550                 555                 560

aca tcc tat act tta tcc cta ggt ggc ata cct gga tac ata tta ctt 1728
Thr Ser Tyr Thr Leu Ser Leu Gly Gly Ile Pro Gly Tyr Ile Leu Leu
                565                 570                 575

ctg agg tgc att aag cca cag ctt aag tct ttt gcc ttg ggt atc tac 1776
Leu Arg Cys Ile Lys Pro Gln Leu Lys Ser Phe Ala Leu Gly Ile Tyr
            580                 585                 590

aca tta gca ata aga gtt ctt gca gga atc cca gct cca gtg tat ttt 1824
Thr Leu Ala Ile Arg Val Leu Ala Gly Ile Pro Ala Pro Val Tyr Phe
        595                 600                 605

gga gtt ttg att gat act tca tgc ctc aaa tgg gga ttt aaa aga tgt 1872
Gly Val Leu Ile Asp Thr Ser Cys Leu Lys Trp Gly Phe Lys Arg Cys
    610                 615                 620

gga agt aga gga tca tgc aga tta tat gat tca aat gtc ttc aga cat 1920
Gly Ser Arg Gly Ser Cys Arg Leu Tyr Asp Ser Asn Val Phe Arg His
625                 630                 635                 640

ata tat ctg gga cta act gtg ata ctg ggc aca gtg tca att ctc cta 1968
Ile Tyr Leu Gly Leu Thr Val Ile Leu Gly Thr Val Ser Ile Leu Leu
                645                 650                 655

agc att gca gta ctt ttc att tta aag aaa aat tat gtt tca aaa cac 2016
Ser Ile Ala Val Leu Phe Ile Leu Lys Lys Asn Tyr Val Ser Lys His
            660                 665                 670

aga agt ttt ata acc aag aga gaa aga aca atg gtg tct aca aga ttc 2064
Arg Ser Phe Ile Thr Lys Arg Glu Arg Thr Met Val Ser Thr Arg Phe
        675                 680                 685

caa aag gaa aat tac act aca agt gat cat ctg cta caa ccc aac tac 2112
Gln Lys Glu Asn Tyr Thr Thr Ser Asp His Leu Leu Gln Pro Asn Tyr
    690                 695                 700

tgg cca ggc aag gaa act caa ctt tag 2139
Trp Pro Gly Lys Glu Thr Gln Leu *
705                 710